# EUROPEAN PATENT SPECIFICATION

(11) **EP 0 513 370 B1**
(45) Date of publication and mention of the grant of the patent: **16.09.1998**
(21) Application number: 91919275.7
(22) Date of filing: 11.10.1991
(51) Int. Cl.: C09B 47/18, C07D 487/22, C09K 3/00, G11B 7/24, G03G 5/06

(54) **HALOGENATED PHTHALOCYANINE COMPOUND, PRODUCTION THEREOF, AND OPTICAL RECORDING MEDIUM PRODUCED THEREFROM**
HALOGENIERTE PHTHALOCYANINVERBINDUNG, DEREN HERSTELLUNG UND DIESE ENTHALTENDES OPTISCHES AUFZEICHNUNGSMEDIUM
COMPOSE DE PHTALOCYANINE HALOGENE, PRODUCTION DE CE COMPOSE ET SUPPORT D'ENREGISTREMENT OPTIQUE FABRIQUE A PARTIR DE CE COMPOSE

(30) Priority: 06.11.1990 JP 298792/90; 01.07.1991 US 723589; 12.07.1991 US 729338
(43) Date of publication of application: 19.11.1992
(73) Proprietor: MITSUI CHEMICALS, INC., Tokyo (JP); Yamamoto Chemicals, Inc., Yao-shi Osaka 581 (JP)
(72) Inventor: ITOH, Hisato, Yokohama-shi, Kanagawa 244 (JP); OGUCHI, Takahisa, Yokohama-shi, Kanagawa 244 (JP); AIHARA, Shin, Yokohama-shi, Kanagawa 244 (JP); SUGIMOTO, Kenichi, Yokohama-shi, Kanagawa 244 (JP); ENOMOTO, Katashi, Zushi-shi, Kanagawa 249 (JP); NISHIZAWA, Tsutomu, Yokohama-shi, Kanagawa 247 (JP)
(74) Representative: Luderschmidt, Schüler & Partner GbR
(86) International application number: JP9101384
(87) International publication number: WO9207911

(56) References cited:
- EP-A- 0 272 565
- EP-A- 0 451 718
- EP-A- 0 462 368
- FR-A- 2 252 386
- GB-A- 2 168 372
- JP-A- 3 062 878
- CHEMICAL ABSTRACTS, Vol. 112, No. 10, 86672m (March 1990), BERNSTEIN P.A. et al. "Two-electron oxidation of cobalt phthalocyanines by thionyl chloride. Implications for lithium/thionyl chloride batteries". p. 626.

## Description

### Technical Field

The present invention relates to a halogenated alkoxyphthalocyanine which is useful as a dye, a pigment, a filter, a liquid crystal display material and a recording material. Furthermore, the present invention relates to an optical recording medium using the compound and a mixture thereof as well as a method for preparing the compound and the mixture.

### Background Art

Halogenated alkoxyphthalocyanines are disclosed in EP 0373643 and Japanese Patent Application Laid-open Nos. 197280/1986 and 85630/1975.

Techniques of using the halogenated alkoxyphthalocyanines as optical recording media are described in EP 373643, Japanese Patent Application Laid-open No. 197280/ 1986, USP 4298975, EP 353394 and USP 4769307. However, the optical recording media using the compounds described in the above-mentioned patents are insufficient in sensitivity, a refractive index, recording properties and reflectance.

In particular, EP 373643 discloses the phthalocyanines each having four alkoxy groups, or four alkoxy groups and four substituents. However, among the phthalocyanines disclosed in this patent, for example, the compounds shown in Examples 93 to 100 had a small refractive index of 2 or less at 780 nm and insufficient sensitivity and recording properties. On the other hand, the compounds shown in Examples 100 to 103 could not provide an industrially homogeneous recording layer, because the solubility of these compounds in a coating solvent was 10 g/l which was too low to prepare the optical recording medium therefrom in accordance with a spin coating process by the use of a polycarbonate substrate. In addition, the sensitivity, recording properties and reflectance of the media were insufficient.

With regard to the halogenation of the phthalocyanine, Publication Board Report No. 65,657 describes a method in which antimony trisulfide or aluminum chloride is used as a catalyst. However, this method could not be applied to such a phthalocyanine containing the alkoxy group as in the present invention, since the alkoxy group substituted on a benzene ring, for example, a phenyl alkyl ether decomposes in the presence of aluminum chloride into phenol and an alcohol. This decomposition reaction is described in the literatures of Chem. Ber., 76B, 900 (1943), J. Org. Chem. 27, 2037 (1962) and Chem. Ber., 93, 2761 (1960).

Furthermore, J. Org. Chem., 6, 852 (1941) and Chemical Industries, 1138 (1967) describe the decomposition reaction in which an acid byproduced in the halogenation reaction, for example, hydrogen chloride or hydrogen bromide produced at the halogenation with chlorine or bromine decomposes a phenyl ether into phenol and an alcohol.

### Disclosure of the Invention

A first object of the present invention is to provide a phthalocyanine mixture which is useful as a recording layer for writable compact disks (abbreviate to "CD-WO") having excellent sensitivity and reflectance, a high refractive index and excellent recording properties.

A second object of the present invention is to provide a synthetic route of a halogenated alkoxyphthalocyanine compound or mixture thereof according to the present invention and optimum conditions for the synthesis.

A third object of the present invention is to provide a recording medium, particularly a CD-WO recording medium in which the halogenated alkoxyphthalocyanine mixture thereof is contained in a recording layer.

The present inventors have intensively investigated with the intention of solving the above-mentioned problems, and as a result, they have found that a mixture into which alkoxy groups having a large steric hindrance and halogen atoms are introduced are excellent in sensitivity, reflectance and recording properties and have a high refractive index as the CD-WO recording layer. It has also been found that the phthalocyanine mixture containing the alkoxy groups in which the total number of the secondary, tertiary or quaternary carbons is from 2 to 4, and containing 1 to 4 halogen atoms, preferably bromine atoms are particularly preferable.

In preparing the optical recording medium, a substrate prepared from polycarbonate by extrusion molding is preferable from the viewpoints of transparency and economy. For the mass production, the recording layer is preferably applied by a spin coating method. That is, it has been found that in order to apply the recording layer in an optimum shape on the polycarbonate substrate without destroying guide grooves on the substrate, among the halogenated alkoxyphthalocyanines which can be provided in accordance with the present invention, what is particularly preferable is an isomer in which the symmetry of the alkoxy groups substituted is low, or an isomer of a phthalocyanine molecule in which the symmetry is low, that is, an isomer having no symmetrical center. Furthermore, it has been found that when five or more isomers are mixed, there can be achieved the optimization of solubility in a coating solvent, the stability of a coating solution and the shape of the applied recording layer.

Moreover, according to the present invention, there can be provided a method for preparing the halogenated alkoxyphthalocyanine mixture. That is, there is a method for forming a phthalocyanine ring by mixing 2 to 4 kinds of isomers of halogenated alkoxyphthalonitriles and their derivatives, or a method for halogenating an alkoxyphthalocyanine. It has been found that particularly as the method for inexpensively preparing a mixture having different halogenation ratios by mixing of optimum isomers, it is preferable to carry out reaction in a mixed two-layer solvent system of water and an organic solvent which can form two layers with water. It has been found that when this method is used, the mixing of the isomers and the control of halogenation ratio are easy, and the halogenated alkoxyphthalocyanine having optimum maximum absorption wave length, a high refractive index, high reflectance, high sensitivity and excellent recording properties can be obtained in a high yield.

### Brief Description of the Drawings

Fig. 1 is a liquid chromatogram of a brominated alkoxyphthalocynine mixture obtained in Example 18.

Fig. 2 is a liquid chromatogram of a brominated alkoxyphthalocynine mixture obtained in Example 19.

Fig. 3 is a liquid chromatogram of a brominated alkoxyphthalocynine mixture obtained in Example 20.

Fig. 4 is a liquid chromatogram of a brominated alkoxyphthalocynine mixture obtained in Example 21.

Fig. 5 is a liquid chromatogram of a brominated alkoxyphthalocynine mixture obtained in Example 22.

Fig. 6 is a liquid chromatogram of a brominated alkoxyphthalocynine mixture obtained in Example 24.

Fig. 7 is a liquid chromatogram of a brominated alkoxyphthalocynine mixture obtained in Example 25.

Fig. 8 is a liquid chromatogram of a brominated alkoxyphthalocynine mixture obtained in Example 26.

Fig. 9 is a liquid chromatogram of a brominated alkoxyphthalocynine mixture obtained in Example 27.

### Best Mode for Carrying Out the Invention

The present inventors have investigated compounds suitable for CD-WO, and they have found that it is particularly important to possess the following five features.
(1) Since CD-WO utilizes a laser beam at about 780 nm to write and read records, a recording material preferably has high sensitivity, a high refractive index and high reflectance at about 780 nm.
   Therefore, the desirable recording material is a compound which has an absorbance at a maximum absorption wave length (λₘₐₓ), i.e., a maximum molecular extinction coefficient (εₘₐₓ) of 150,000 or more and which can provide a recording medium having a refractive index of 1.8 or more, particularly preferably 2.0 or more at 780 nm.
(2) A phthalocyanine having a large steric hindrance, particularly a phthalocyanine having the combination of bromine atoms and alkoxy groups which has 2 to 4 of the secondary, tertiary or quaternary carbons and in which the total number of the carbon atoms is from 6 to 9 has good decomposability (a balance between a thermal decomposition starting temperature and a melting temperature) in a writing machine equipped with a semiconductor laser for carrying out CD-WO record.
   A preferable substitution site of the alkoxy group is an α-position. That is, an isomer having any one of the following formulae (1) to (4) and particularly, having no symmetrical center and a mixture containing 50% or more of this kind of isomer is preferable.
(3) In order to have the high refractive index, the central metal is preferably an atom having a large atomic radius, i.e., Pd, Pt, Rh, Ru, In, VO or a derivative of Sn, or Cu, Ni, Co, Fe, Pd, Pt or VO in the viewpoint of durability.
(4) In the case that the symmetry of the molecule is low, for example, compounds represented by the following formulae (2), (3) and (4) as well as the undermentioned formulae (1-3), (1-4), (1-5), (1-6), (1-7) and (1-8) are excellent in solubility in a coating solvent. As a result, the uniform coating of the recording layer is possible, and it is also possible to form the recording layer in an optimum shape on a substrate.
(5) CD-WO is required a reflectance of 65% or more. Therefore, it is preferable to introduce, into a phthalocyanine, a substituent having a large steric hindrance and a halogen atom, preferably bromine or iodine having a large atomic refractive index as an auxiliary group for improving its steric hindrance effect.

That is, the present inventors have intensively investigated to solve the above-mentioned problems, and as a result, they have found that a mixture of halogenated alkoxyphthalocyanine compounds substituted by alkoxy groups having 2 to 4 of the secondary to quaternary carbon atoms which can be represented by the formulae (1) to (4) is a preferable compound: 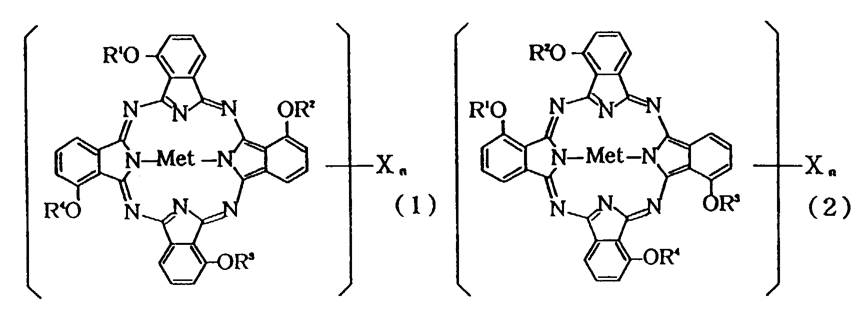 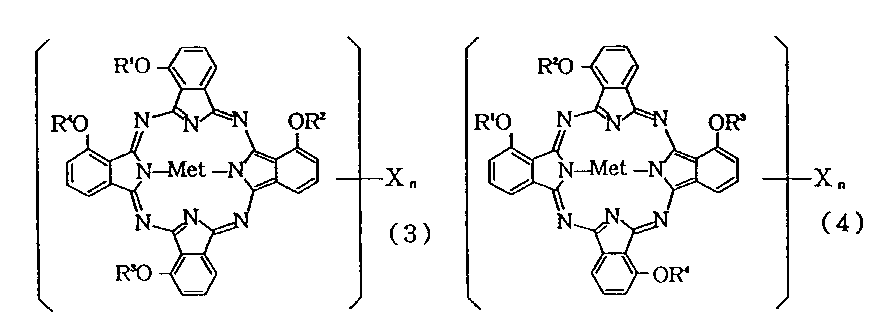 wherein each of R¹ to R⁴ is independently an alkyl group which has 2 to 4 of the secondary, tertiary or quaternary carbon atoms and in which the total number of the carbon atoms is from 6 to 9; Met is a divalent metallic atom, a trivalent one-substituted metallic atom or a tetravalent two-substituted metallic atom; X is a halogen atom such as chlorine, bromine or iodine; and n is the substitution number of X and it is from 1 to 4.

That is, the increase of the steric hindrance of the alkoxy groups leads to drop a decomposition temperature and to elevate a melting point. Accordingly, the writing can be effected by a small laser power, and the shape of each written signal is good.

Among the halogenated alkoxyphthalocyanines represented by the formulae (1) to (4), the following mixtures of compounds are particularly preferable. That is, it has been found that mixtures of compounds which are particularly desirable to solve the above-mentioned problems are the mixtures of halogenated alkoxyphthalocyanine compounds in which the substituent represented by each of R¹ to R⁴ is one selected from the group consisting of a 1-iso-propyl-2-methylbutyl group, 1-t-butyl-2-methylpropyl group, 1-iso-propyl-2-methylpropyl group, 1,2-dimethylbutyl group, 1-iso-propylpropyl group and 1-iso-propylbutyl group; the central metal Met is one selected from the group consisting of VO (vanadium oxide), divalent metallic atoms such as iron, cobalt, nickel, copper, zinc, ruthenium, rhodium, palladium and platinum and their derivatives; and the halogen atom is a bromine atom; and the number of the bromine atoms is from 1 to 4.

A compound which is preferable as a raw material in the method for preparing the compound of the present invention is one to four kinds of alkoxyphthalonitriles or alkoxydiiminoisoindolines represented by the formula (5) or (6) 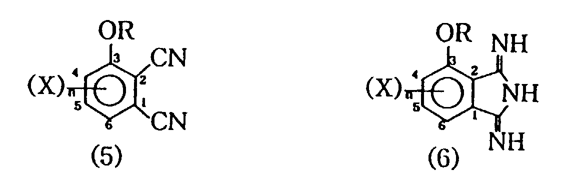 wherein R is an alkyl group which has 2 to 4 of the secondary, tertiary or quaternary carbon atoms and in which the total number of the carbon atoms is from 6 to 9; X is a halogen atom such as chlorine, bromine or iodine and is bonded at the 4-position or 6-position; and n is 0 or 1, and at least one of these compounds has the substituent X.

The particularly preferable raw material is a compound of the formula (5) or (6) in which R is one selected from the group consisting of a 1-iso-propyl-2-methylbutyl group, 1-t-butyl-2-methylpropyl group, 1-iso-propyl-2-methylpropyl group, 1,2-dimethylbutyl group, 1-iso-propylpropyl group and 1-iso-propylbutyl group; and X is bromine.

Examples of a metal or a metallic compound which is another raw material of the present invention include aluminum, silicon, titanium, vanadium, chromium, manganese, iron, cobalt, nickel, copper, zinc, gallium, germanium, ruthenium, rhodium, palladium, indium, tin, platinum and chlorides, bromides, iodides, acetates and oxides thereof. Examples of the particularly preferable raw material include iron chloride, iron bromide, iron acetate, cobalt chloride, cobalt bromide, cobalt acetate, nickel chloride, nickel bromide, nickel acetate, copper chloride, copper bromide, copper iodide, copper acetate, zinc chloride, zinc bromide, zinc acetate, ruthenium chloride, rhodium chloride, rhodium bromide, palladium chloride, palladium bromide, palladium acetate, platinum chloride and platinum bromide.

With regard to conditions for the synthesis of a phthalocyanine ring, one to four kinds of alkoxyphthalonitriles or alkoxydiiminoisoindolines which are the raw materials are heated and reacted at 10 to 300°C in a solvent, preferably an alcohol. When the raw material is the alkoxyphthalonitrile represented by the formula (5), a reaction temperature is preferably from 80 to 160°C. Furthermore, when the raw material is the alkoxydiiminoisoindoline represented by the formula (6), the reaction temperature is preferably from 140 to 200°C. In addition, an auxiliary such as diazabicycloundecene (DBU) or diazabicylononene (DBN) may be added as a catalyst for the ring formation reaction.

The alkoxyphthalonitrile (5) or the diiminoisoindoline (6) which is used in the present invention can be synthesized by the following procedure:

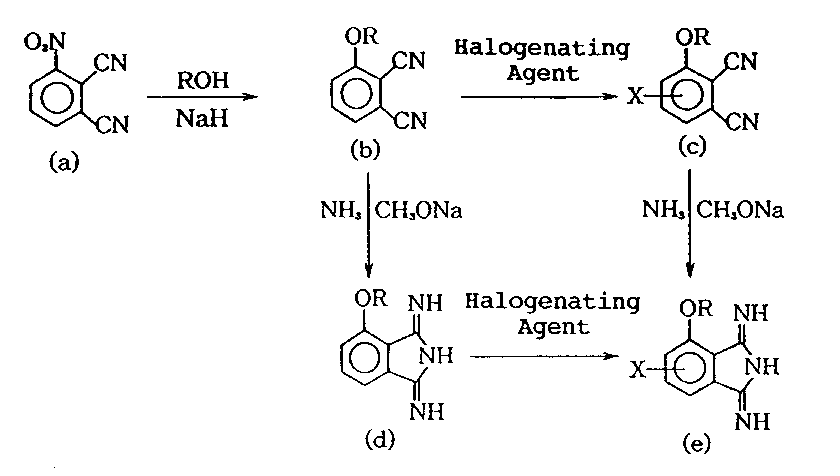

3-nitrophthalonitrile is available, for example, from Tokyo Chemicals, Inc. The synthesis of an alkoxyphthalonitrile (b) from a nitrophthalonitrile (a) can be achieved by a method described in NOUVEAU JOURNAL DE CHIMIE, Vol. 6, No. 12, p 653-58 (1982). That is, an alcohol is converted into sodium alkoxide with the aid of sodium hydride, and it is successively reacted with nitrophthalonitrile at 0 to 100°C to form the alkoxyphthalonitrile.

A halogenated alkoxyphthalonitrile (c) can be synthesized by halogenating an alkoxyphthalonitrile in accordance with a method described in I. T. Harrison and S. Harrison, "COMPENDIUM OF ORGANIC SYNTHETIC METHODS", Vol. 1-6, Wiley-Interscience Co., Ltd., and then carrying out separation/purification by column chromatography. Preferable examples of a halogenating agent which can be used in the above-mentioned halogenation include chlorine, bromine, iodine, sulfuryl chloride, thionyl chloride, antimony chloride, ICl₃, FeCl₃, phosphorus pentachloride, phosphorus oxychloride, t-butyl hypochlorite, N-chlorosuccinic acid imide, cupric bromide, quaternary ammonium bromide, N-bromosuccinimide, iodine monochloride, quaternary ammonium iodide and potassium triiodide. The halogenating agent can be suitably used in the range of 1 to 2 moles per mole of the alkoxyphthalonitrile.

Moreover, an alkoxydiiminoisoindoline (d) can be synthesized from the above-mentioned alkoxyphthalonitrile (b), and a halogenated alkoxydiiminoisoindoline (e) can be synthesized from the above-mentioned halogenated alkoxyphthalonitrile (c) by a reaction with ammonia. In addition, the compound (e) can be synthesized by halogenating the compound (d) in the same manner as mentioned above.

The present inventors have found an economically excellent procedure of synthesizing a mixture of isomers having different halogenation degrees in which the halogenated alkoxyphthalocyanine having a controlled halogenation ratio can be obtained by reacting the alkoxyphthalocyanine at an adjusted reaction temperature in an adjusted amount of a two-layer system mixed solvent containing, as the main components, water and an organic solvent which is not substantially mixed with water such as an aliphatic hydrocarbon, a halogenated hydrocarbon and a straight-chain or cyclic ether. In consequence, the present invention has been attained. If this reaction is carried out by a conventional technique, it does not wonder that a hydroxyphthalocyanine which is product obtained from a decomposition reaction of the alkoxy groups is produced in large quantities as a by-product. However, surprisingly, the present inventors have found that when the present invention is carried out, the by-production of the hydroxyphthalocyanine is so extremely small as not to affect the quality of the aimed product.

In general, the method for preparing the halogenated alkoxyphthalocyanine is described in Japanese Patent Laid-Open No. 50-85630 and J. Chem. Soc., Perkin Trans. I, p. 2453-58 (1988). The former describes a method comprising the step of substituting the halogenated phthalocyanine by an alkali metal salt of an aliphatic alcohol or an alkali metal salt of an aromatic alcohol to prepare the desired halogenated alkoxyphthalocyanine or halogenated aryloxyphthalocyanine. The latter describes a method comprising the step of subjecting a dialkoxydihalogenophthalonitrile to a ring closure reaction to prepare the desired halogenated alkoxyphthalocyanine. However, this method could not be applied to a process for introducing 1 to 4 halogen atoms into a tetraalkoxyphthanocyanine.

In general, a halogenation method comprises the step of dissolving a substrate in a solvent which is inert to the halogenating agent to accomplish the halogenation. However, the inventors have found that when the reaction is carried out in a halogenating agent such as chloroform or carbon tetrachloride or acetic acid at the time of the halogenation of the alkoxyphthalocyanine compound, a solid precipitates during the reaction, so that the halogenating reaction does not proceed sufficiently and it is difficult to control the amount of the halogen atom to be introduced.

That is, the present invention is directed to a method for preparing a halogenated phthalocyanine compound represented by the formula (8) 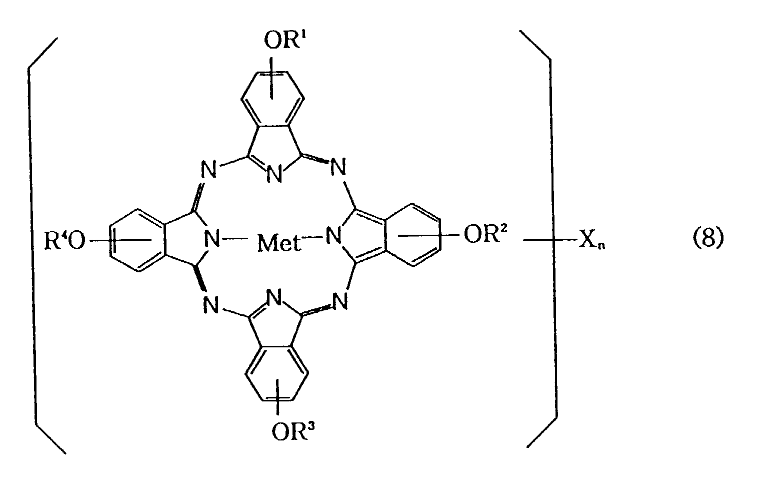 wherein each of R¹, R², R³ and R⁴ independently is an alkyl group having 1 to 20 carbon atoms, preferably a substituted or unsubstituted alkyl group having 3 to 10 carbon atoms; and Met is two hydrogen atoms, a divalent metallic atom, a trivalent or a tetravalent metallic derivative; X is chlorine, bromine or iodine; and n is 1 ≤ n ≤ 12, which comprises the step of reacting a phthalocyanine compound represented by the formula (7) 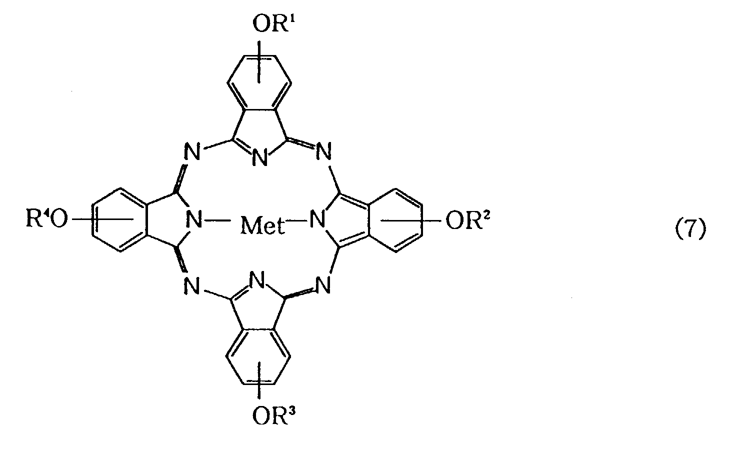 wherein R¹, R², R³, R⁴ and Met have the same meanings as in the formula (8),
with a halogenating agent at 20 to 90°C in a mixed solvent of an organic solvent and water. The compounds represented by the formulae (7) and (8) in which two or more of OR¹, OR², OR³ and OR⁴ are bonded to one benzene ring are also in the category of the present invention.

It can be presumed that when the phthalocyanine compound is reacted with the halogenating agent in the mixed solvent of water and the organic solvent which is not substantially mixed with water in accordance with the present invention, a hydrogen halide or a salt of the halogenating agent and the like which are by-products of the reaction are dissolved in water, whereby the phthalocyanine compound is prevented from precipitating together with the by-products of the reaction from the organic solvent which is the reaction solvent. In the case of the alkoxy group having the large steric hindrance, the compound substituted by 2 or more halogen atoms cannot be synthesized sometimes, if water is not present.

The particularly preferable alkoxyphthalocyanines as the raw material of the present invention are α-alkoxyphthalocyanines represented by the formulae (9) to (12). As the raw material, it is preferable to use a mixture of (9) to (12) 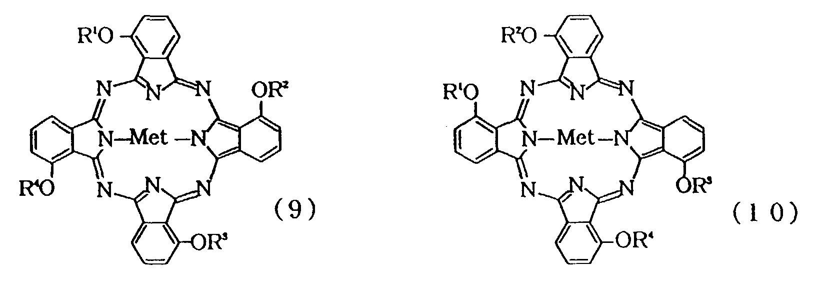 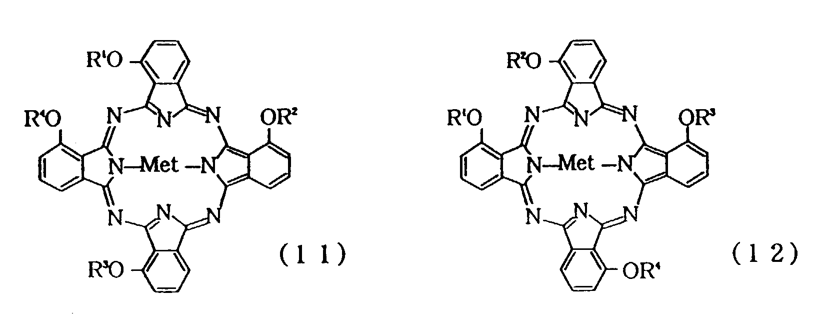 wherein each of R¹, R², R³ and R⁴ is independently an alkyl group having 1 to 20 carbon atoms, preferably a substituted or unsubstituted alkyl group having 3 to 10 carbon atoms; Met is two hydrogen atoms, a divalent metallic atom, a trivalent or a tetravalent metallic derivative.

In the preferable raw material, R¹, R², R³ and R⁴ of the formulae (9) to (12) are the branched alkyl groups. In the particularly preferable raw material, R¹, R², R³ and R⁴ of the formulae (9) to (12) are the secondary alkyl groups.

Examples of the substituted and unsubstituted alkyl groups of R¹, R², R³ and R⁴ in the formulae (7) and (9) to (12) include hydrocarbon groups such as a methyl group, ethyl group, n-propyl group, iso-propyl group, n-butyl group, iso-butyl group, sec-butyl group, t-butyl group, n-pentyl group, iso-pentyl group, neo-pentyl group, 1,2-dimethylpropyl group, n-hexyl group, cylcohexyl group, 1,3-dimethylbutyl group, 1-iso-propylpropyl group, 1,2-dimethylbutyl group, n-heptyl group, 1,4-dimethylpentyl group, 2-methyl-1-iso-propylpropyl group, 1-ethyl-3-methylbutyl group, n-octyl group, 2-ethylhexyl group, 3-methyl-1-iso-propylbutyl group, 2-methyl-1-iso-propylbutyl group, 1-t-butyl-2-methylpropyl group and n-nonyl group; alkoxyalkyl groups such as a methoxymethyl group, methoxyethyl group, ethoxyethyl group, propoxyethyl group, butoxyethyl group, methoxyethoxyethyl group, ethoxyethoxyethyl group, dimethoxymethyl group, diethoxymethyl group, dimethoxyethyl group and diethoxyethyl group; and halogenated alkyl groups such as a chloromethyl group, 2,2,2-trichloroethyl group, trifluoromethyl group, 1,1,1,3,3,3-hexafluoro-2-propyl group.

Above all, the preferable alkyl group is the alkyl group having 2 to 4 of the secondary, tertiary and quaternary carbon atoms in all, and particularly, examples of such an alkyl group include a 1,2-dimethylpropyl group, 1,3-dimethylbutyl group, 1-iso-propylpropyl group, 1,2-dimethylbutyl group, 1,4-dimethylpentyl group, 2-methyl-1-iso-propylpropyl group, 1-ethyl-3-methylbutyl group, 3-methyl-1-isopropylbutyl group, 2-methyl-1-iso-propylbutyl group and 1-t-butyl-2-methylpropyl group.

Examples of the divalent metal represented by Met in the formulae (7) and (9) to (12) include Cu, Zn, Fe, Co, Ni, Ru, Rh, Pd, Pt, Mn and Sn; examples of the monosubstrated trivalent metal include Al-Cl, Al-Br, In-Cl, In-Br and In-I; examples of the divalent tetravalent metal include SiCl₂, SiBr₂, SiF₂, SnCl₂, SnBr₂, SnF₂, GeCl₂, GeBr₂, GeF₂, Si(OH)₂, Sn(OH)₂ and Ge(OH)₂; and examples of the metallic oxide include VO and TiO. Particularly preferable examples include Cu, Ni, Pd and Pt.

The halogenating agent which can be used in the present invention is a compound represented by the formula (13)

X - Y (13)

wherein X is a halogen atom, and Y is a residue of the halogenating agent. Examples of the halogen atoms include F, Cl, Br and I, and Br is preferable. Examples of the residue of the halogenating agent include Cl, Br, I, SO₂Cl, SOCl, FeCl₂, PCl₄, POCl₂, CuBr and quaternary ammonium.

Typical examples of the halogenating agent include chlorine, bromine, iodine, sulfury chloride, thionyl chloride, antimony chloride, ICl₃, FeCl₃, phosphorus pentachloride, phosphorus oxychloride, t-butyl hypochlorite, N-chlorosuccinic imide, cupric bromide, quaternary ammonium bromide, N-bromosuccinimide, iodine monochloride, quaternary ammonium iodide and potassium triiodide. Particularly, bromine is preferable. The halogenating agent can be suitably used in the molar ratio of 1 to 6 moles followed by the desirably introduced halogen quantity. It has been found that when bromine is used, there is a particularly definite feature. That is, when bromine is used in a molar ratio of 2 moles per mole of the alkoxyphthalocyanine, 1, 2, 3 or 4 bromine atoms are introduced. When bromine is used in a molar ratio of 2.5 to 4.0 moles, 2, 3 or 4 bromine atoms are introduced. Even if 4 moles or more of bromine are used, the maximum number of the bromine atoms to be introduced is 4.

A reaction temperature is in the range of 20 to 90°C, preferably 40 to 70°C. When the reaction temperature is less than 20°C, the reaction does not proceed successively, and when it is more than 90°C, it is difficult to control a halogenation ratio.

The organic solvent is a solvent which is substantially immiscible with water, i.e., a solvent which forms two layers with water and which can dissolve the phthalocyanine compounds of the formulae (7) and (9) to (12). The preferable organic solvent is one or more selected from saturated hydrocarbons, ethers and halogenated hydrocarbons. The further preferable organic solvent is one or more selected from the group consisting of n-hexane, n-pentane, n-octane, cyclohexane, methylcyclohexane, ethylcyclohexane, tetrahydrofuran, n-butyl ether, n-propyl ether, isopropyl ether, carbon tetrachloride, chloroform, dichloromethane, 1,1,1-trichloroethane, 1,1,2-trichloroethane and 1,1,2,2-tetrachloroethane.

The amount of the organic solvent is 2 to 500 times by weight, preferably 3 to 200 times by weight as much as that of the phthalocyanine which is the raw material. It is necessary to completely dissolve the phthalocyanine. However, when the amount of the organic solvent is less than 2 times by weight, a solid tends to precipitate during the reaction and impedes the reaction, and conversely when it is more than 500 times by weight, the reaction is improperly too slow. In particular, when 1,1,2-trichloroethane or 1,1,2,2-tetrachloroethane is used, the amount of the organic solvent is preferably from 4 to 10 times by weight.

The amount of water is 0.05 to 10 times by weight, preferably 0.1 to 5 times by weight as much as that of the organic solvent. It is necessary to obtain such a ratio that many interfaces are formed between water and the organic solvent. When the amount of water is less than 0.05 times by weight, there is no effect of mixing water, and the solid easily precipitates during the reaction and impedes the reaction. Conversely, when it is more than 10 times by weight, the amount of the solvent is too much and the efficiency of the reaction deteriorates improperly.

The halogenated alkoxyphthalocyanine prepared under the above-mentioned conditions is represented by the formula (8): 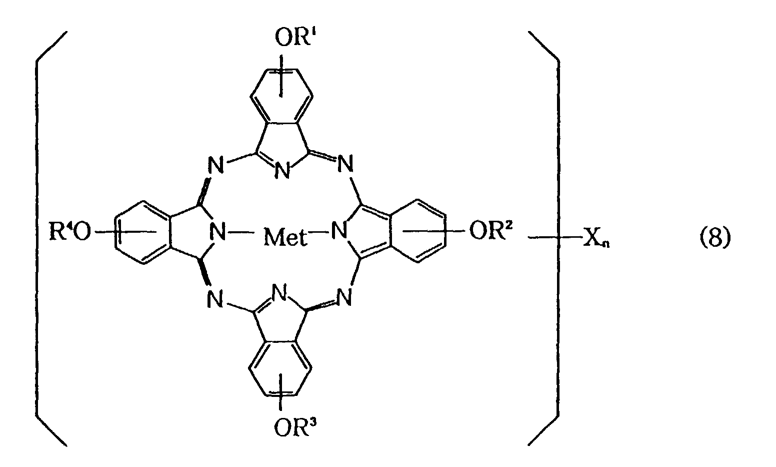 wherein R¹, R², R³, R⁴ and Met have the same meanings as in the formula (7); X is chlorine, bromine or iodine; and n is 1 ≤ n ≤ 12. Preferable examples of the halogenated alkoxyphthanocyanine are compounds represented by the following formulae (1) to (4) and mixtures thereof: 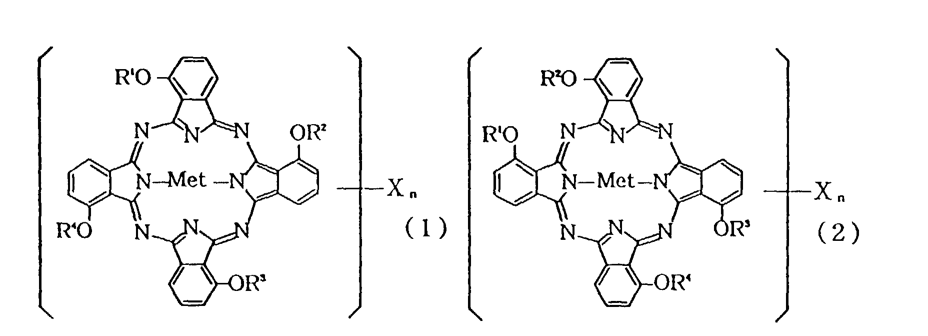 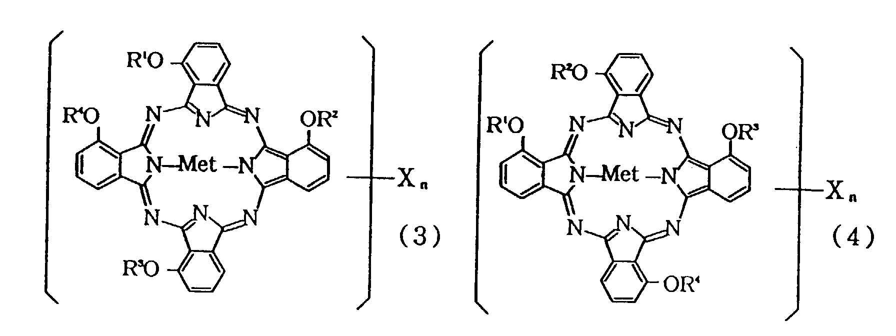 wherein R¹, R², R³, R⁴, Met and X have the same meanings as in the formula (8).

In the halogenated alkoxyphthalocyanines represented by the formulae (1) to (4) and the mixtures thereof, it is most preferable that each of R¹ to R⁴ is a secondary alkyl group, particularly an alkyl group having 2 to 4 of the secondary, tertiary and quaternary carbon atoms in all.

The thus prepared halogenated alkoxyphthalocyanine is a mixture of 5 or more kinds of isomers or compounds having different contents of bromine. When this mixture is used without separation to prepare an optical recording medium, the medium which can solve the above-mentioned problems can be obtained. Even if the composition ratio of this mixture alters, the performance of the mixture as the optical recording medium does not deteriorate, but one or a mixture of 2 or 3 of the halogenated alkoxyphthalocyanines cannot solve the above-mentioned problems to a sufficient degree.

The recording medium which is suitable for the compound of the present invention has a constitution in which a recording layer, a reflective layer and a protective layer are laminated in this order on a substrate.

A resin for use in the substrate of the present invention should be optically transparent. Example of the material for the substrate include a polyacrylic resin, polyolefin resin, polycarbonate resin and polyester resin. In this case, the surface of the substrate may be covered with a photosetting resin or thermosetting resin.

Examples of the resin for the protective layer include a thermosetting resin and photosetting resin such as an acrylic resin and a urethane resin. The thickness of the protective layer is preferably from 1 µm to 1 mm.

Examples of the material for the reflective layer include metals such as aluminum, gold and silver. The thickness of the reflective layer is preferably from 20 to 200 nm.

The recording layer can be prepared by applying, on the substrate, a coating solution containing the compound of the present invention and a binder resin in an amount of 20% by weight or less based on the compound by the use of a spin coater. The concentration of the coating solution is preferably from 5 to 100 g/l. Any coating solvent can be used, so long as it does not attack the substrate. Preferable examples of the coating solvent include n-hexane, cyclohexane, n-octane, ethylcyclohexane, methylcyclohexane, cyclooctane, tetrachloroethane, carbon tetrachloride, dichloromethane, chloroform, THF, dioxane and a mixture thereof. The thickness of the recording layer is preferably from 50 to 300 nm.

The alkoxyphthalocyanines represented by the formulae (7), (9) to (12) can be prepared by methods described in U. S. Patent No. 4,769,307 and NOUVEAU JOURNAL DE CHIMIE, Vol. 6, No. 12, p. 653-58 (1982). That is, the alkoxyphthalocyanine can be synthesized in accordance with the following reaction (14):

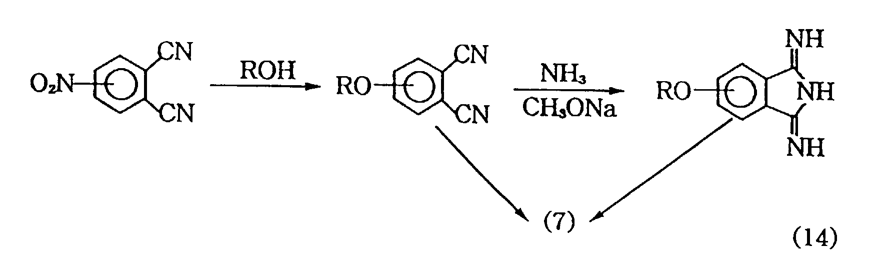

In the first place, an alcohol is reacted with sodium hydride at 0 to 30°C to form a sodium alkoxide, and nitrophthalonitrile is successively added thereto and reaction is then carried out at 0 to 100°C to obtain an alkoxyphthalonitrile. Next, the thus obtained alkoxyphthalonitrile is reacted with a metallic salt being a molar ratio of 0.8 to 1.2 at 100 to 300°C in an alcohol to obtain an alkoxyphthalocyanine. Alternatively, a diiminoisoindoline may be derived from the alkoxyphthalonitrile and then reacted with a metallic salt to similarly obtain the alkoxyphthalocyanine.

Now, the present invention will be described in detail with reference to examples, but the scope of the present invention should not be limited only to these examples.

### Example 1

Ten grams of phthalonitrile represented by the following formula (5-1) were mixed with 2 g of palladium chloride, 4 g of DBU and 200 g of n-amyl alcohol, and reaction was then carried out at 95°C for 24 hours.

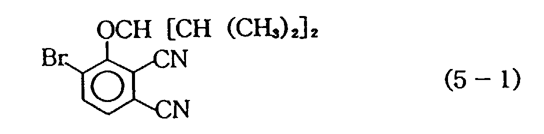

The resultant reaction mixture was poured into 1 liter of methanol, and the precipitated tar was then separated/purified by column chromatography to obtain 2 g of the following formula (1-1), 2 g of (2-1), 0.5 g of (3-1) and 0.5 g of (4-1). Physical properties of these compounds are set forth in Table 1.

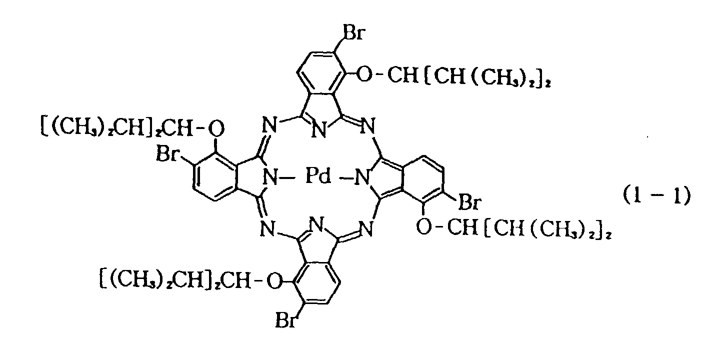

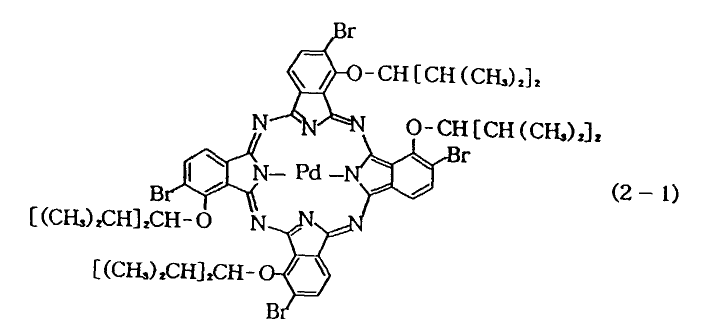

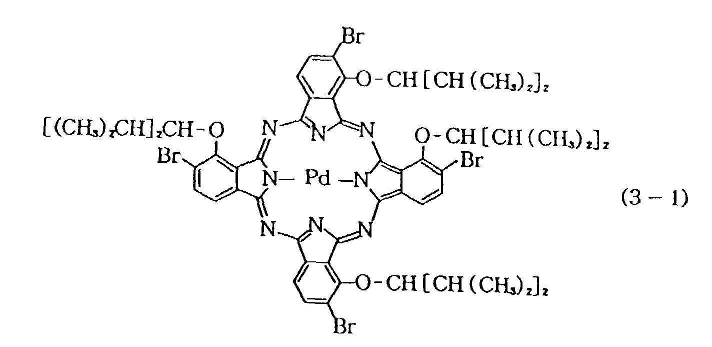

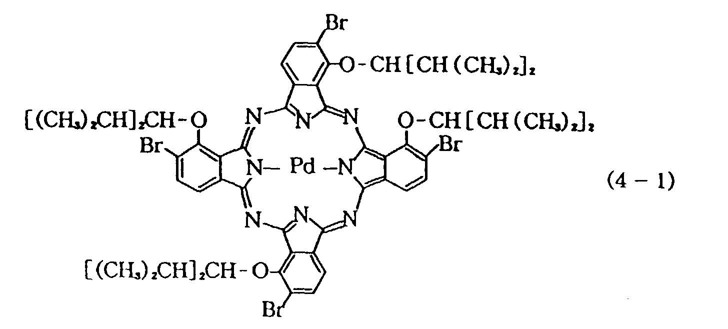

**Table 1**

| Compound No. | λₘₐₓ(nm) (εₘₐₓ×10⁻⁵) | mass (m/e) | Elemental Analysis (C,H,N) Found (Calcd.) |
|---|---|---|---|
| (1-1) | 715 | 1391 | 51.82, 4.91, 8.03 |
| | (2.2) | | (51.80, 4.93, 8.05) |
| (2-1) | 716 | 1391 | 51.78, 4.90, 8.06 |
| | (2.2) | | (51.80, 4.93, 8.05) |
| (3-1) | 715 | 1391 | 51.82, 4.94, 8.03 |
| | (2.1) | | (51.80, 4.93, 8.05) |
| (4-1) | 716 | 1391 | 51.81, 4.91, 8.07 |
| | (2.2) | | (51.80, 4.93, 8.05) |

### Example 2

Ten grams of diiminoisoindoline represented by the following formula (6-1), 2 g of palladium chloride, 4 g of DBU and 200 g of n-octyl alcohol were mutually mixed, and reaction was then carried out under reflux for 4 hours.

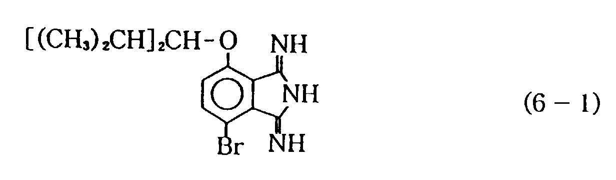

The resultant reaction mixture was poured into 1 liter of methanol, and the precipitated tar was then separated/purified by column chromatography to obtain 0.5 g of the following formula (1-2), 0.1 g of (2-2), 5.1 g of (3-2) and 0.5 g of (4-2). Physical properties of these compounds are set forth in Table 2.

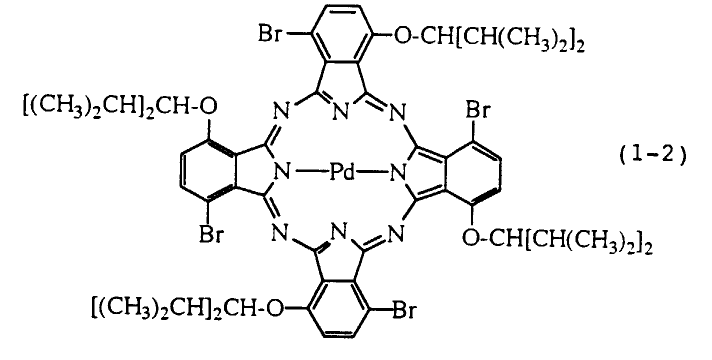

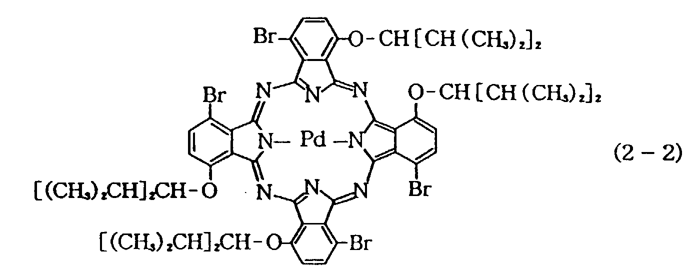

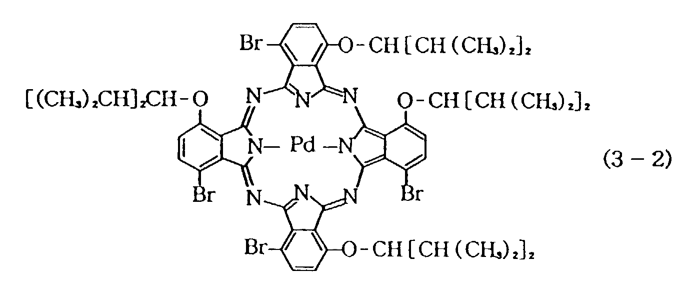

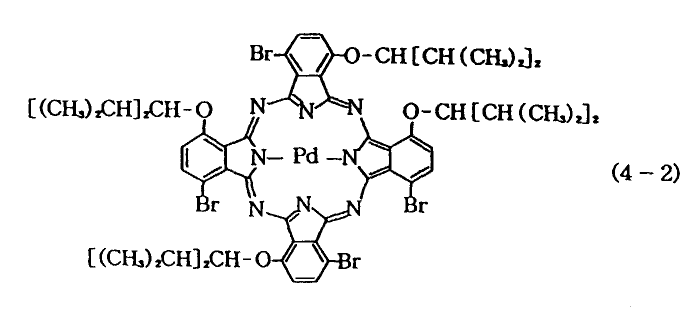

**Table 2**

| Compound No. | λₘₐₓ(nm) (εₘₐₓ×10⁻⁵) | mass (m/e) | Elemental Analysis (C,H,N) Found (Calcd.) |
|---|---|---|---|
| (1-2) | 716 | 1391 | 51.79, 4.92, 8.04 |
| | (2.2) | | (51.80, 4.93, 8.05) |
| (2-2) | 715 | 1391 | 51.81, 4.90, 8.06 |
| | (2.2) | | (51.80, 4.93, 8.05) |
| (3-2) | 716 | 1391 | 51.82, 4.92, 8.06 |
| | (2.1) | | (51.80, 4.93, 8.05) |
| (4-2) | 715 | 1391 | 51.79, 4.90, 8.04 |
| | (2.2) | | (51.80, 4.93, 8.05) |

### Example 3

Five grams of phthalonitrile represented by said formula (5-1), 5 g of phthalonitrile represented by the following formula (5-2), 2 g of palladium chloride, 4 g of DBU and 200 g of n-amyl alcohol were mutually mixed, and reaction was then carried out at 95°C for 4 hours.

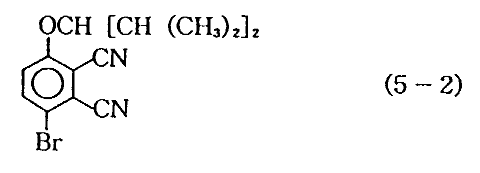

The resultant reaction mixture was poured into 1 liter of methanol, and the precipitated tar was then separated/purified by column chromatography to obtain 0.1 g of the following formula (1-3), 0.1 g of (1-4), 0.5 g of (1-5) and 0.3 g of (1-6). Physical properties of these compounds are set forth in Table 3.

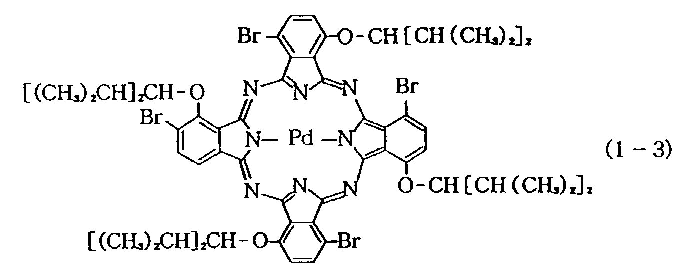

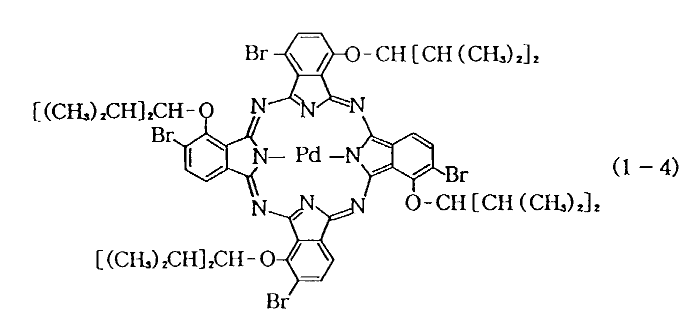

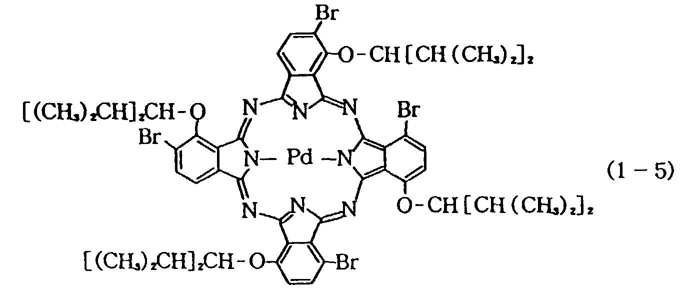

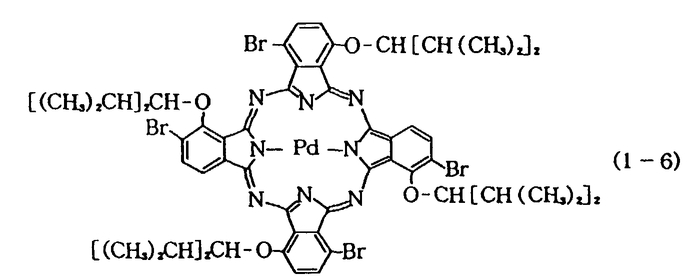

**Table 3**

| Compound No. | λₘₐₓ(nm) (εₘₐₓ×10⁻⁵) | mass (m/e) | Elemental Analysis (C,H,N) Found (Calcd.) |
|---|---|---|---|
| (1-3) | 716 | 1391 | 51.67, 4.95, 8.10 |
| | (2.2) | | (51.80, 4.93, 8.05) |
| (1-4) | 716 | 1391 | 51.90, 4.88, 7.99 |
| | (2.2) | | (51.80, 4.93, 8.05) |
| (1-5) | 716 | 1391 | 51.85, 4.87, 8.11 |
| | (2.2) | | (51.80, 4.93, 8.05) |
| (1-6) | 715 | 1391 | 51.78, 4.87, 7.99 |
| | (2.2) | | (51.80, 4.93, 8.05) |

### Example 4

Eight grams of phthalonitrile represented by said formula (5-1), 2 g of phthalonitrile represented by the following formula (5-3), 2 g of palladium chloride, 4 g of DBU and 300 g of n-amyl alcohol were mutually mixed, and reaction was then carried out at 95°C for 24 hours.

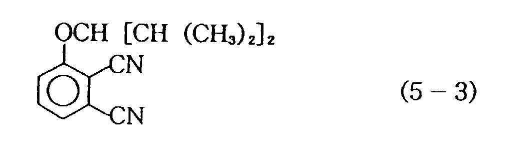

The resultant reaction mixture was poured into 1 liter of methanol, and the precipitated tar was then separated/purified by column chromatography to obtain 0.2 g of the following formula (3-3), 0.5 g of (3-4), 0.3 g of (3-5), 0.2 g of (1-7), 0.2 g of (1-1) and 0.2 of (3-1). Physical properties of these compounds are set forth in Table 4.

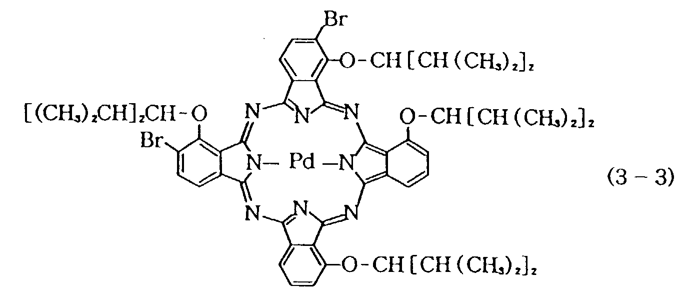

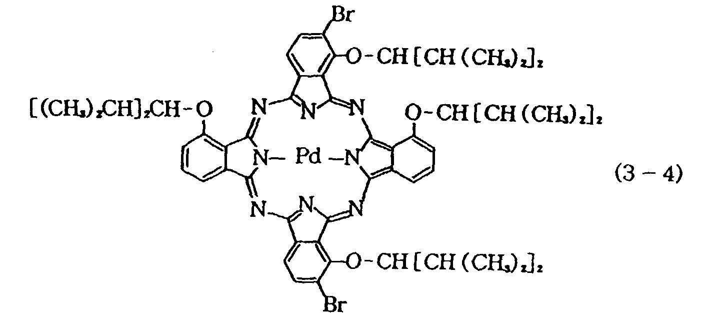

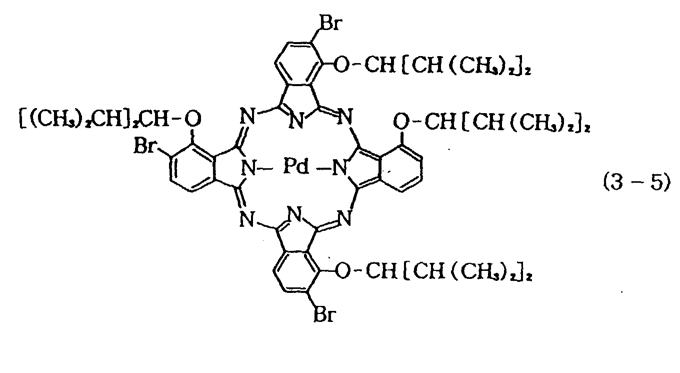

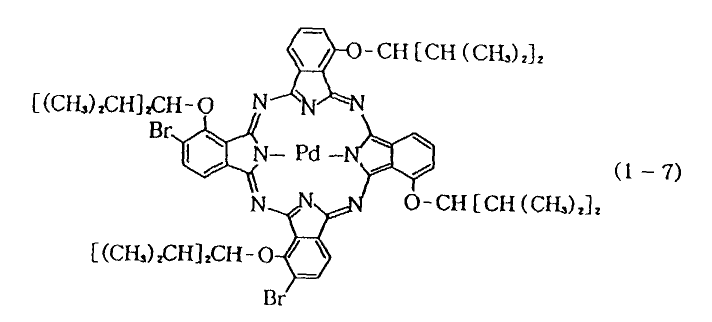

**Table 4**

| Compound No. | λₘₐₓ(nm) (εₘₐₓ×10⁻⁵) | mass (m/e) | Elemental Analysis (C,H,N) Found (Calcd.) |
|---|---|---|---|
| (3-3) | 700 | 1233 | 58.22, 5.90, 9.10 |
| | (2.2) | | (58.42, 5.72, 9.08) |
| (3-4) | 700 | 1233 | 58.01, 5.87, 9.11 |
| | (2.3) | | (58.42, 5.72, 9.08) |
| (3-5) | 706 | 1312 | 54.78, 5.57, 8.38 |
| | (2.2) | | (54.91, 5.30, 8.54) |
| (1-7) | 699 | 1233 | 58.23, 5.59, 8.98 |
| | (2.2) | | (58.42, 5.72, 9.08) |

### Example 5

Seven grams of diiminoisoindoline represented by said formula (6-1), 3 g of diiminoisoindoline represented by the following formula (6-2), 2 g of palladium chloride, 4 g of DBU and 300 g of n-octyl alcohol were mutually mixed, and reaction was then carried out under reflux for 4 hours.

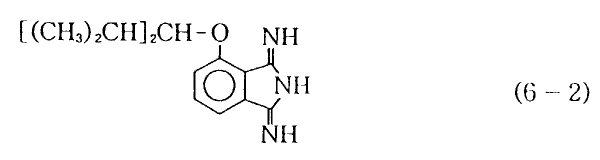

The resultant reaction mixture was poured into 1 liter of methanol, and the precipitated tar was then separated/purified by column chromatography to obtain 0.4 g of the following formula (3-6), 0.3 g of (1-8), 0.2 g of (3-7), 0.2 g of (3-8), 0.2 g of (1-9), 0.1 g of (3-9), 0.2 g of (1-10), 0.2 g of (3-2) and 0.2 g of (1-2). Physical properties of these compounds are set forth in Table 5.

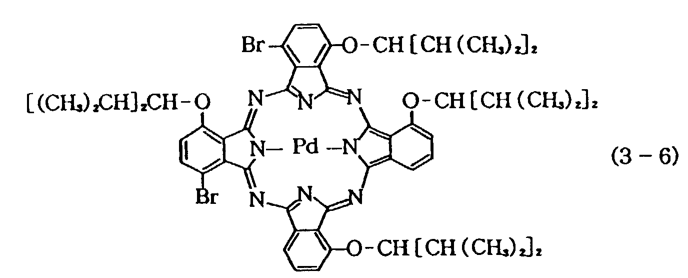

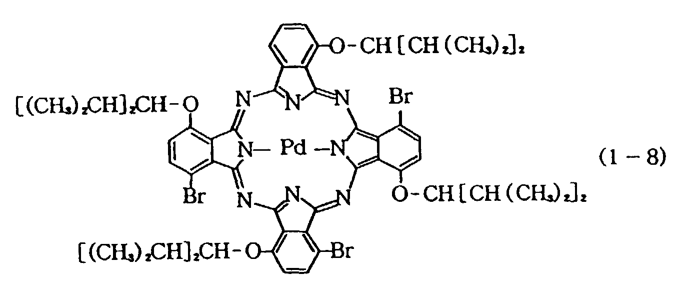

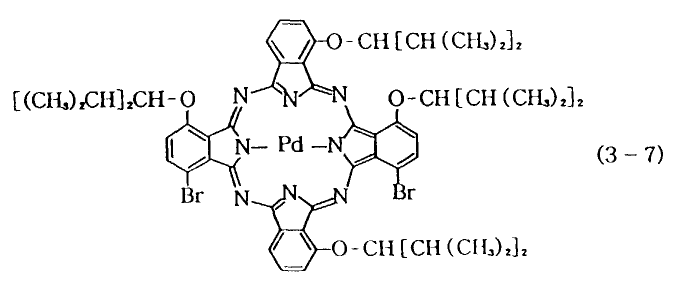

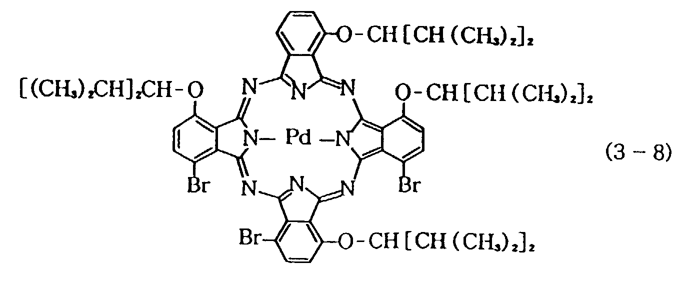

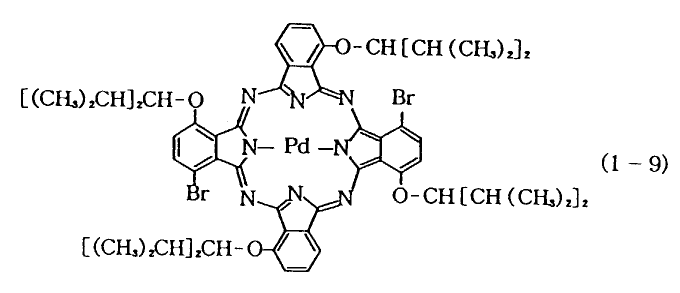

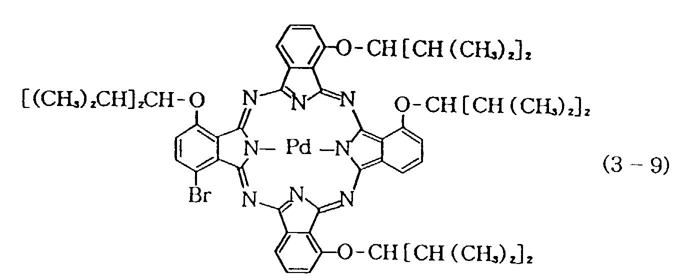

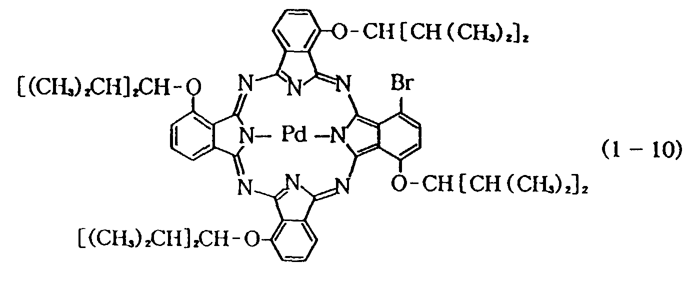

**Table 5**

| Compound No. | λₘₐₓ(nm) (εₘₐₓ×10⁻⁵) | mass (m/e) | Elemental Analysis (C,H,N) Found (Calcd.) |
|---|---|---|---|
| (3-6) | 698 | 1233 | 58.31, 5.87, 9.01 |
| | (2.3) | | (58.42, 5.72, 9.08) |
| (1-8) | 705 | 1312 | 54.98, 5.28, 8.61 |
| | (2.2) | | (54.91, 5.30, 8.54) |
| (3-7) | 699 | 1233 | 58.52, 5.66, 9.03 |
| | (2.3) | | (58.42, 5.72, 9.08) |
| (3-8) | 707 | 1312 | 55.01, 5.27, 8.48 |
| | (2.2) | | (54.91, 5.30, 8.54) |
| (1-9) | 698 | 1233 | 58.33, 5.79, 9.15 |
| | (2.2) | | (58.42, 5.72, 9.08) |
| (3-9) | 694 | 1154 | 62.21 6.27, 9.48 |
| | (2.3) | | (62.41, 6.20, 9.70) |
| (1-10) | 695 | 1154 | 62.63, 6.79, 9.15 |
| | (2.2) | | (62.41, 6.20, 9.70) |

### Example 6

Seven grams of diiminoisoindoline represented by the following formula (6-3), 2 g of diiminoisoindoline represented by the following formula (6-4), 2 g of palladium chloride, 4 g of DBU and 300 g of n-octyl alcohol were mutually mixed, and reaction was then carried out at 175°C for 12 hours.

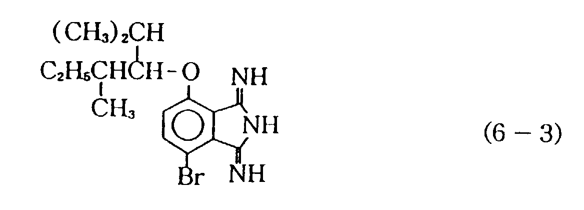

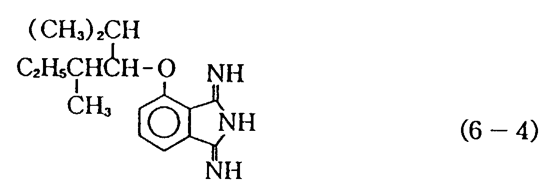

The resultant reaction mixture was poured into 1 liter of methanol, and the precipitated tar was then separated/purified by column chromatography to obtain 0.3 g of the following formula (1-11), 0.4 g of (4-3), 0.2 g of (3-10), 0.2 g of (3-11), 0.2 g of (4-4), 0.1 g of (1-12), 0.2 g of (4-5), 0.2 g of (3-12) and 0.2 g of (4-6). Physical properties of these compounds are set forth in Table 6.

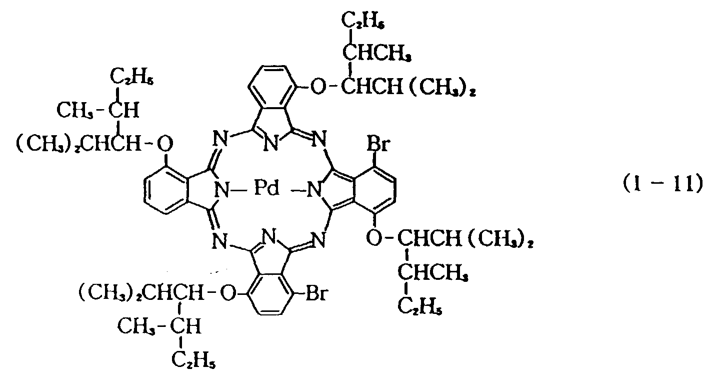

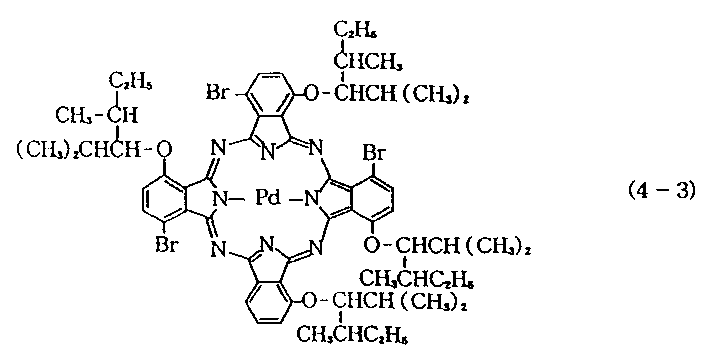

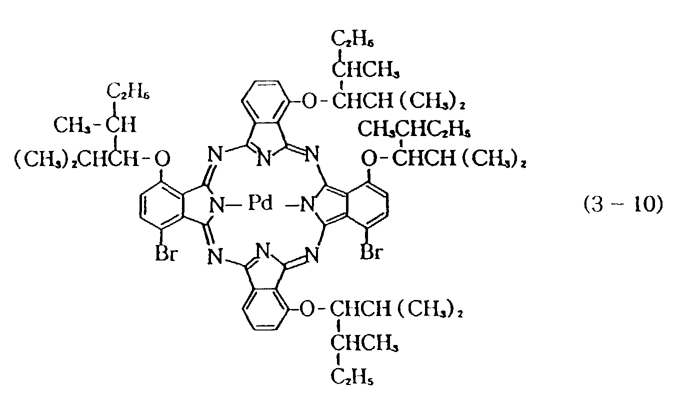

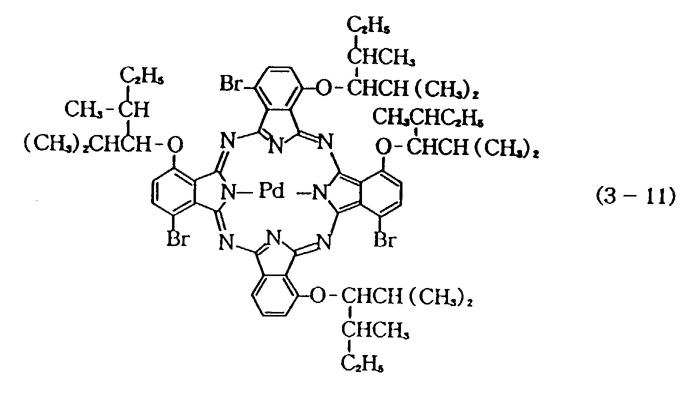

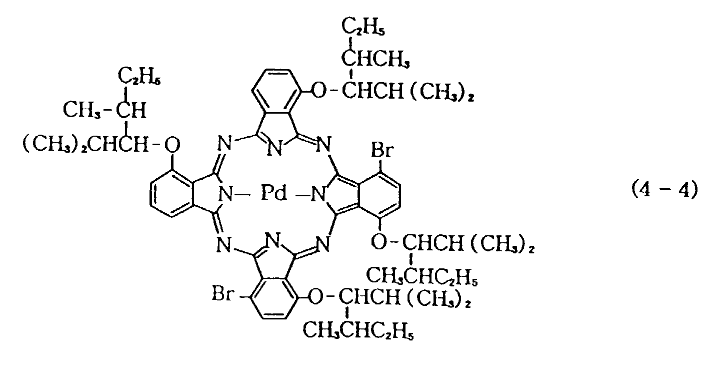

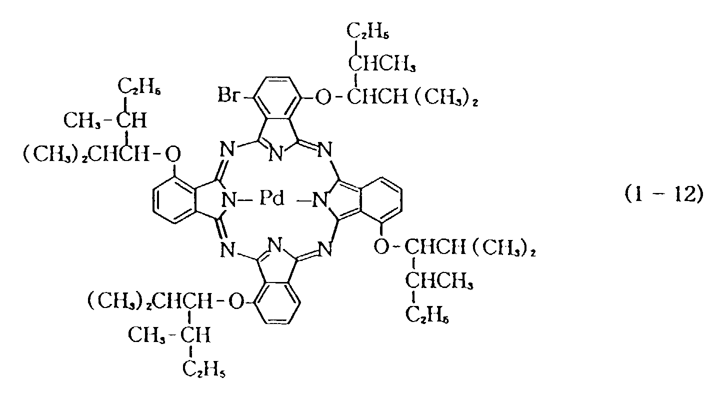

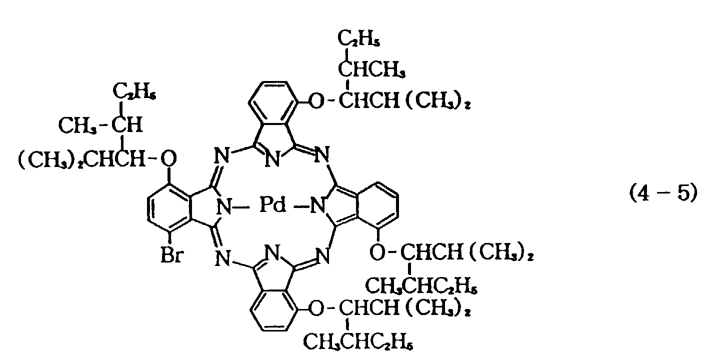

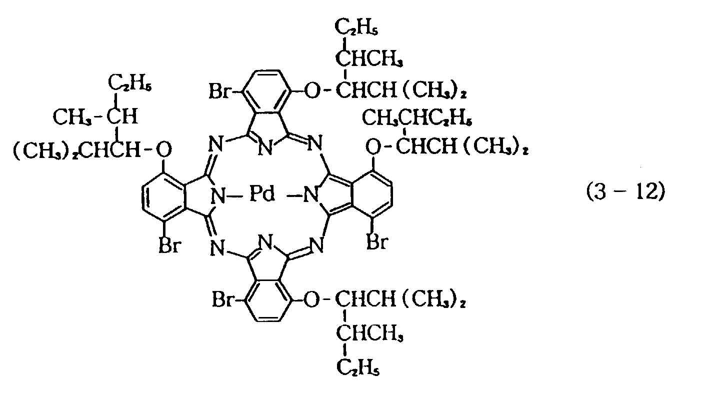

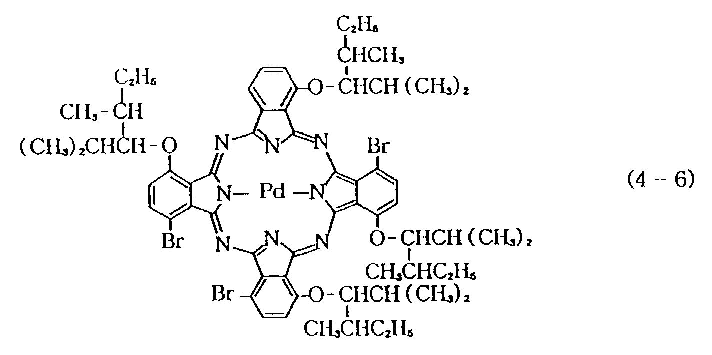

**Table 6**

| Compound No. | λₘₐₓ(nm) (εₘₐₓ×10⁻⁵) | mass (m/e) | Elemental Analysis (C,H,N) Found (Calcd.) |
|---|---|---|---|
| (1-11) | 698 | 1289 | 59.11, 5.97, 8.51 |
| | (2.3) | | (59.61, 6.10, 8.69) |
| (4-3) | 704 | 1368 | 55.98, 5.28, 8.32 |
| | (2.2) | | (56.16, 5.67, 8.19) |
| (3-10) | 700 | 1289 | 59.52, 5.96, 8.73 |
| | (2.3) | | (59.61, 6.10, 8.69) |
| (3-11) | 707 | 1368 | 56.01, 5.27, 8.48 |
| | (2.2) | | (56.16, 5.67, 8.19) |
| (4-4) | 699 | 1289 | 59.33, 5.99, 8.45 |
| | (2.2) | | (59.61, 6.10, 8.69) |
| (1-12) | 694 | 1210 | 63.21 6.47, 9.38 |
| | (2.3) | | (63.49, 6.58, 9.26) |
| (4-5) | 694 | 1210 | 63.63, 6.79, 9.15 |
| | (2.2) | | (63.49, 6.58, 9.26) |
| (3-12) | 715 | 1447 | 53.21, 5.47, 7.38 |
| | (2.1) | | (53.11, 5.29, 7.74) |
| (4-6) | 714 | 1447 | 53.02, 5.18, 7.81 |
| | (2.1) | | (53.11, 5.29, 7.74) |

### Example 7

Seven grams of diiminoisoindoline represented by the following formula (6-5), 3 g of diiminoisoindoline represented by the following formula (6-6), 2 g of palladium chloride, 4 g of DBU and 300 g of n-octyl alcohol were mutually mixed, and reaction was then carried out at 175°C for 12 hours.

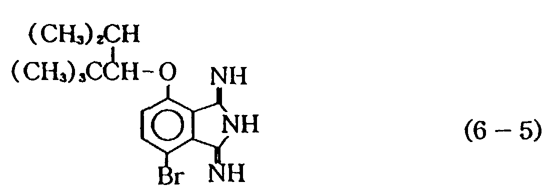

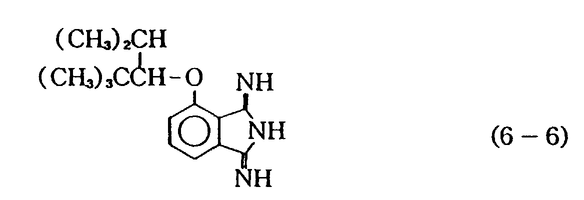

The resultant reaction mixture was poured into 1 liter of methanol, and the precipitated tar was then separated/purified by column chromatography to obtain 0.2 g of the following formula (1-13), 0.4 g of (1-14), 0.1 g of (4-7), 0.1 g of (4-8), 0.2 g of (3-13), 0.1 g of (3-14), 0.2 g of (4-9), 0.2 g of (1-15) and 0.2 g of (3-15). Physical properties of these compounds are set forth in Table 7.

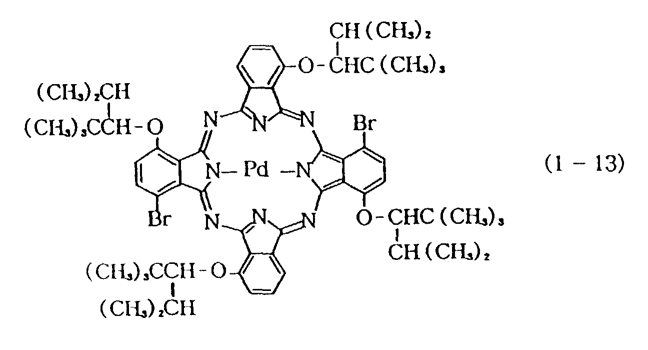

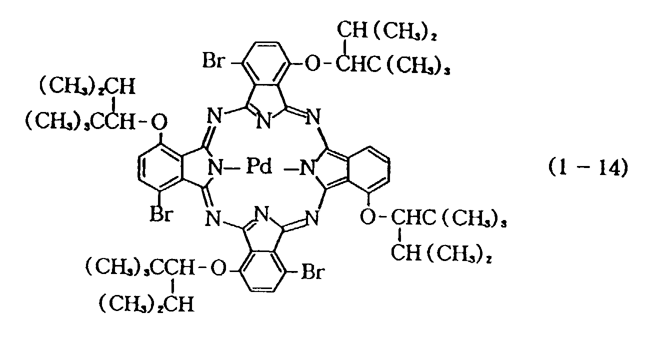

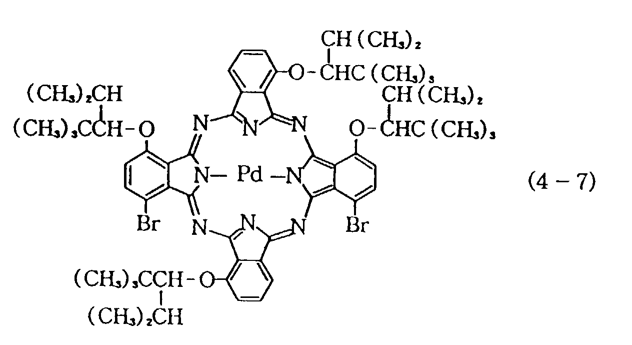

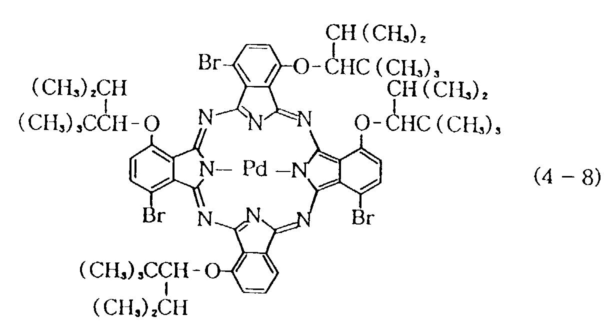

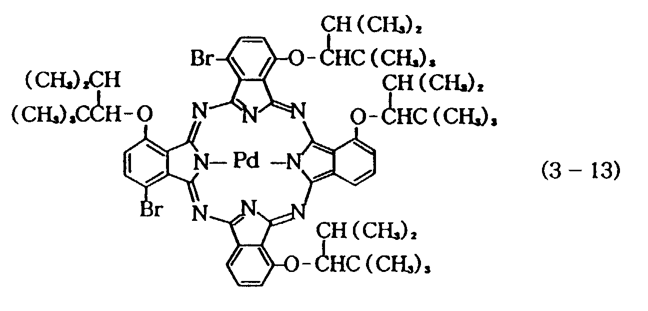

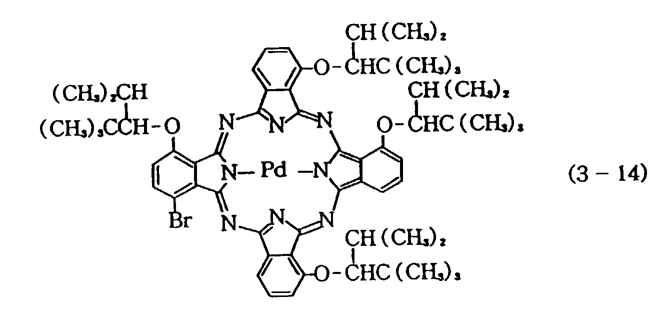

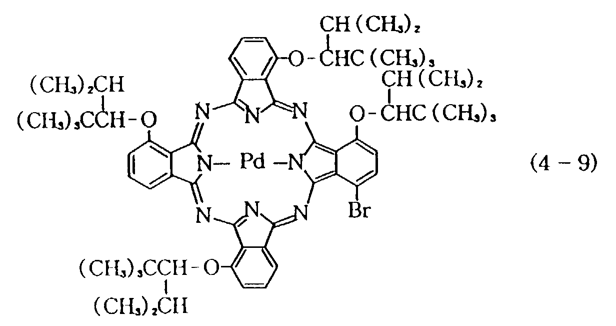

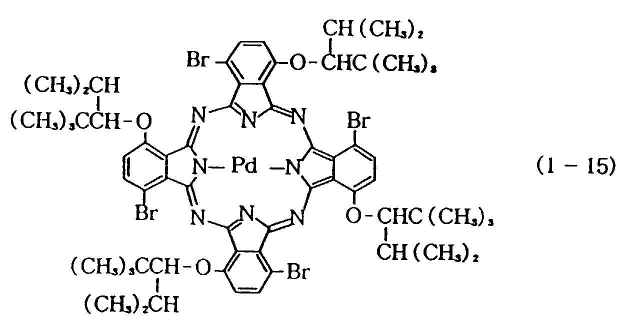

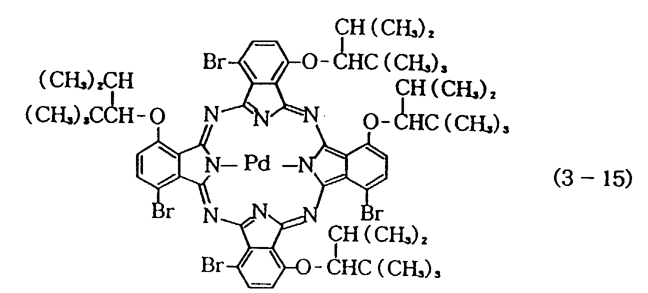

**Table 7**

| Compound No. | λₘₐₓ(nm) (εₘₐₓ×10⁻⁵) | mass (m/e) | Elemental Analysis (C,H,N) Found (Calcd.) |
|---|---|---|---|
| (1-13) | 702 | 1289 | 59.11, 5.97, 8.51 |
| | (2.3) | | (59.61, 6.10, 8.69) |
| (1-14) | 709 | 1364 | 55.98, 5.28, 8.32 |
| | (2.2) | | (56.16, 5.67, 8.19) |
| (4-7) | 705 | 1289 | 59.52, 5.96, 8.73 |
| | (2.3) | | (59.61, 6.10, 8.69) |
| (4-8) | 709 | 1391 | 56.01, 5.27, 8.48 |
| | (2.2) | | (56.16, 5.67, 8.19) |
| (3-13) | 703 | 1289 | 59.33, 5.99, 8.45 |
| | (2.2) | | (59.61, 6.10, 8.69) |
| (3-14) | 697 | 1210 | 63.21 6.47, 9.38 |
| | (2.3) | | (63.49, 6.58, 9.26) |
| (4-9) | 695 | 1210 | 63.63, 6.79, 9.15 |
| | (2.2) | | (63.49, 6.58, 9.26) |
| (1-15) | 718 | 1447 | 53.21, 5.47, 7.38 |
| | (2.1) | | (53.11, 5.29, 7.74) |
| (3-15) | 719 | 1447 | 53.02, 5.18, 7.81 |
| | (2.1) | | (53.11, 5.29, 7.74) |

### Comparative Example 1

Thirty grams of a compound represented by the above-mentioned formula (1-3) were dissolved in 1 liter of dimethylcyclohexane, and the resultant solution was then applied as thick as 120 nm on a polycarbonate substrate having grooves by a spin coating method. Next, gold was sputtered so as to have a thickness of 100 nm. Successively, an ultraviolet-curing resin was subjected to spin coating and then cured with ultraviolet rays. Its thickness was 6 µm. When the thus prepared optical recording medium for CD-WO was used to carry out recording at a linear velocity of 1.4 m/sec at an optimum power (8 mW) by the use of a laser beam of 780 nm, a record of 60 dB was obtained and recording properties (the symmetry, jitter and cross talk of signals) were also excellent. Reflectance was 69%. The refractive index of this recording layer was 2.12 at 780 nm.

### Comparative Examples 2 to 8

The same procedure as in Comparative Example 1 was effected using the compounds obtained the preceding examples, to prepare optical recording media for CD-WO. For the thus obtained optical recording media, optimum power, recording sensitivity, symmetry, jitter and cross talk were inspected. The results are set forth in Table 8. Incidentally, reflectances all were between 65 and 72%.

**Table 8**

| Comparative Example | Compound | Optimum Power (mW) | Record (dB) | Symmetry | Jitter | Cross Talk |
|---|---|---|---|---|---|---|
| 1 | 2-1 | 7.8 | 62 | O | O | O |
| 2 | 3-2 | 8.1 | 63 | O | O | O |
| 3 | 3-4 | 6.7 | 61 | O | O | O |
| 4 | 1-9 | 7.3 | 60 | O | O | O |
| 5 | 4-3 | 7.1 | 62 | O | O | O |
| 6 | 3-11 | 7.3 | 61 | O | O | O |
| 7 | 4-5 | 7.5 | 61 | O | O | O |
| 8 | 3-14 | 6.9 | 61 | O | O | O |

### Example 8

Fifteen grams of a mixture of palladium tetraα-(1,3-dimethylbutyloxy)phthalocyanines in which the ratio of the following formulae (10-1), (11-1), (9-1) and (12-1) was 20:30:30:20 were added to a mixed solvent of 50 g (38 ml) of dichloromethane, 50 g (76 ml) of n-hexane and 100 g (100 ml) of water.

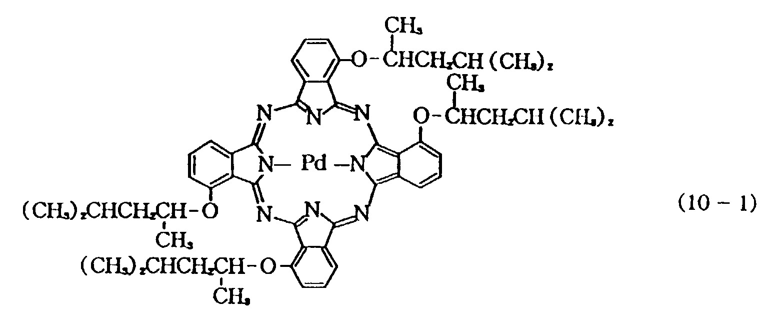

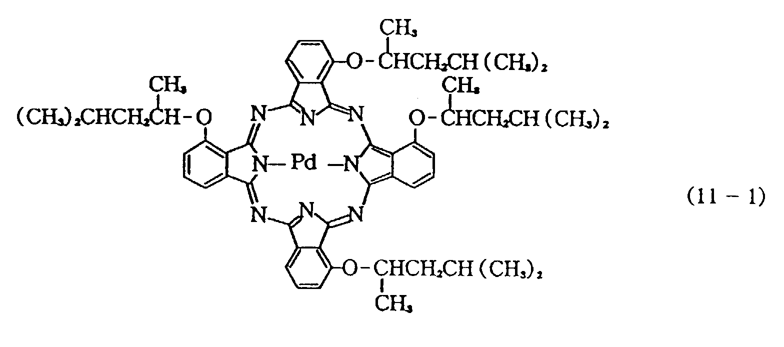

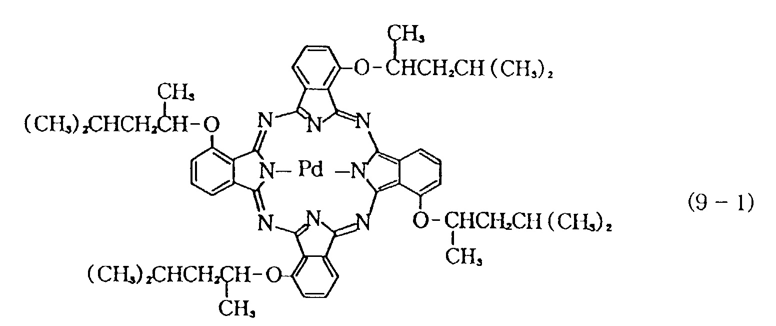

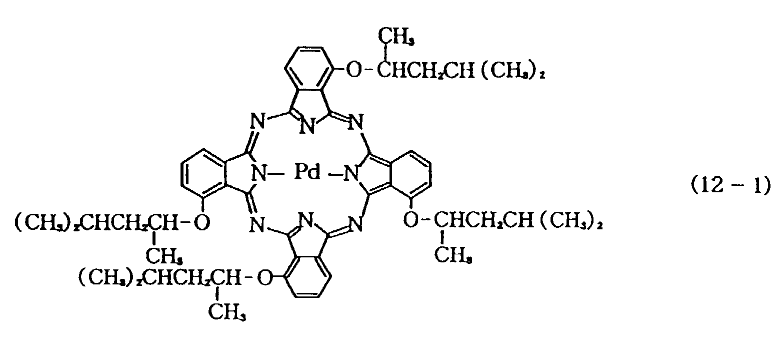

Furthermore, 9.5 g of bromine were added thereto, and reaction was then carried out at 40°C for 2 hours. After cooling to 20°C, 50 g of toluene were added, followed by separation. Successively, the resultant organic solvent layer was washed with 100 g of a 10% aqueous sodium hydrogensulfite solution and 100 g of a 5% aqueous sodium hydrogencarbonate solution. The organic solvent was distilled off, and separation was then carried out by toluene-silica gel chromatography to obtain 16 g of a mixture of brominated phthalocyanines represented by the following formulae (2-3), (3-16), (1-16) and (4-10). The maximum absorption wave length λₘₐₓ of the mixture was 700 nm and εₘₐₓ was 1.6 × 10⁵g⁻¹cm².

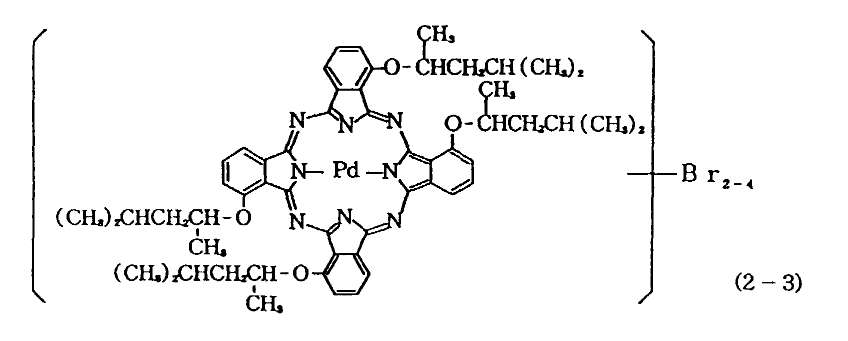

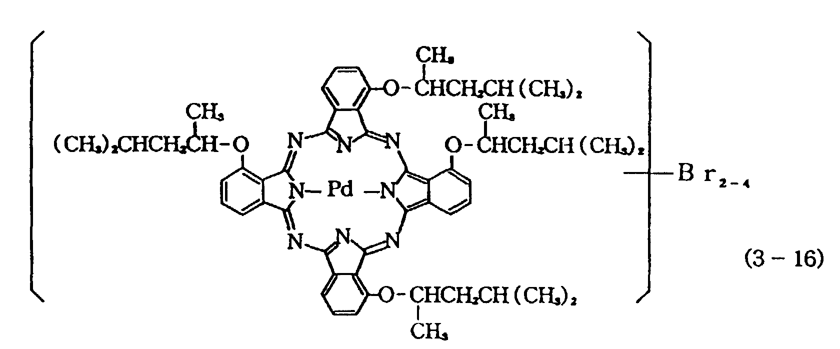

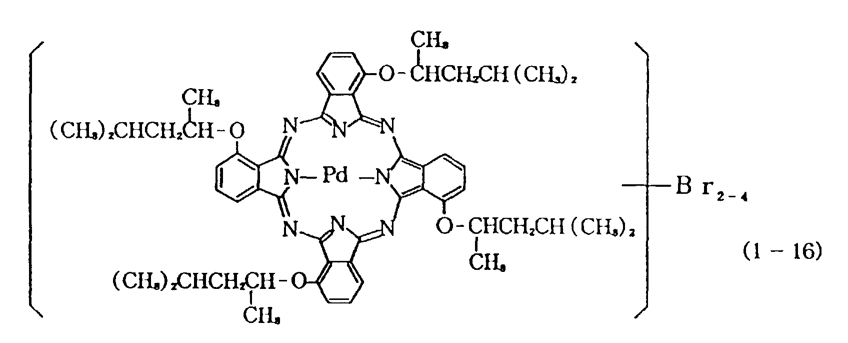

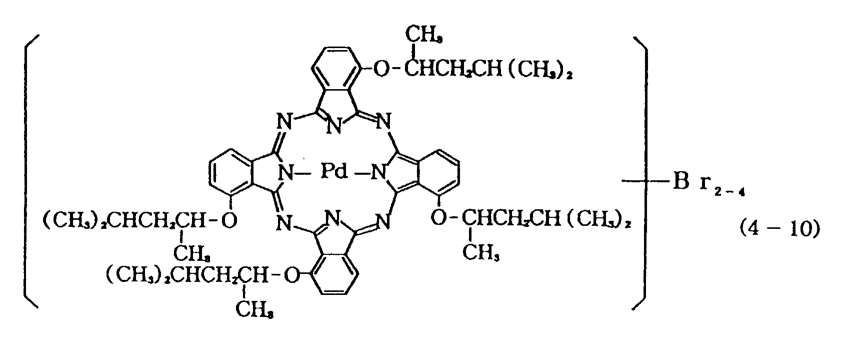

### Example 9

Fifteen grams (13.25 mmol) of a mixture of palladium tetraα-(1-iso-propyl-3-methylbutyloxy)phthalocyanines in which ratio of the following formulae (10-2), (11-2), (9-2) and (12-2) was 20:30:30:20 were added to a mixed solvent of 50 g (56 ml) of tetrahydrofuran, 50 g (76 ml) of n-hexane and 100 g (100 ml) of water.

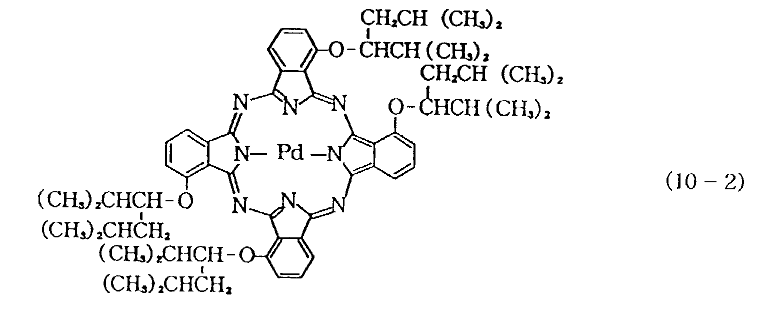

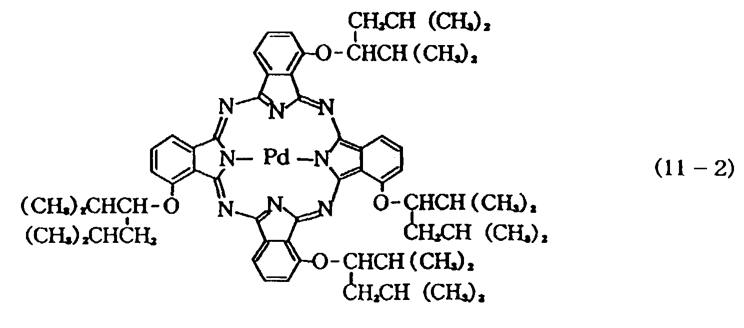

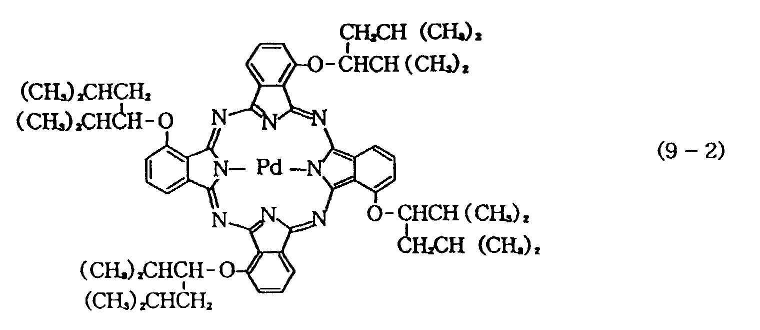

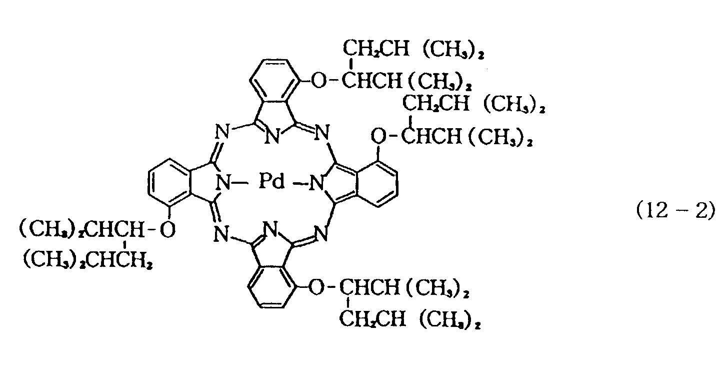

Furthermore, 15 g (93.86 mmol) of bromine were added thereto, and reaction was then carried out at 40°C for 2 hours. After cooling to 20°C, 50 g of toluene were added, followed by separation. Successively, the resultant organic solvent layer was washed with 100 g of a 10% aqueous sodium hydrogensulfite solution and 100 g of a 5% aqueous sodium hydrogencarbonate solution. The organic solvent was distilled off, and separation was then carried out by toluene-silica gel chromatography to obtain 15 g of a mixture of brominated phthalocyanines represented by the following formulae (2-4), (3-17), (1-17) and (4-11).

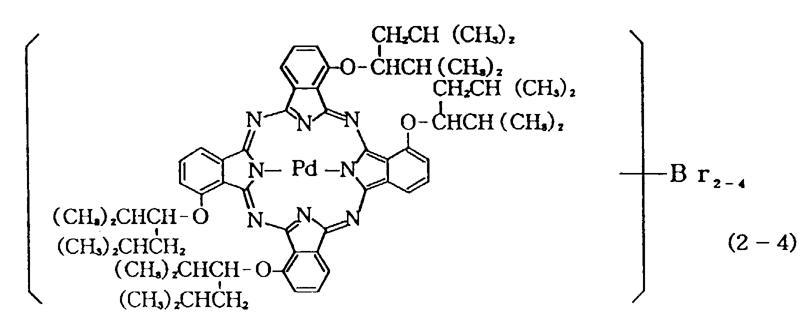

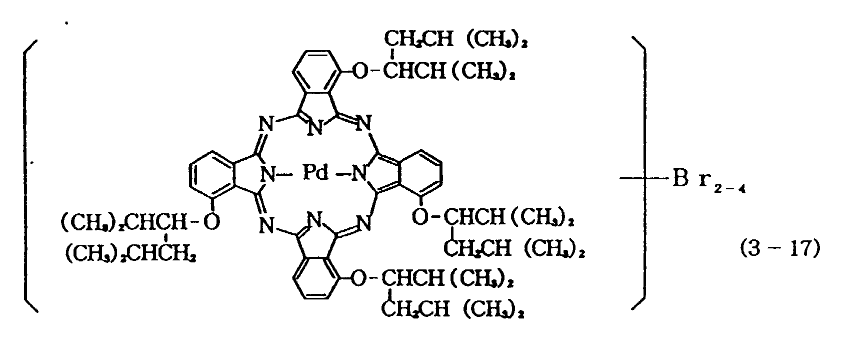

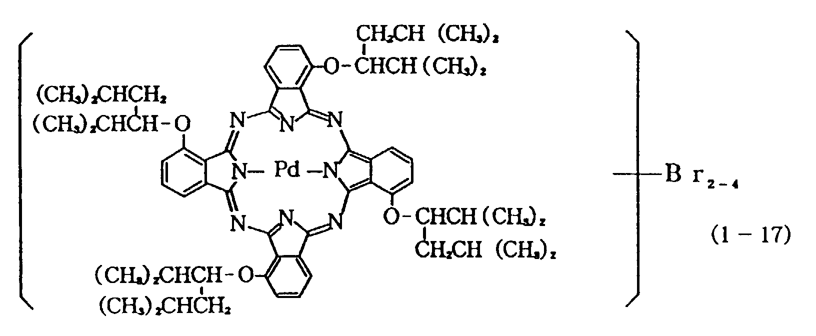

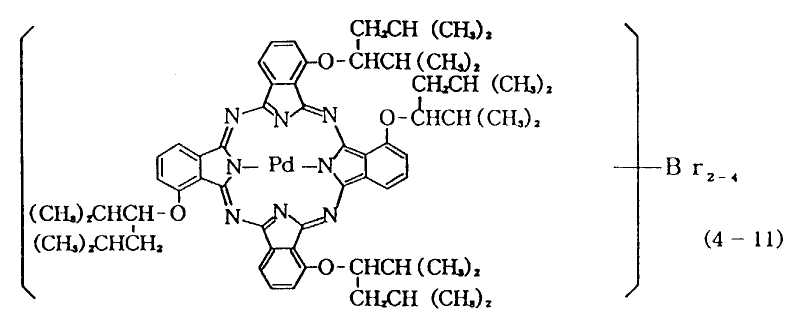

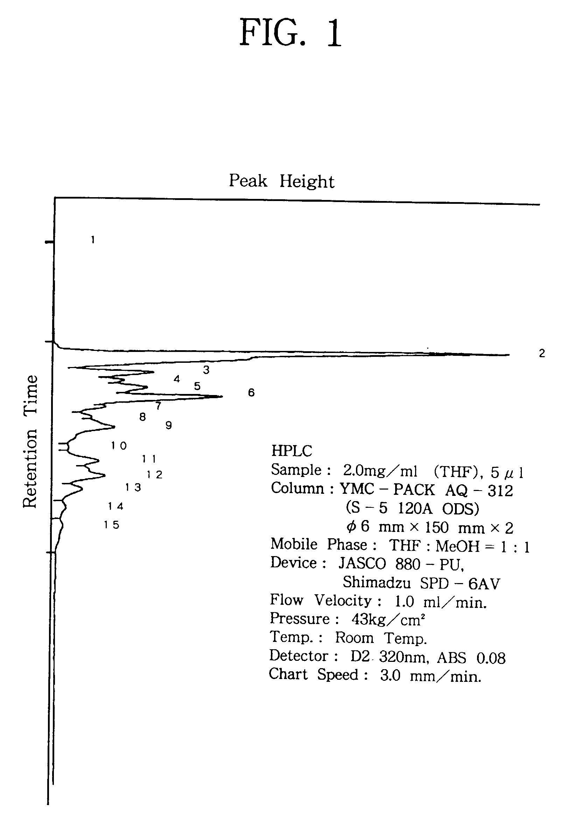

The liquid chromatogram of this mixture is shown in Fig. 1. A retention time and concentration of each peak are set forth in Table 10. The maximum absorption wave length λₘₐₓ of the mixture was 706 nm and εₘₐₓ was 1.4 × 10⁵g⁻¹cm², and a melting point was 149-72°C.

**Table 10**

| Peak No. | Retention Time | Concentration |
|---|---|---|
| 1 | 5.892 | 0.004 |
| 2 | 16.145 | 35.3123 |
| 3 | 17.7 | 6.9134 |
| 4 | 18.345 | 3.7454 |
| 5 | 19.048 | 8.3346 |
| 6 | 19.972 | 13.2588 |
| 7 | 20.715 | 3.1194 |
| 8 | 21.613 | 2.224 |
| 9 | 22.55 | 8.873 |
| 10 | 24.283 | 0.9399 |
| 11 | 25.573 | 4.6923 |
| 12 | 26.915 | 6.4882 |
| 13 | 28.083 | 3.1528 |
| 14 | 29.717 | 1.5366 |
| 15 | 31.382 | 1.4054 |
| | Total | 100 |

### Example 10

Fifty grams (46.48 mmol) of a mixture of palladium tetraα-(1-iso-propyl-2-methylpropyloxy)phthalocyanines in which the ratio of the following formulae (10-3), (11-3), (9-3) and (4-3) was 2:80:15:3 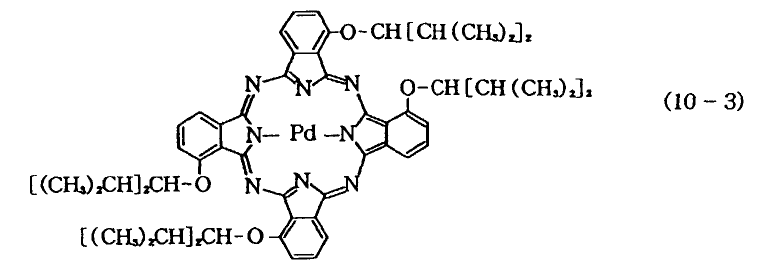 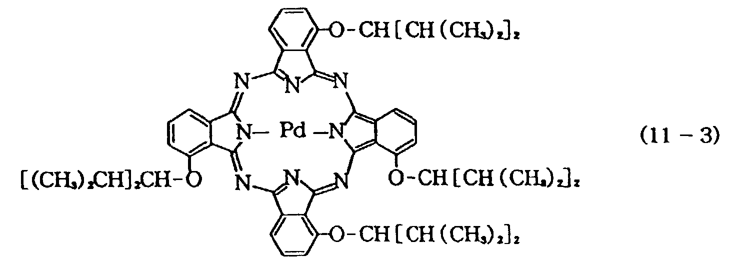 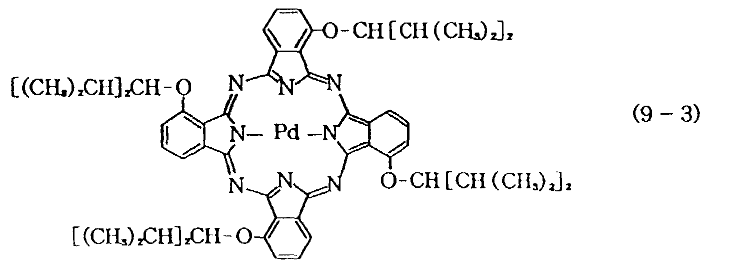 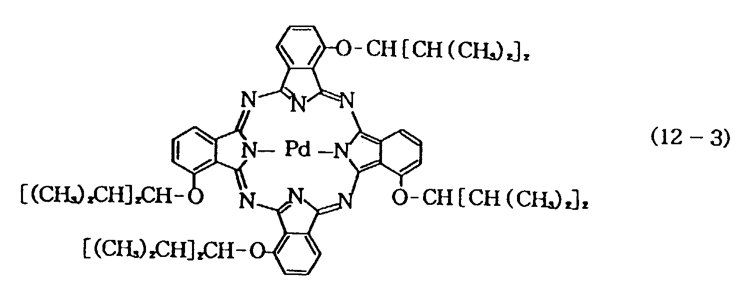 were dissolved in 300 g (208 ml) of 1,1,2-trichloroethane, and 100 g (100 ml) of water were added thereto. Next, a mixture of 22.7 g (142.03 mmol) of bromine and 63 g (44 ml) of 1,1,2-trichloroethane was added dropwise at 50 to 55°C, and reaction was then carried out at 55 to 60°C for 1 hour. Afterward, 50 g of a 15% aqueous sodium hydrogensulfite solution were added, followed by washing. The resultant organic layer was added dropwise to 800 g of methanol, and the precipitated crystals were filtered, thereby obtaining 59.8 g of a mixture of brominated phthalocyanines represented by the formulae (2-5), (3-18), (1-18) and (4-12).

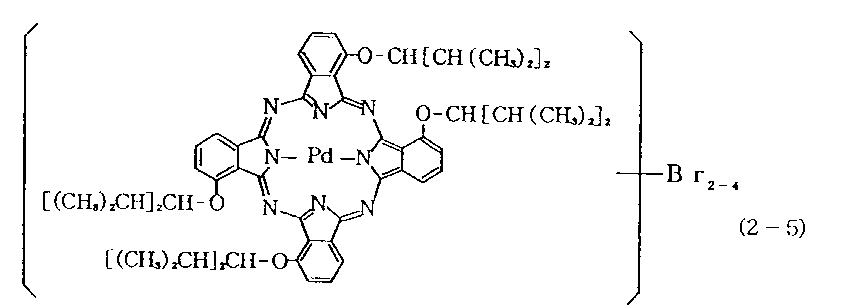

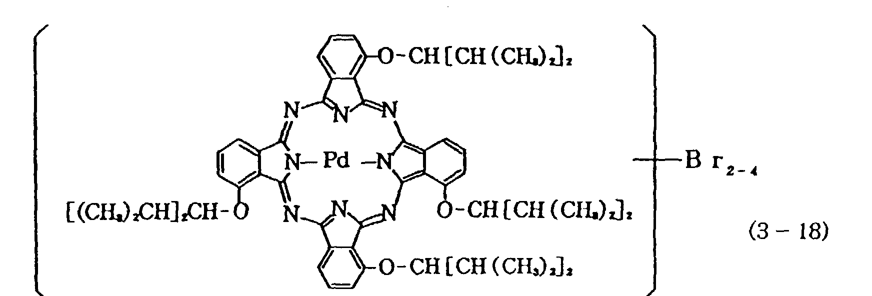

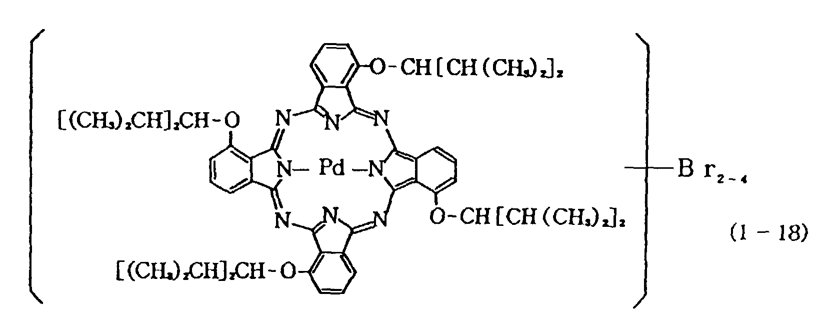

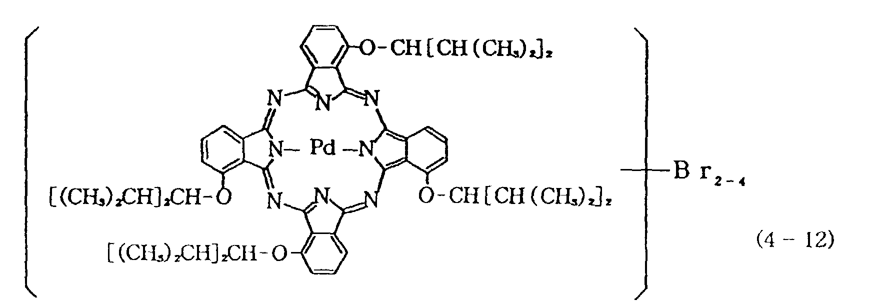

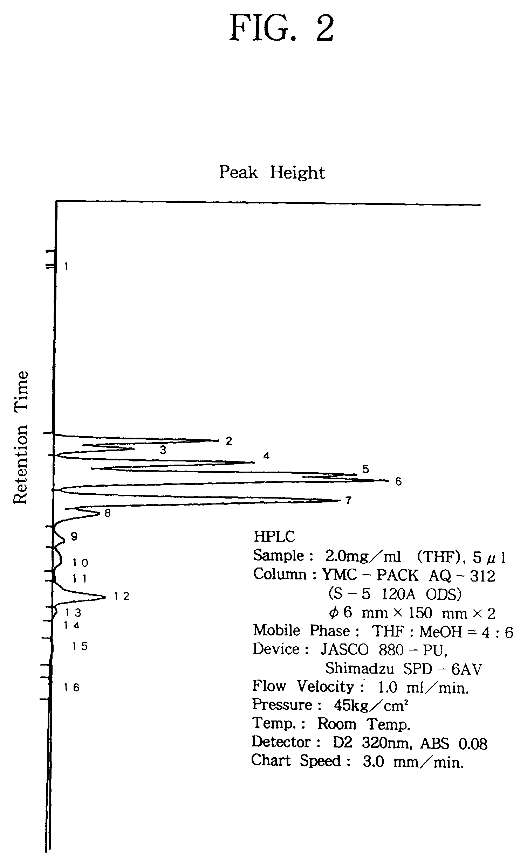

The liquid chromatogram of the mixture is shown in Fig. 2. A retention time and concentration of each peak are set forth in Table 11. The maximum absorption wave length λₘₐₓ of the mixture was 706 nm, εₘₐₓ was 1.6 × 10⁵g⁻¹cm², and a melting point was 215-45°C.

**Table 11**

| Peak No. | Retention Time | Concentration |
|---|---|---|
| 1 | 7.408 | 0.0137 |
| 2 | 23.008 | 8.5536 |
| 3 | 23.722 | 4.2271 |
| 4 | 24.992 | 12.3603 |
| 5 | 26.045 | 17.6083 |
| 6 | 26.553 | 19.6252 |
| 7 | 28.41 | 22.9657 |
| 8 | 29.59 | 4.299 |
| 9 | 32.058 | 1.4879 |
| 10 | 33.923 | 1.719 |
| 11 | 35.255 | 0.3461 |
| 12 | 37.122 | 5.5754 |
| 13 | 38.325 | 0.5243 |
| 14 | 39.858 | 0.234 |
| 15 | 41.59 | 0.3649 |
| 16 | 45.258 | 0.0957 |
| | Total | 100 |

### Example 11

Fifteen grams (13.25 mmol) of a mixture of palladium tetraα-(2-ethylhexyloxy)phthalocyanines in which the ratio of the following formulae (10-4), (11-4), (9-4) and (12-4) was 10:40:30:20 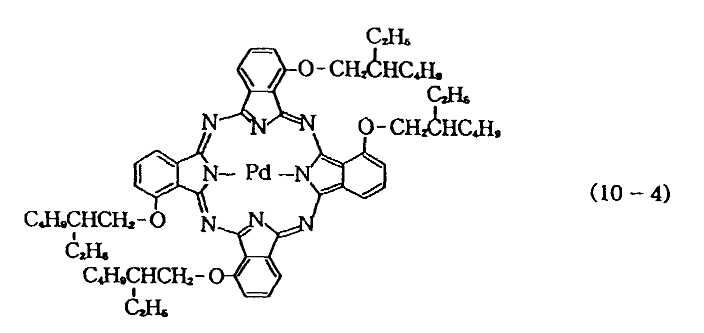 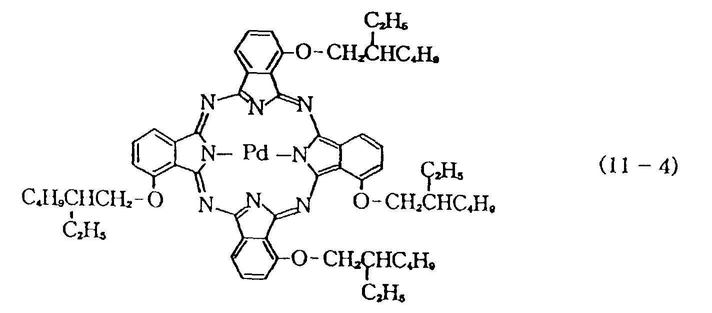 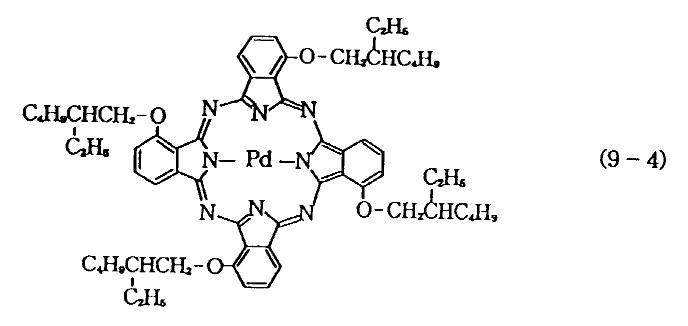 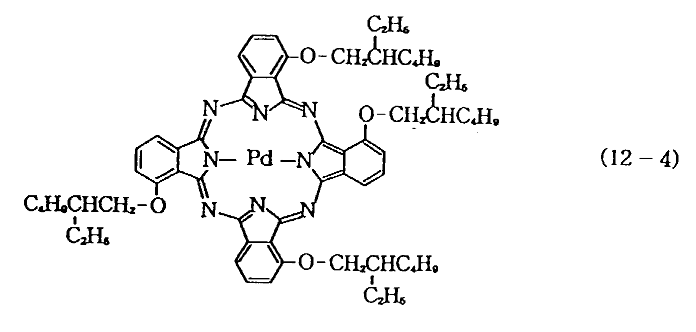 were added to a mixed solvent of 50 g (31 ml) of 1,1,2,2-tetrachloroethane, 50 g (63 ml) of ethylcyclohexane and 100 g (100 ml) of water. Next, a mixture of 6.2 g (39.42 mmol) of bromine was added, and reaction was then carried out at 60°C for 2 hours. After cooling to 20°C, 50 g of toluene were added, followed by separation. Successively, the resultant organic solvent layer was washed with 100 g of a 10% aqueous sodium hydrogensulfite solution and 100 g of a 5% aqueous sodium hydrogencarbonate solution. The organic solvent was distilled off, and separation was then carried out by toluene-silica gel chromatography to obtain 16 g of a mixture of brominated phthalocyanines represented by the following formulae (2-6), (3-19), (1-19) and (4-13).

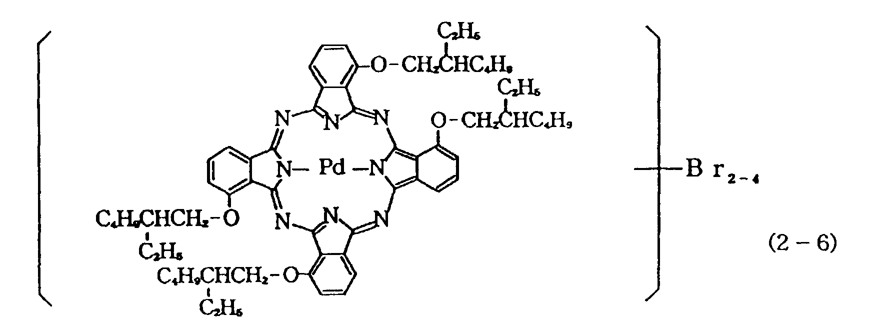

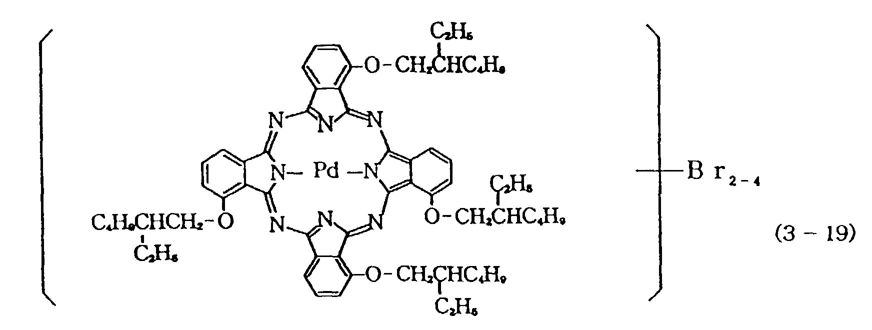

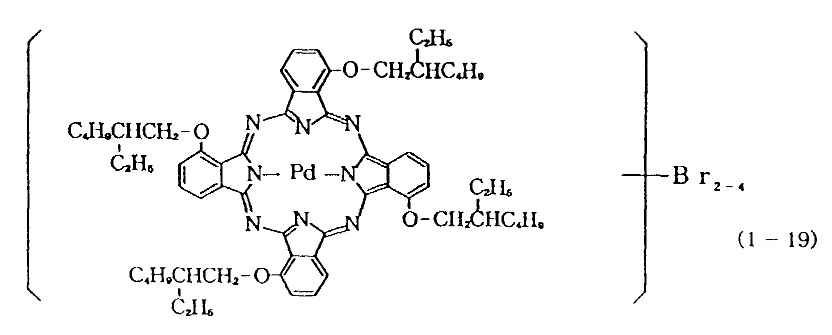

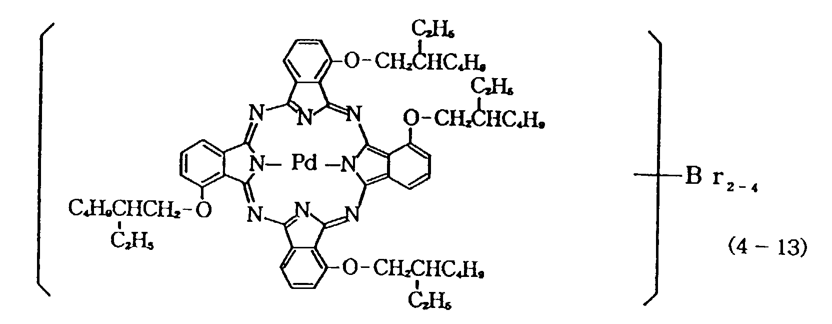

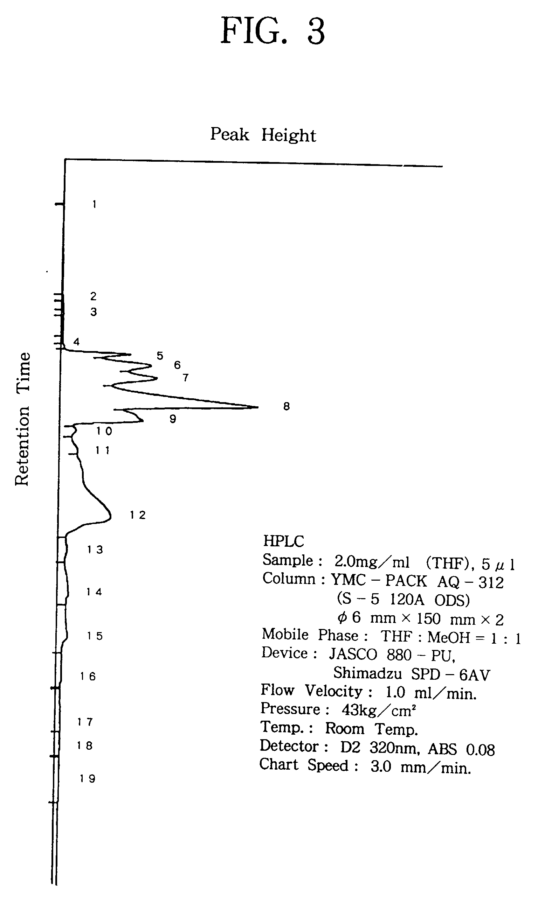

The liquid chromatogram of the mixture is shown in Fig. 3. A retention time and concentration of each peak are set forth in Table 12. The maximum absorption wave length λₘₐₓ of the mixture was 695 nm, εₘₐₓ was 2.0 × 10⁵g⁻¹cm², and a melting point was 108-50°C.

**Table 12**

| Peak No. | Retention Time | Concentration |
|---|---|---|
| 1 | 5.892 | 0.0031 |
| 2 | 14.158 | 0.0312 |
| 3 | 15.487 | 0.0261 |
| 4 | 18.673 | 0.0682 |
| 5 | 19.408 | 3.5649 |
| 6 | 20.422 | 9.9876 |
| 7 | 21.545 | 11.2846 |
| 8 | 24.07 | 28.6963 |
| 9 | 25.39 | 10.4533 |
| 10 | 26.208 | 1.3327 |
| 11 | 27.942 | 2.393 |
| 12 | 33.915 | 24.1779 |
| 13 | 36.875 | 1.2773 |
| 14 | 40.742 | 2.7678 |
| 15 | 44.742 | 2.54 |
| 16 | 48.342 | 0.5219 |
| 17 | 52.07 | 0.5587 |
| 18 | 54.602 | 0.2424 |
| 19 | 56.192 | 0.073 |
| | Total | 100 |

### Example 12

Ten grams (8.84 mmol) of a mixture of palladium tetraα-(1-iso-propyl-2-methylbutyloxy)phthalocyanines in which the ratio of the following formulae (10-5), (11-5), (9-5) and (12-5) was 10:55:30:5 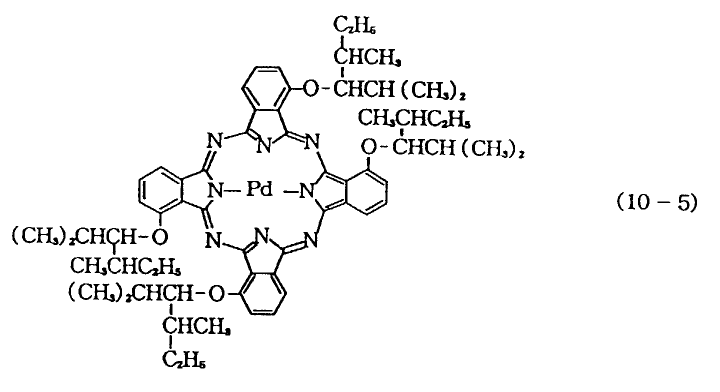 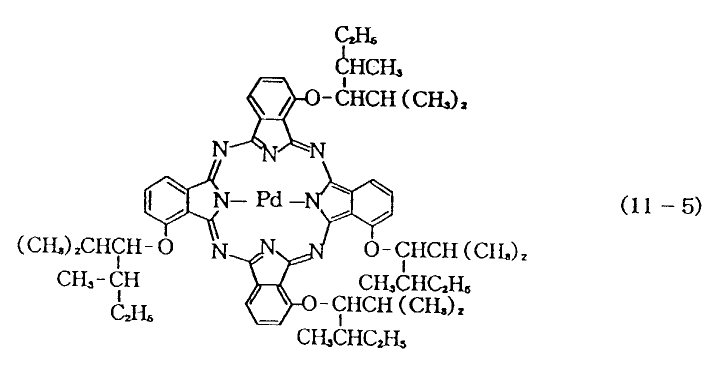 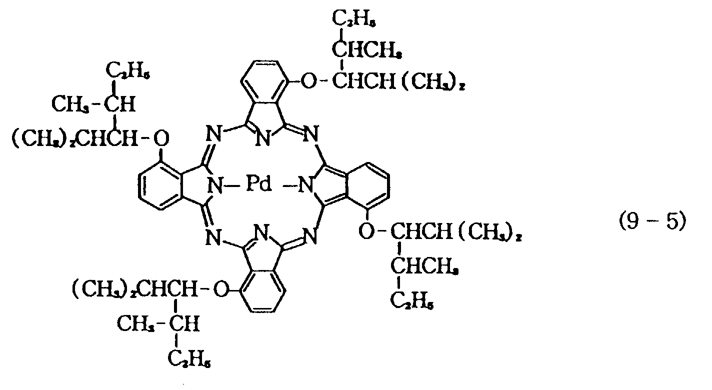 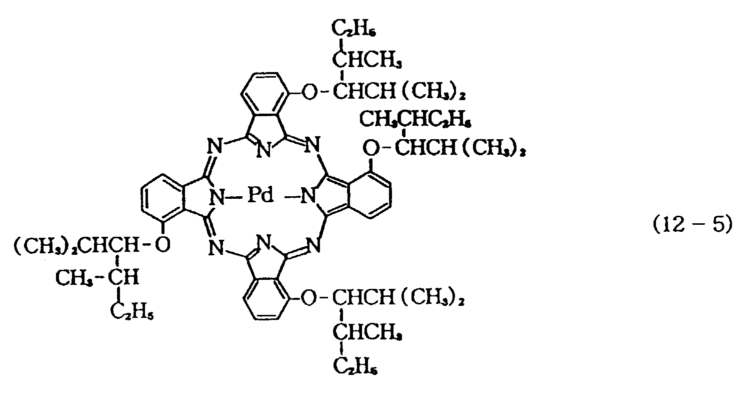 were dissolved in 48 g (30 ml) of 1,1,2,2-tetrachloroethane, and 20 g (20 ml) of water were added thereto. Next, a mixture of 5.51 g (34.48 mmol) of bromine and 16 g (10 ml) of 1,1,2,2-tetrachloroethane was added dropwise at 50 to 55°C, and reaction was then carried out at 55 to 60°C for 1 hour. Afterward, 25 g of a 10% aqueous sodium hydrogensulfite solution were added, followed by washing. The resultant organic layer was added dropwise to 158 g of methanol, and the precipitated crystals were filtered, thereby obtaining 12.5 g of a mixture of a brominated phthalocyanines represented by the formulae (2-7), (3-20), (1-20) and (4-14).

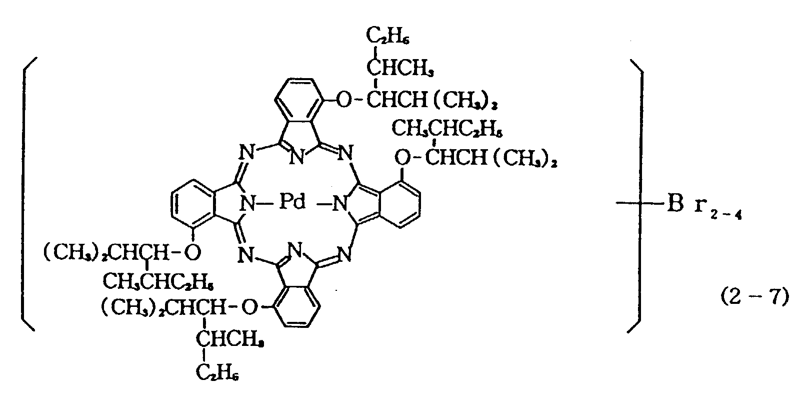

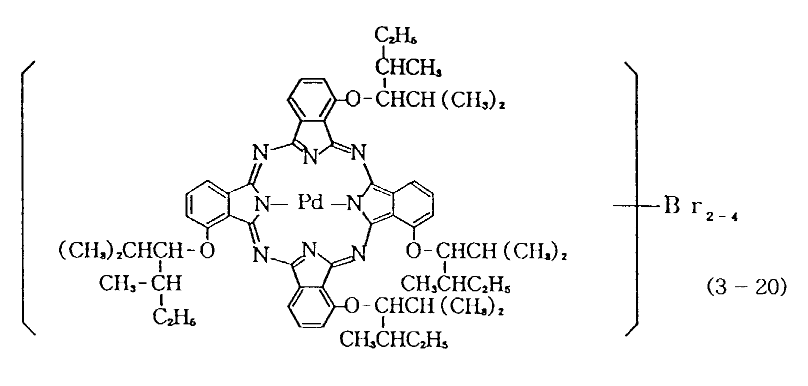

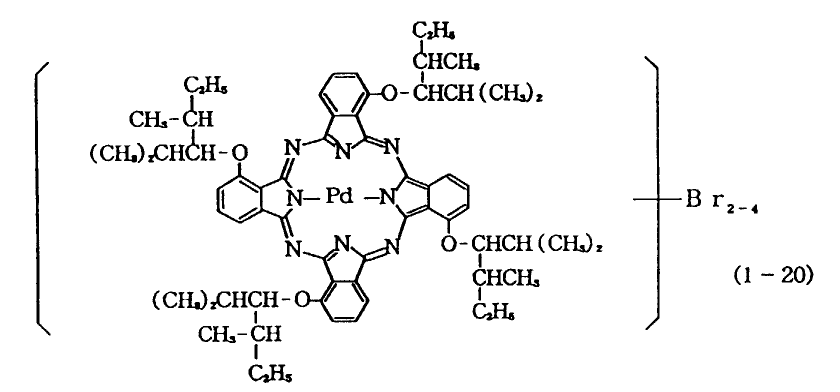

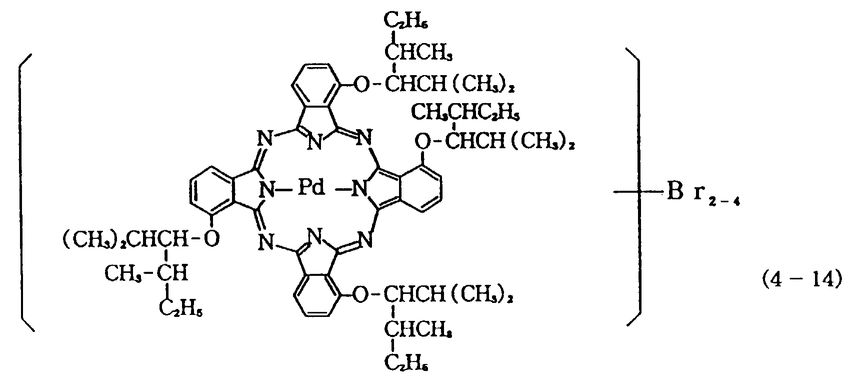

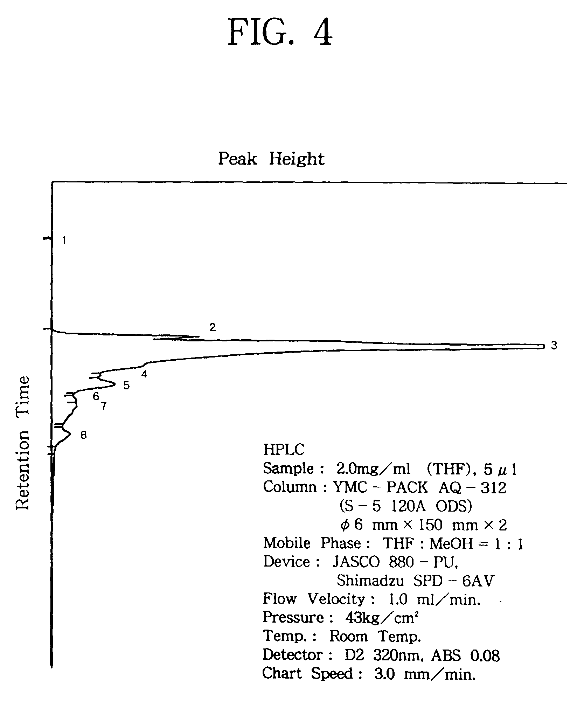

The liquid chromatogram of the mixture is shown in Fig. 4. A retention time and concentration of each peak are set forth in Table 13. The maximum absorption wave length λₘₐₓ of the mixture was 706 nm, εₘₐₓ was 1.4 × 10⁵g⁻¹cm², and a melting point was 201-28°C.

**Table 13**

| Peak No. | Retention Time | Concentration |
|---|---|---|
| 1 | 5.9 | 0.0037 |
| 2 | 14.82 | 6.5073 |
| 3 | 15.96 | 89.0199 |
| 4 | 18.242 | 0.1087 |
| 5 | 19.108 | 2.2992 |
| 6 | 20.542 | 0.2509 |
| 7 | 21.073 | 0.8823 |
| 8 | 23.572 | 0.928 |
| | Total | 100 |

### Example 13

Ten grams (8.84 mmol) of a mixture of palladium tetraα-(1-tert-butyl-2-methylpropyloxy)phthalocyanines in which the ratio of the following formulae (10-6), (11-6), (9-6) and (12-6) was 5:45:30:20 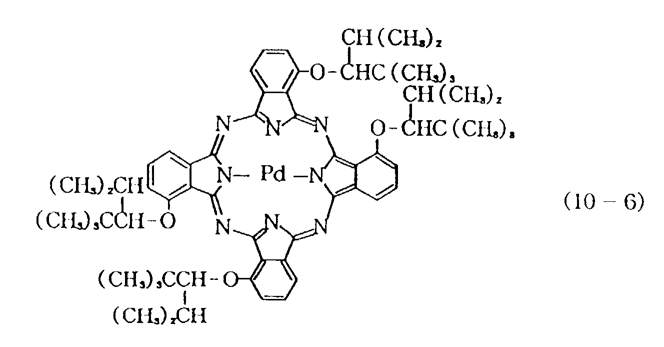 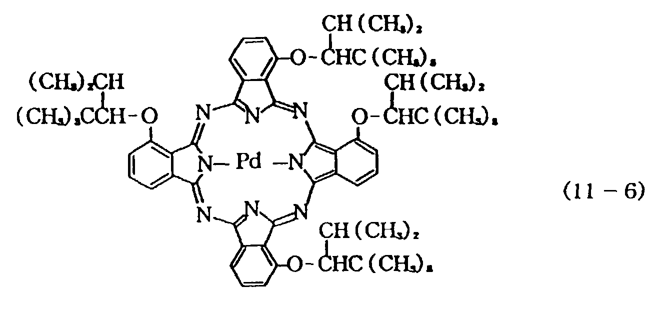 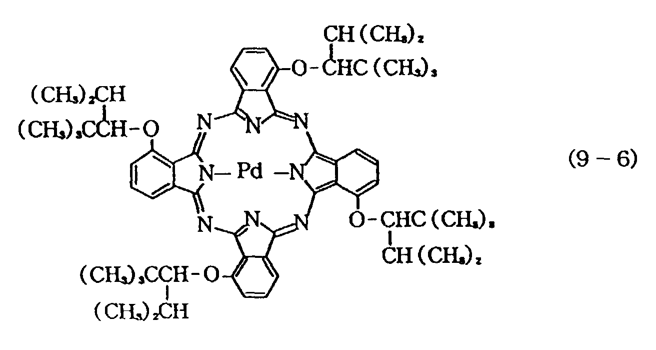 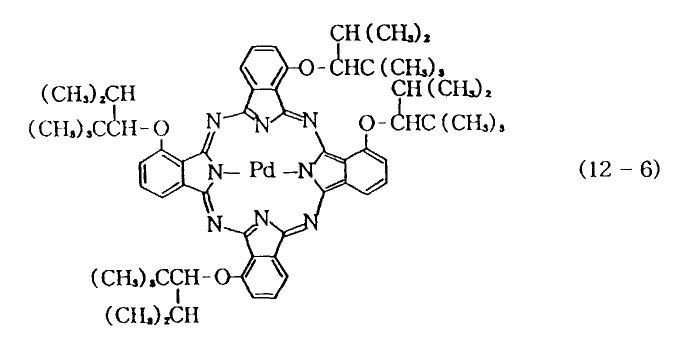 were dissolved in 56 g (39 ml) of 1,1,2-trichloroethane, and 20 g (20 ml) of water were added thereto. Next, a mixture of 4.94 g (30.91 mmol) of bromine and 12 g (8 ml) of 1,1,2-trichloroethane was added dropwise at 50 to 55°C, and reaction was then carried out at 55 to 60°C for 1 hour. Afterward, 20 g of a 10% aqueous sodium hydrogensulfite solution were added, followed by washing. The resultant organic layer was added dropewise to 135 g of methanol, and the precipitated crystals were filtered, thereby obtaining 12 g of a mixture of brominated phthalocyanines represented by the formulae (2-8), (3-21), (1-21) and (4-15).

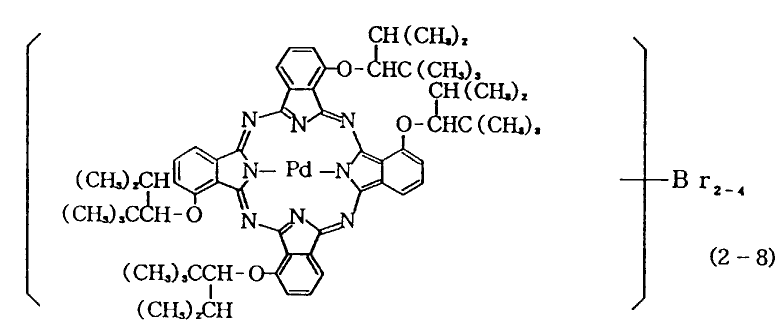

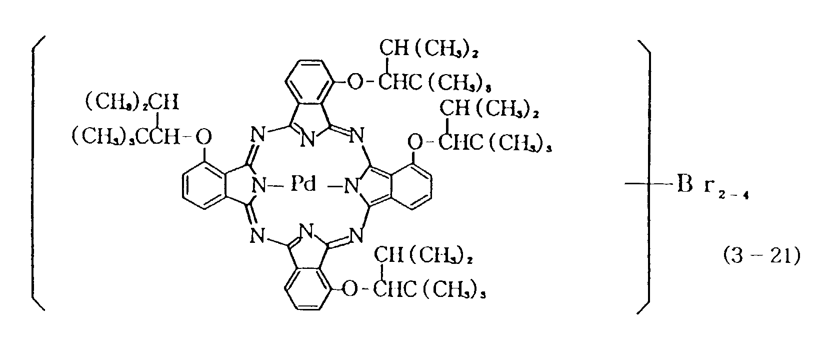

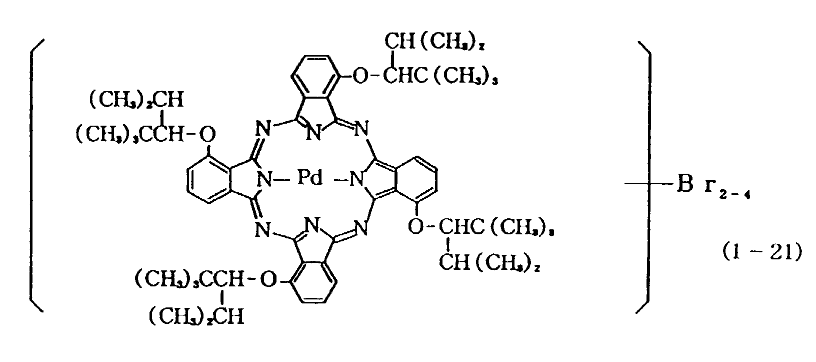

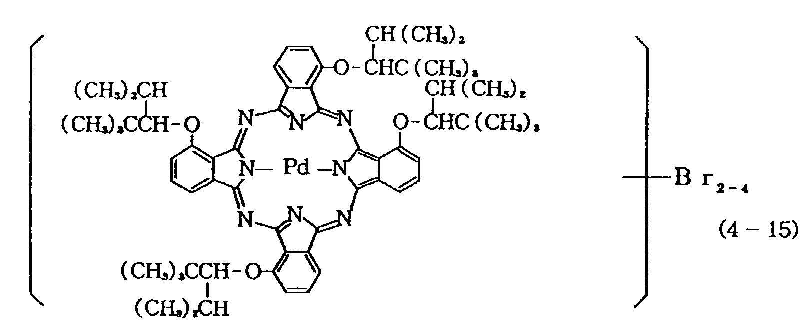

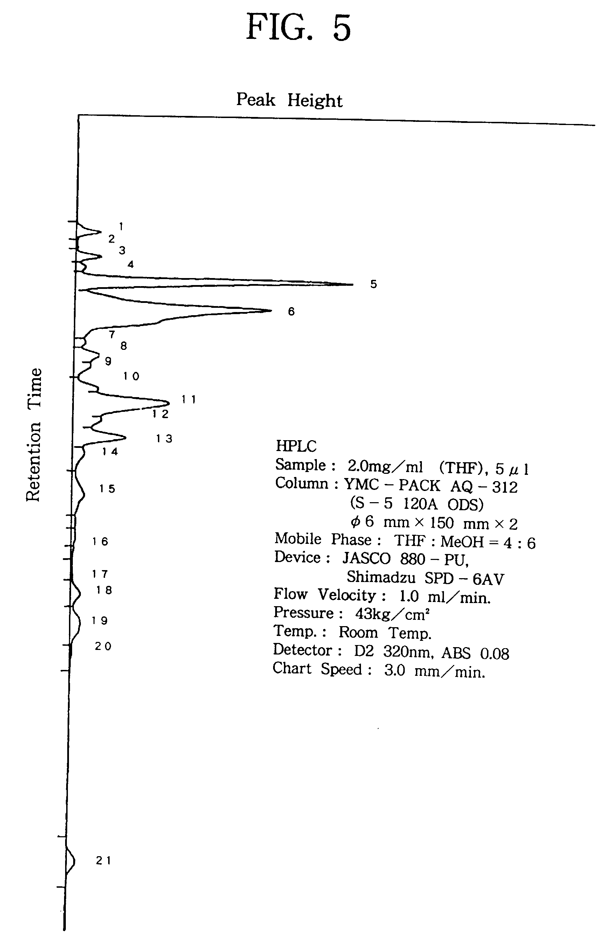

The liquid chromatogram of the mixture is shown in Fig. 5. A retention time and concentration of each peak are set forth in Table 14. The maximum absorption wave length λₘₐₓ of the mixture was 705 nm, εₘₐₓ was 1.7 × 10⁵g⁻¹cm², and a melting point was 268-86°C.

**Table 14**

| Peak No. | Retention Time | Concentration |
|---|---|---|
| 1 | 23.775 | 1.5088 |
| 2 | 24.99 | 0.0758 |
| 3 | 25.942 | 1.4933 |
| 4 | 26.948 | 0.6703 |
| 5 | 28.242 | 20.3779 |
| 6 | 30.742 | 36.4356 |
| 7 | 33.673 | 0.1498 |
| 8 | 34.808 | 2.6473 |
| 9 | 35.723 | 1.4776 |
| 10 | 37.738 | 2.0874 |
| 11 | 39.067 | 14.626 |
| 12 | 40.535 | 2.6031 |
| 13 | 42.178 | 6.0621 |
| 14 | 43.542 | 1.3456 |
| 15 | 47.407 | 1.9147 |
| 16 | 51.138 | 0.1066 |
| 17 | 54.542 | 0.2127 |
| 18 | 56.338 | 1.51 |
| 19 | 58.805 | 2.5835 |
| 20 | 61.592 | 0.3165 |
| 21 | 80.675 | 1.7949 |
| | Total | 100 |

### Example 14

Fifteen grams (15.57 mmol) of a mixture of palladium tetraα-(1,2-dimethylpropyloxy)phthalocyanines in which the ratio of the following formulae (10-7), (11-7), (9-7) and (12-7) was 10:40:30:20 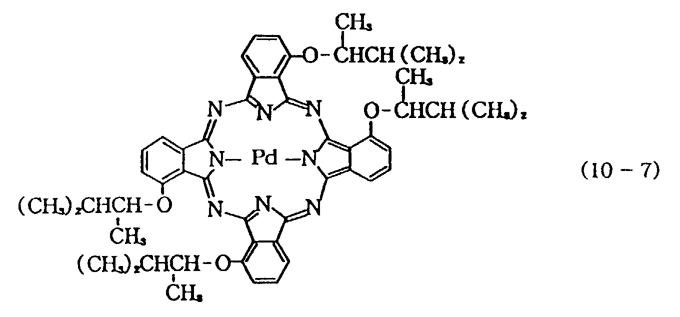 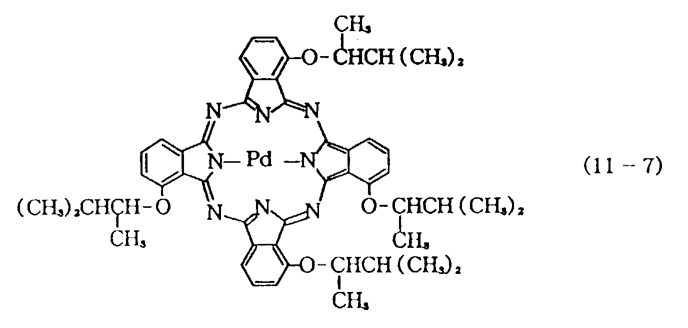 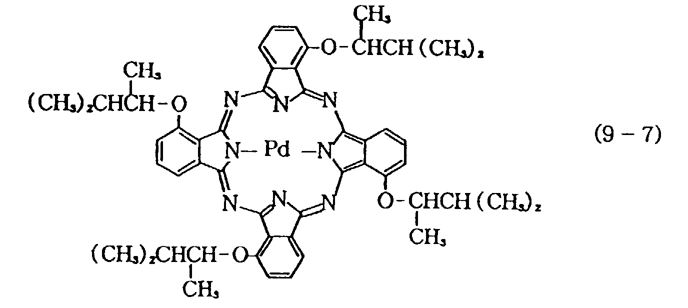 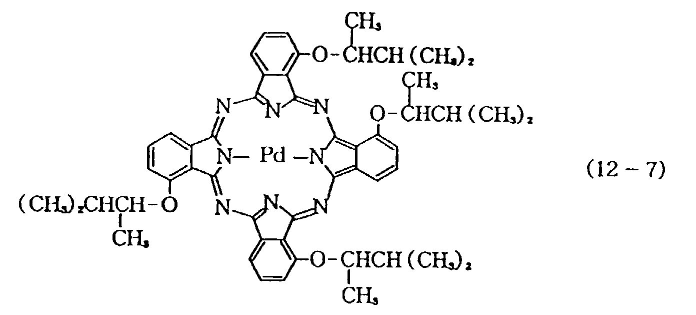 were added to a mixed solvent of 120 g (75 ml) of 1,1,2,2-tetrachloroethane and 100 g (100 ml) of water. Next, 6.2 g (38.79 mmol) of bromine were added, and reaction was then carried out at 60°C for 2 hours. After cooling to 20°C, separation was carried out. Successively, the resultant organic solvent layer was washed with 100 g of a 10% aqueous sodium hydrogensulfite solution and 100 g of a 5% aqueous sodium hydrogencarbonate solution. The separated organic layer was added dropwise to 240 g of methanol, and the precipitated crystals were then filtered to obtain 16 g of a mixture of brominated phthalocyanines represented by the following formulae (2-9), (3-22), (1-22) and (4-16). The maximum absorption wave length λₘₐₓ of the mixture was 702 nm, εₘₐₓ was 1.5 × 10⁵g⁻¹cm², and a melting point was 172-220°C.

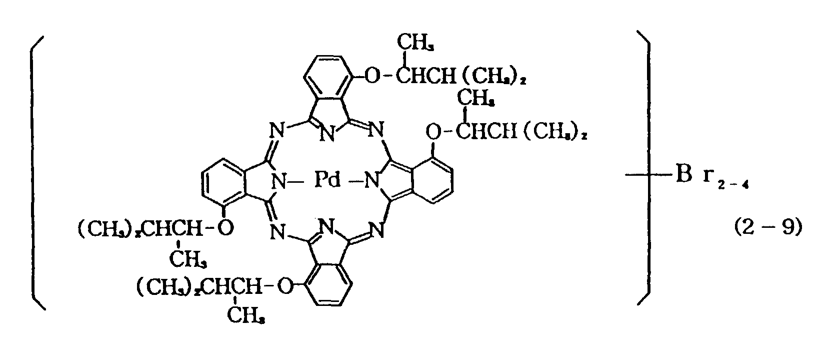

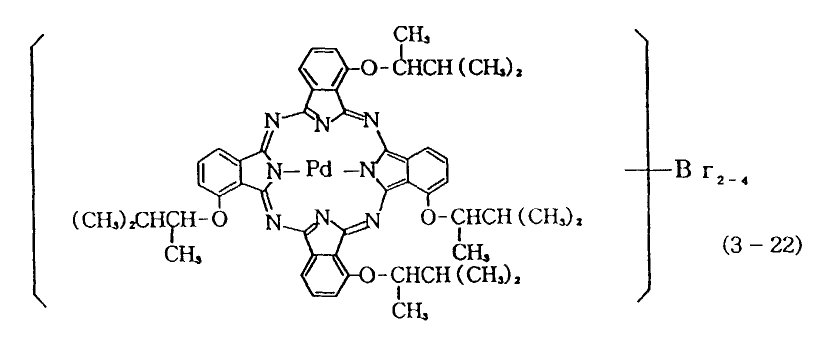

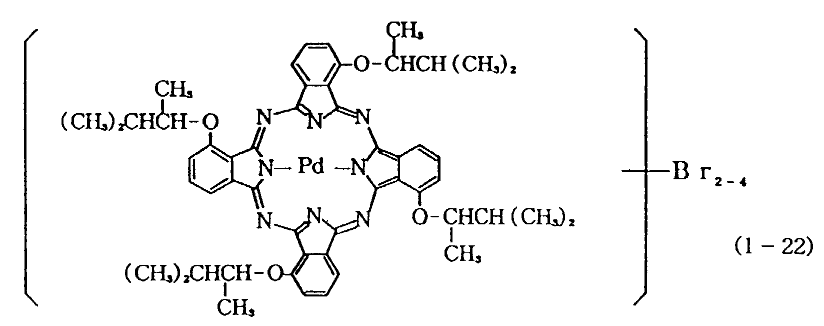

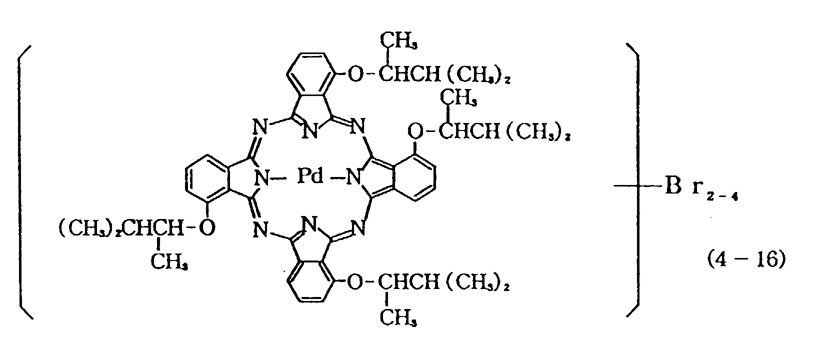

### Example 15

Fifteen grams (13.95 mmol) of a mixture of palladium tetraα-(1-iso-propylbutyloxy)phthalocyanines in which the ratio of the following formulae (10-8), (11-8), (9-8) and (12-8) was 10:50:30:10 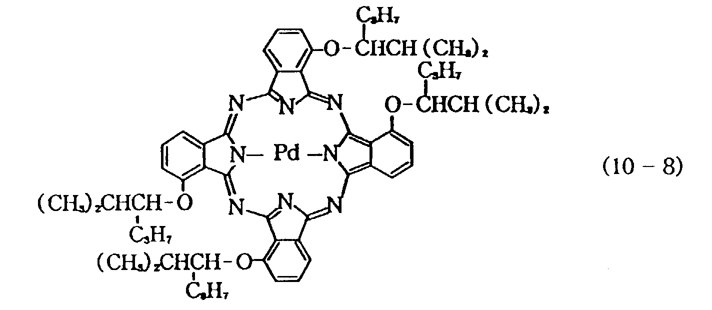 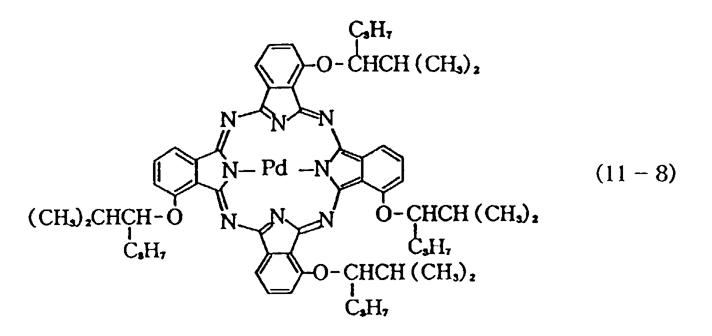 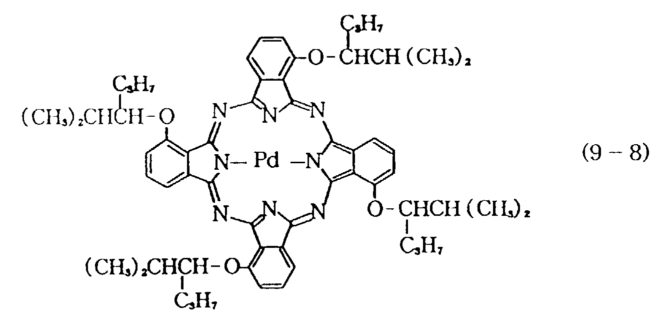 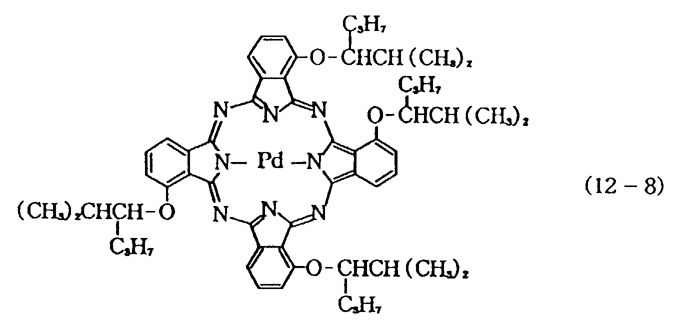 were added to a mixed solvent of 50 g (56 ml) of tetrahydrofuran, 50 g (76 ml) of n-hexane and 100 g (100 ml) of water. Next, 7.2 g (45.05 mmol) of bromine were added, and reaction was then carried out at 60°C for 2 hours. After cooling to 20°C, 50 g of toluene were added, followed by separation. Successively, the resultant organic solvent layer was washed with 100 g of a 10% aqueous sodium hydrogensulfite solution and 100 g of a 5% aqueous sodium hydrogencarbonate solution. The organic solvent was distilled off, and separation was then carried out through toluene-silica gel chromatography to obtain 16 g of a mixture of brominated phthalocyanines represented by the following formulae (2-10), (3-23), (1-23) and (4-17).

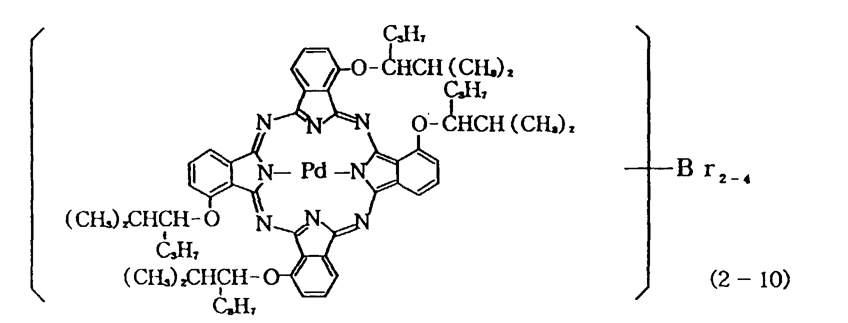

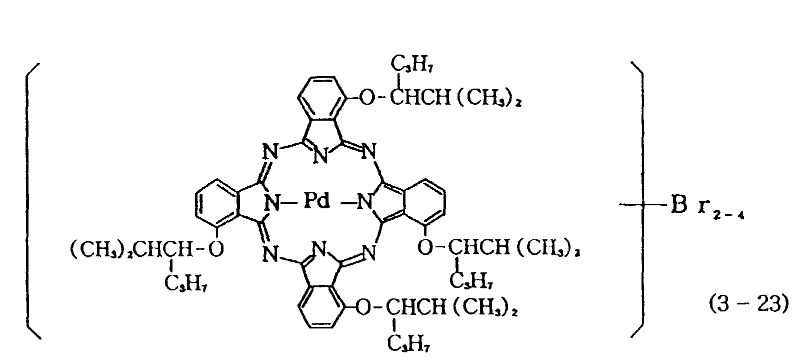

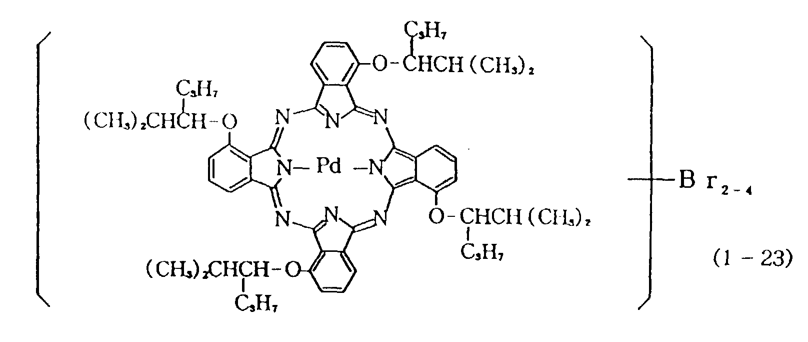

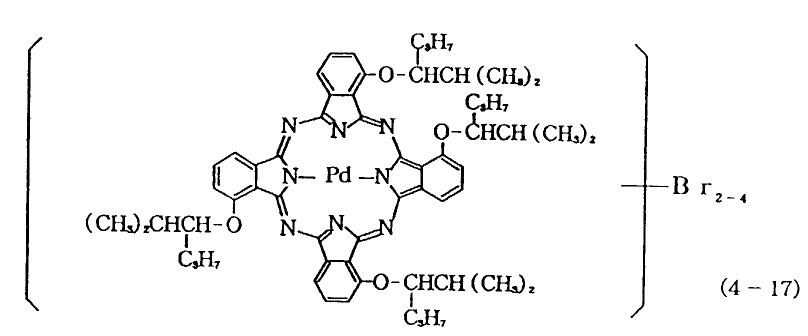

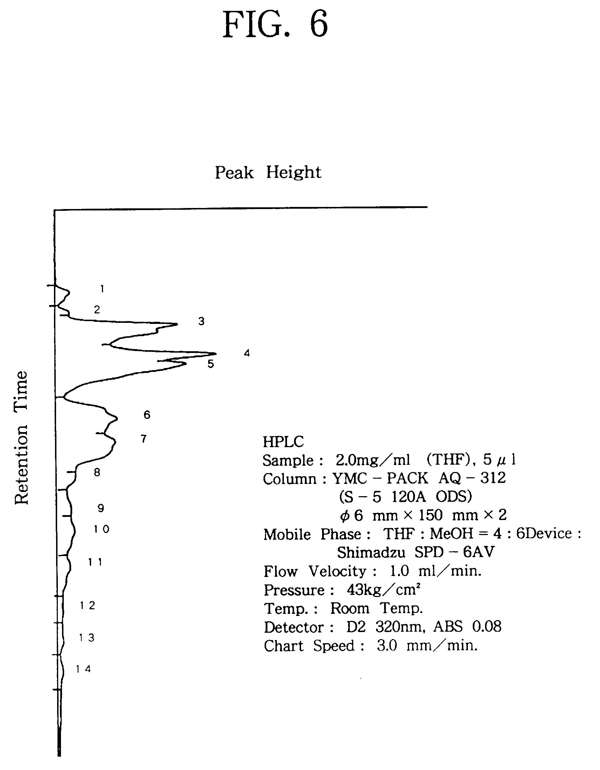

The liquid chromatogram of the mixture is shown in Fig. 6. A retention time and concentration of each peak are set forth in Table 15. The maximum absorption wave length λₘₐₓ of the mixture was 705 nm, εₘₐₓ was 1.5 × 10⁵g⁻¹cm², and a melting point was 149-203°C.

**Table 15**

| Peak No. | Retention Time | Concentration |
|---|---|---|
| 1 | 27.217 | 1.287 |
| 2 | 29.017 | 0.7368 |
| 3 | 30.29 | 19.2416 |
| 4 | 33.06 | 17.039 |
| 5 | 33.855 | 18.6948 |
| 6 | 38.598 | 14.3601 |
| 7 | 40.732 | 15.0406 |
| 8 | 43.667 | 2.2369 |
| 9 | 46.788 | 2.6558 |
| 10 | 48.665 | 4.4521 |
| 11 | 51.933 | 2.6343 |
| 12 | 55.533 | 0.6101 |
| 13 | 58.595 | 0.589 |
| 14 | 61.2 | 0.422 |
| | Total | 100 |

### Example 16

Fifteen grams (14.71 mmol) of a mixture of palladium tetraα-(1-iso-propylpropyloxy)phthalocyanines in which the ratio of the following formulae (10-9), (11-9), (9-9) and (12-9) was 10:45:35:15 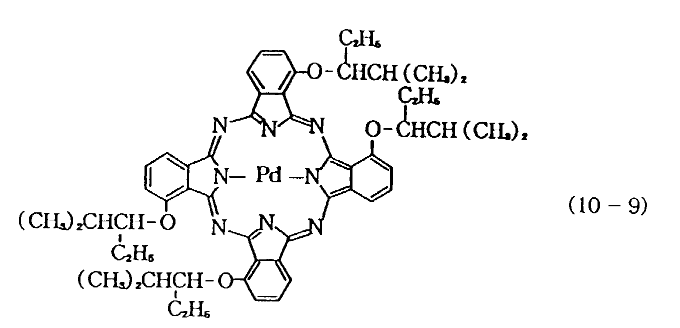 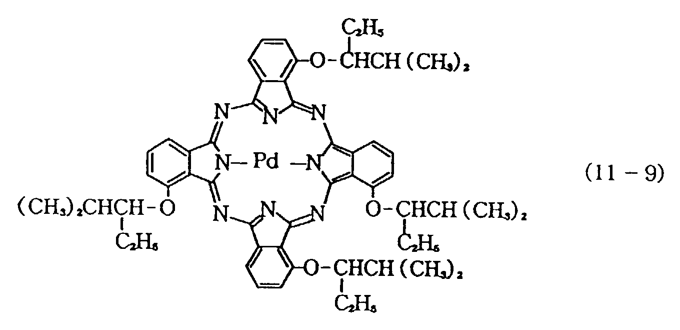 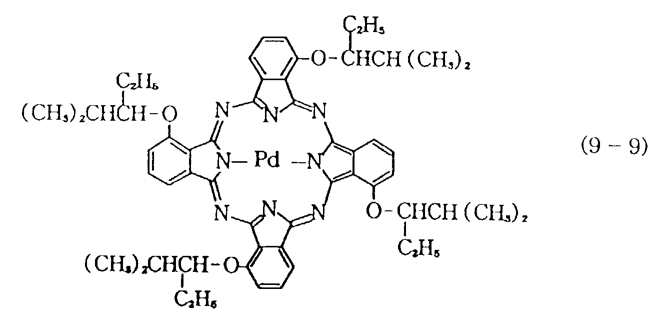 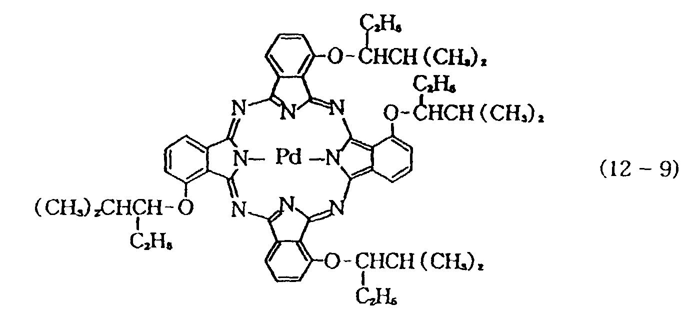 were added to a mixed solvent of 150 g (94 ml) of carbon tetrachloride and 100 g (100 ml) of water. Next, 7.2 g (45.05 mmol) of bromine were added, and reaction was then carried out at 60°C for 3 hours. After the solution was cooled to 20°C, followed by separation. Successively, the resultant organic solvent layer was washed with 100 g of a 10% aqueous sodium hydrogensulfite solution and 100 g of a 5% aqueous sodium hydrogencarbonate solution. The organic solvent was distilled off, and separation was then carried out by toluene-silica gel chromatography to obtain 15 g of a mixture of brominated phthalocyanines represented by the following formulae (2-11), (3-24), (1-24) and (4-18).

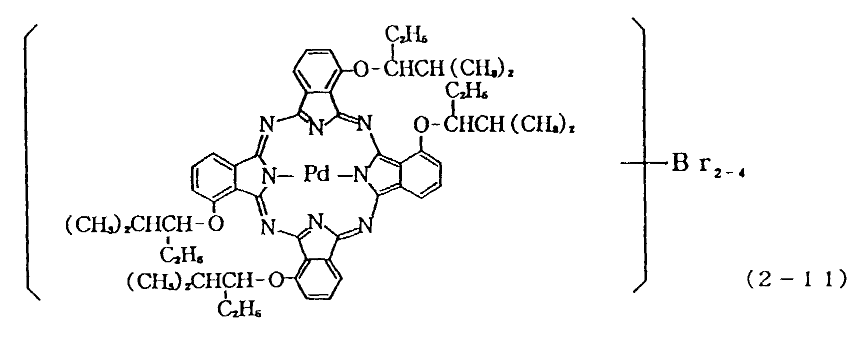

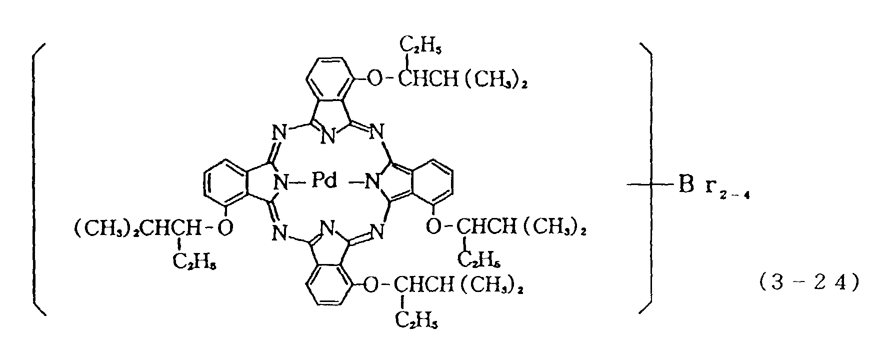

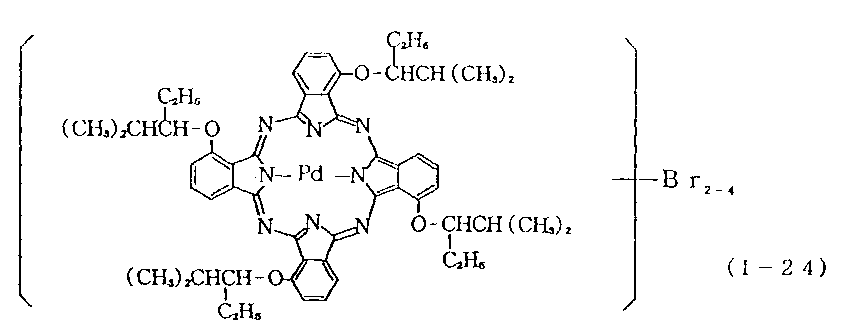

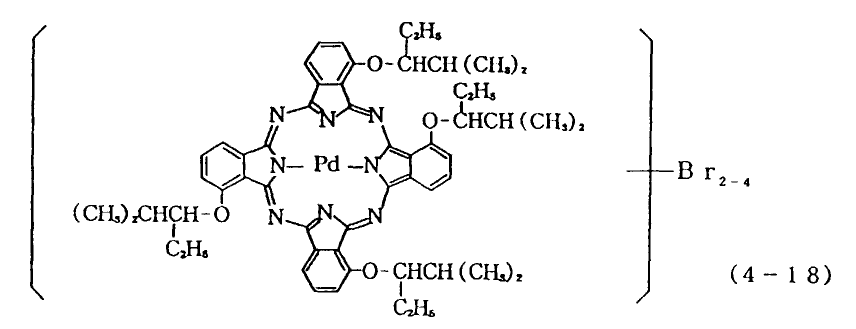

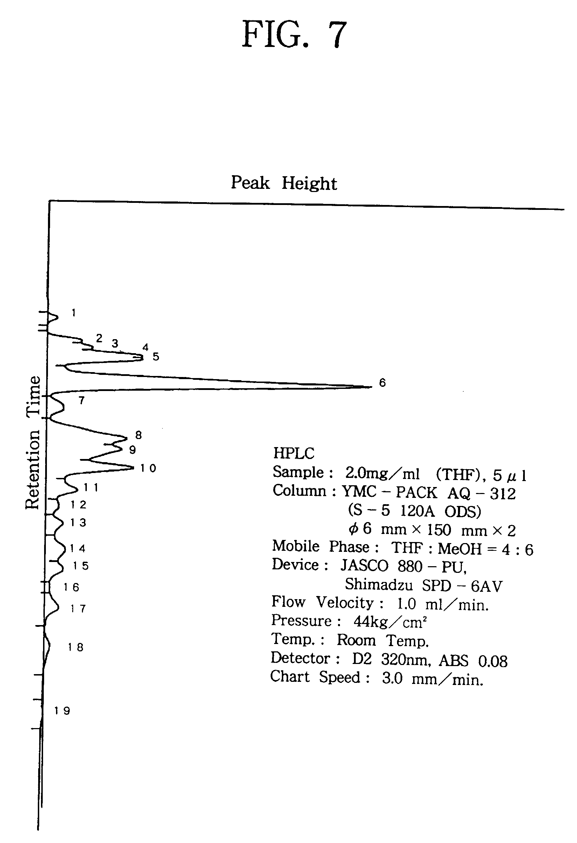

The liquid chromatogram of the mixture is shown in Fig. 7. A retention time and concentration of each peak are set forth in Table 16. The maximum absorption wave length λₘₐₓ of the mixture was 705 nm, εₘₐₓ was 1.5 × 10⁵g⁻¹cm², and a melting point was 190-242°C.

**Table 16**

| Peak No. | Retention Time | Concentration |
|---|---|---|
| 1 | 24.4 | 0.5385 |
| 2 | 26.4 | 1.8936 |
| 3 | 27.033 | 2.7709 |
| 4 | 27.728 | 5.1211 |
| 5 | 28.033 | 5.4332 |
| 6 | 30.295 | 32.6837 |
| 7 | 32.467 | 2.3957 |
| 8 | 35.2 | 11.1743 |
| 9 | 36.158 | 9.2337 |
| 10 | 37.84 | 9.6109 |
| 11 | 39.907 | 4.3202 |
| 12 | 41.333 | 1.712 |
| 13 | 42.933 | 2.4794 |
| 14 | 45.265 | 3.7565 |
| 15 | 47 | 2.503 |
| 16 | 48.732 | 0.5462 |
| 17 | 50.467 | 2.357 |
| 18 | 53.898 | 1.2538 |
| 19 | 60.067 | 0.2164 |
| | Total | 100 |

### Example 17

Fifteen grams (14.71 mmol) of a mixture of palladium tetraα-(1,2-dimethylbutyloxy)phthalocyanines in which the ratio of the following formulae (10-10), (11-10), (9-10), and (12-10) was 10:45:35:15 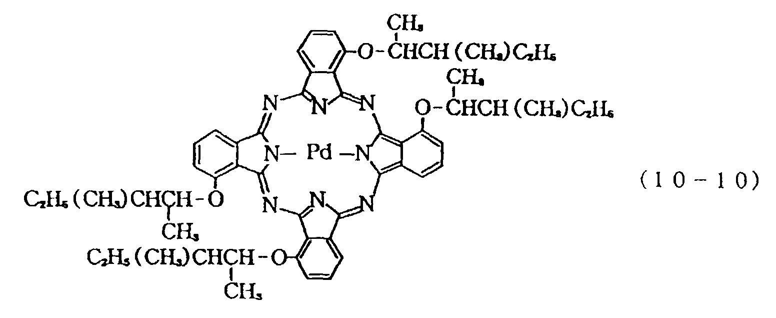 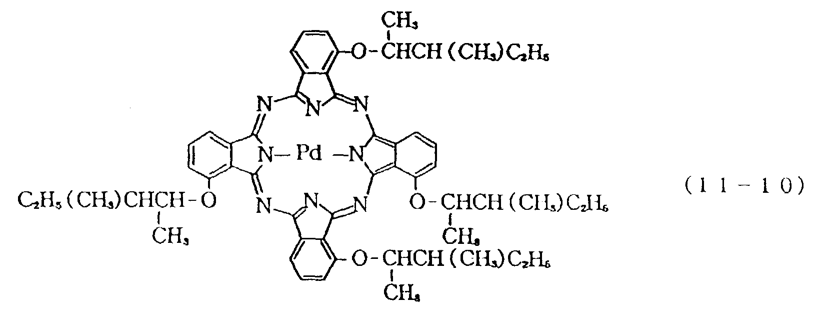 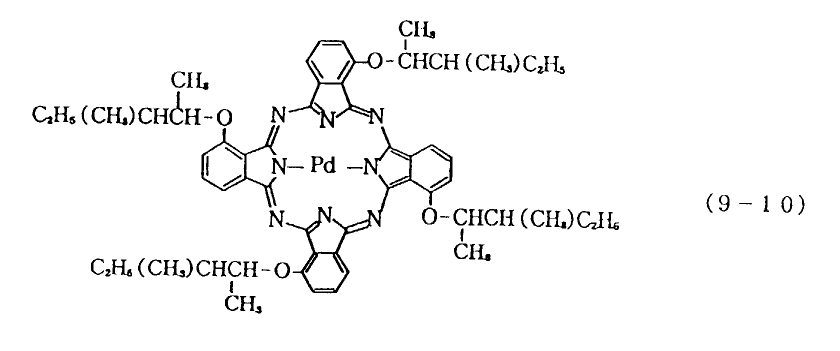 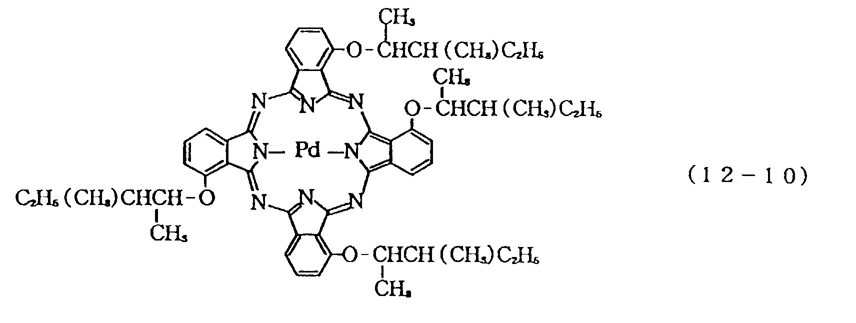 were added to a mixed solvent of 150 g (94 ml) of carbon tetrachloride and 50 g (50 ml) of water. Next, 7.2 g (45.05 mmol) of bromine were added, and reaction was then carried out at 60°C for 3 hours. After the solution was cooled to 20°C, followed by separation. Successively, the resultant organic solvent layer was washed with 100 g of a 10% aqueous sodium hydrogensulfite solution and 100 g of a 5% aqueous sodium hydrogencarbonate solution. The organic solvent was distilled off, and separation was then carried out by toluene-silica gel chromatography to obtain 15 g of a mixture of brominated phthalocyanines represented by the following formulae (2-12), (3-25), (1-25) and (4-19).

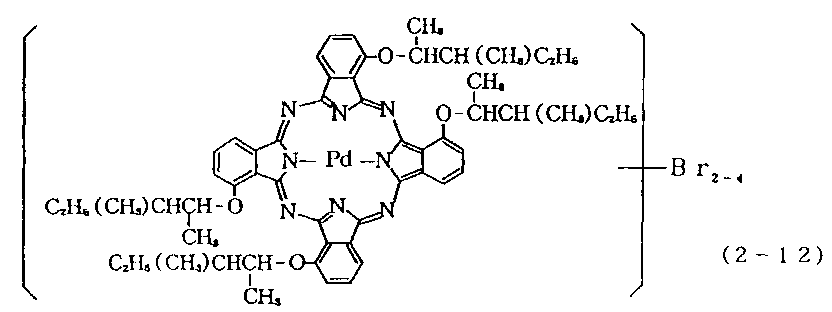

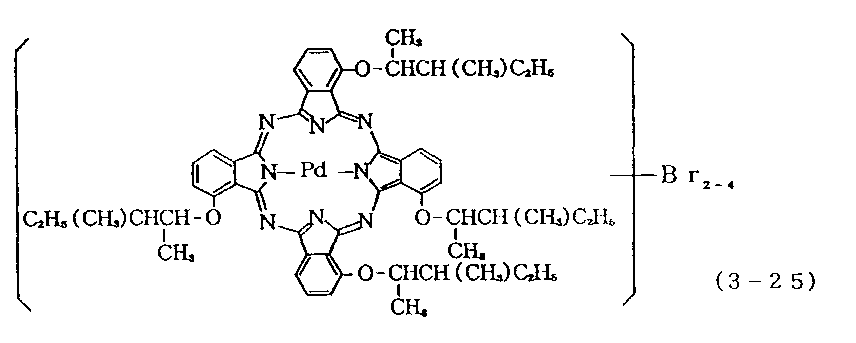

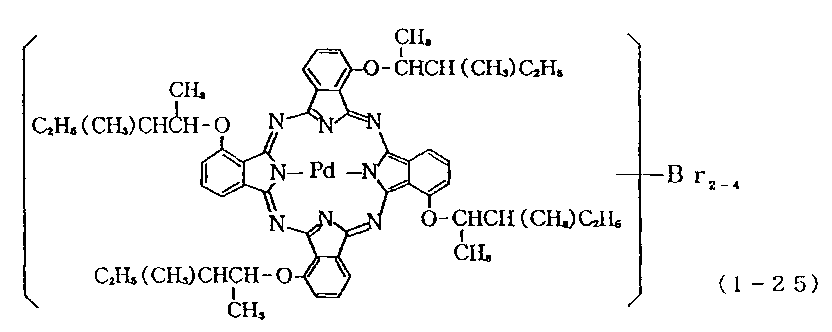

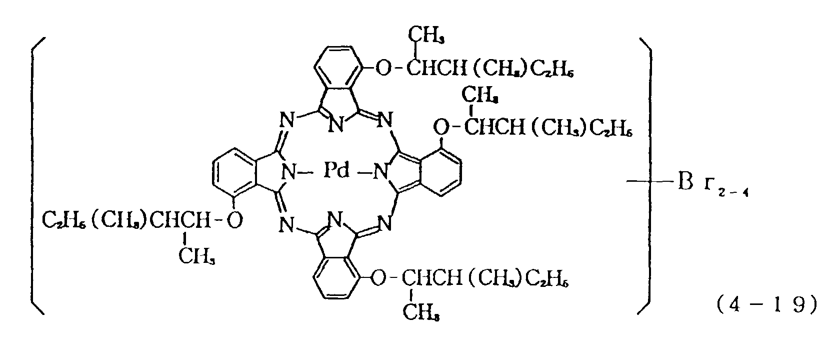

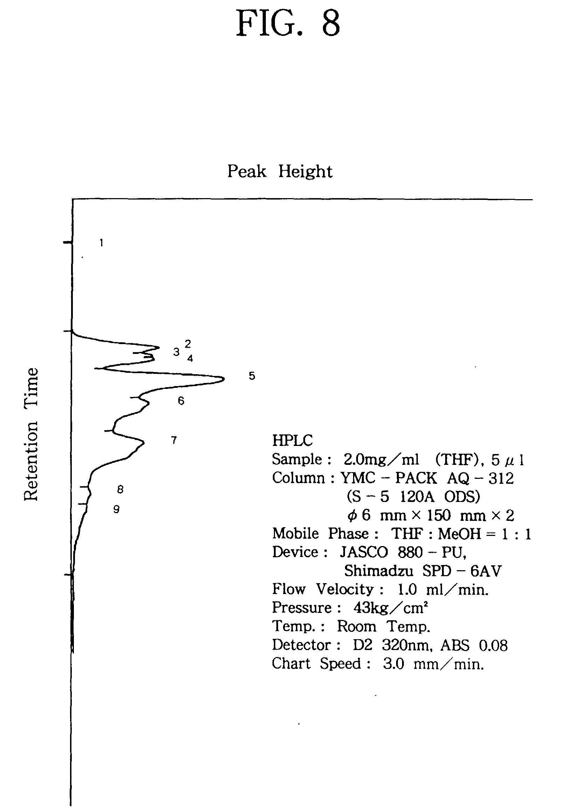

The liquid chromatogram of the mixture is shown in Fig. 8. A retention time and concentration of each peak are set forth in Table 17. The maximum absorption wave length λₘₐₓ of the mixture was 702 nm, εₘₐₓ was 1.5 × 10⁵g⁻¹cm², and a melting point was 153-230°C.

**Table 17**

| Peak No. | Retention Time | Concentration |
|---|---|---|
| 1 | 5.892 | 0.0035 |
| 2 | 15.733 | 10.0303 |
| 3 | 16.43 | 3.7456 |
| 4 | 16.745 | 6.2047 |
| 5 | 18.633 | 30.4854 |
| 6 | 20.733 | 19.1149 |
| 7 | 24.232 | 23.7787 |
| 8 | 28.6 | 2.8332 |
| 9 | 30.065 | 3.8037 |
| | Total | 100 |

### Example 18

Fifteen grams (14.52 mmol) of a mixture of copper tetraα-(1-iso-propyl-2-methylpropyloxy)phthalocyanines in which the ratio of the following formulae (10-11), (11-11), (9-11) and (12-11) was 10:45:35:15 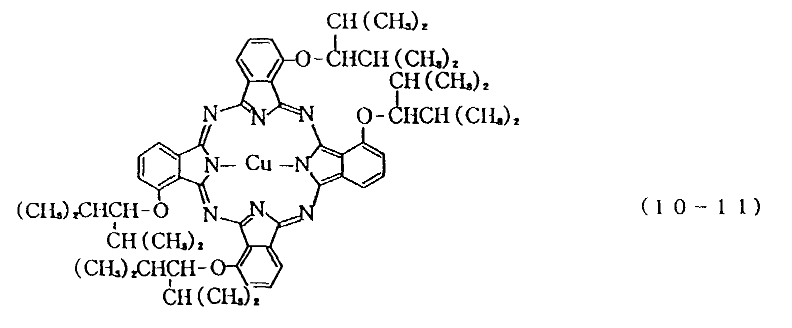 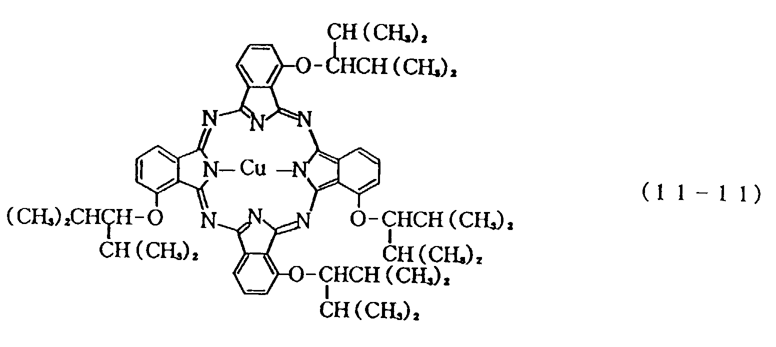 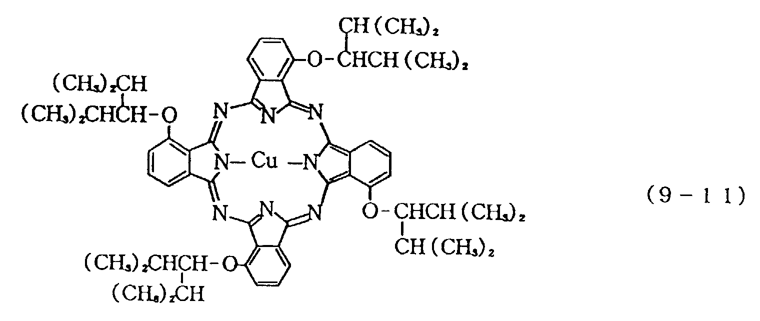 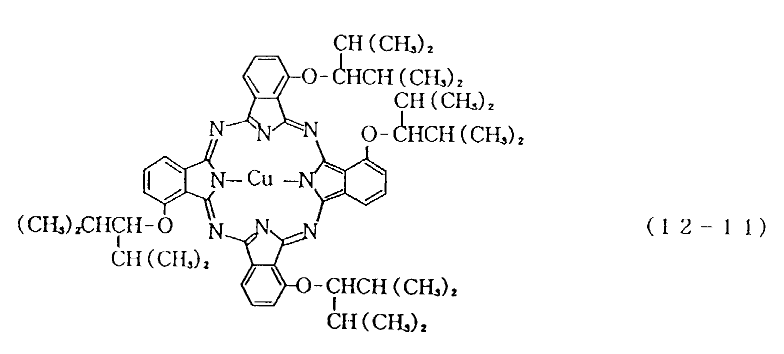 were added to a mixed solvent of 150 g (94 ml) of carbon tetrachloride and 80 g (80 ml) of water. Next, 3.6 g (22.52 mmol) of bromine were added, and reaction was then carried out at 60°C for 3 hours. The reaction solution was cooled to 20°C, followed by separation. Successively, the resultant organic solvent layer was washed with 100 g of a 10% aqueous sodium hydrogensulfite solution and 100 g of a 5% aqueous sodium hydrogencarbonate solution. The organic solvent was distilled off, and separation was then carried out by toluene-silica gel chromatography to obtain 15 g of a mixture of brominated phthalocyanines represented by the following formulae (2-13), (3-26), (1-26) and (4-20).

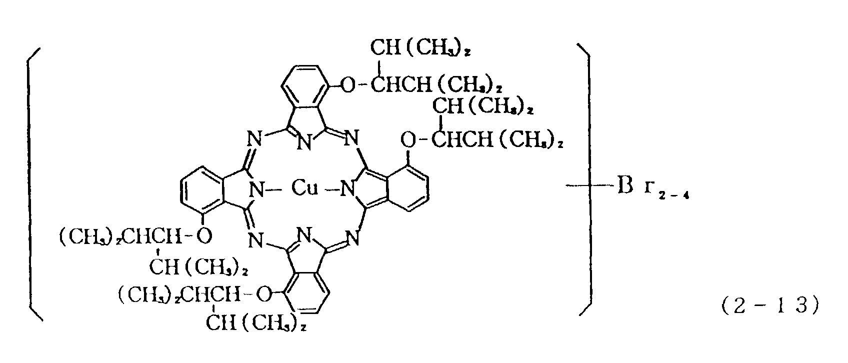

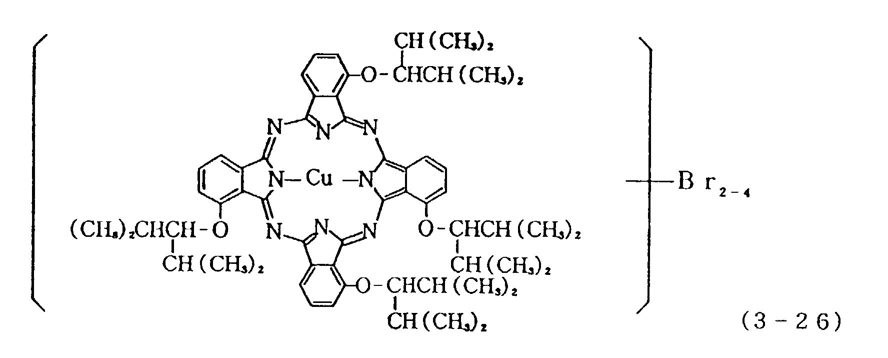

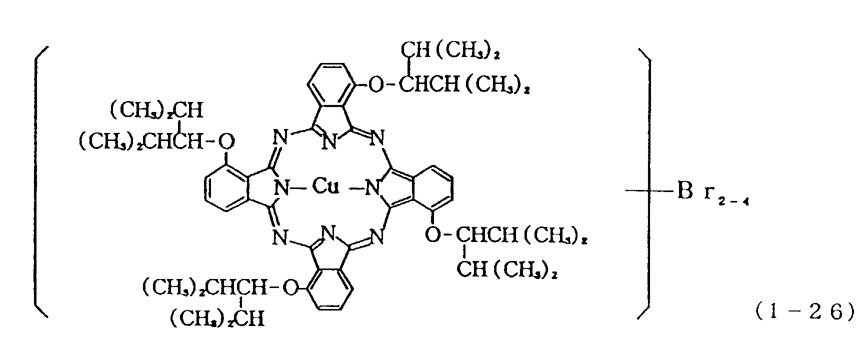

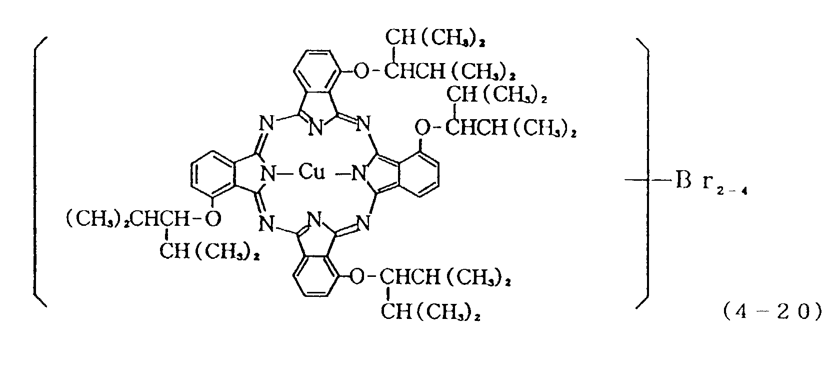

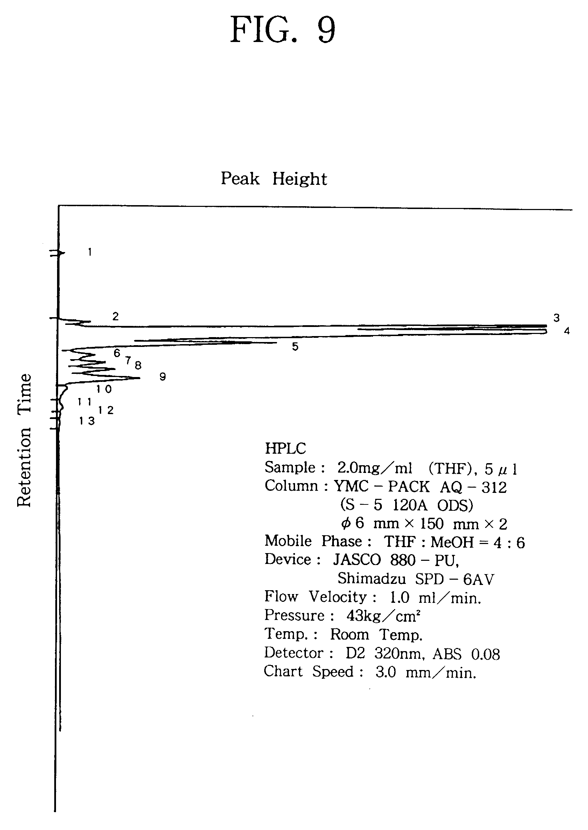

The liquid chromatogram of the mixture is shown in Fig. 9. A retention time and concentration of each peak are set forth in Table 18. The maximum absorption wave length λₘₐₓ of the mixture was 708 nm, εₘₐₓ was 2.8 × 10⁵g⁻¹cm², and a melting point was 195-240°C.

**Table 18**

| Peak No. | Retention Time | Concentration |
|---|---|---|
| 1 | 6.967 | 0.1361 |
| 2 | 13.2 | 1.1805 |
| 3 | 13.965 | 27.5883 |
| 4 | 14.425 | 44.2342 |
| 5 | 15.242 | 11.1951 |
| 6 | 16.167 | 2.7052 |
| 7 | 16.855 | 2.4921 |
| 8 | 17.467 | 3.1933 |
| 9 | 18.33 | 6.0078 |
| 10 | 19.065 | 0.7638 |
| 11 | 20.767 | 0.3368 |
| 12 | 21.39 | 0.0794 |
| 13 | 22.167 | 0.0874 |
| | Total | 100 |

### Example 19

Fifteen grams (15.43 mmol) of a mixture of nickel tetraα-(1,2-dimethylbutyloxy)phthalocyanines in which the ratio of the following formulae (10-12), (11-12), (9-12) and (12-12) was 10:45:35:15 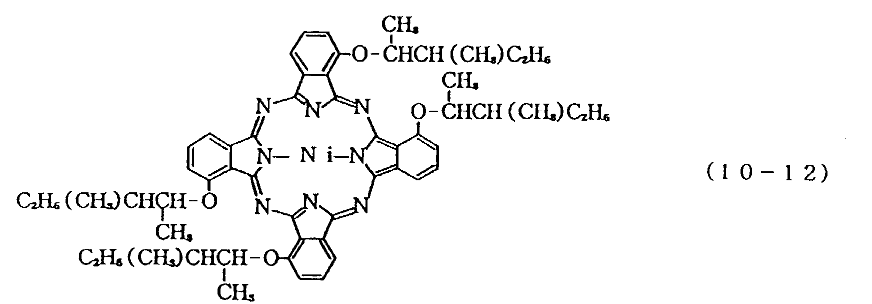 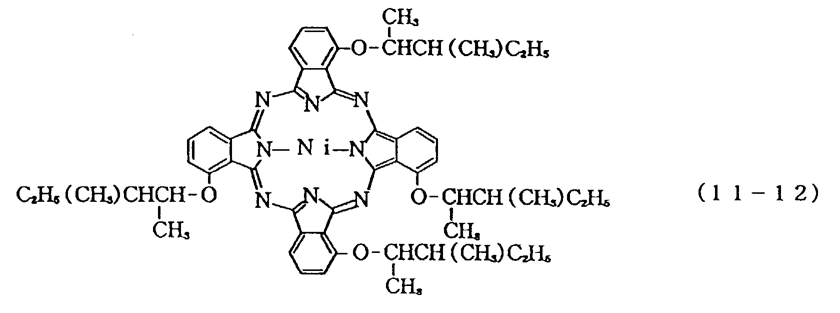 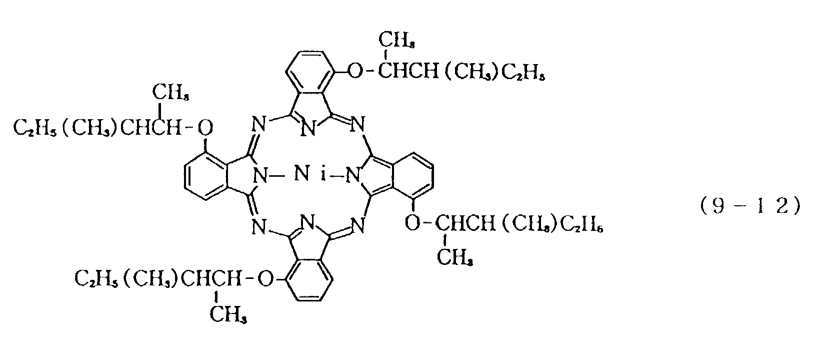 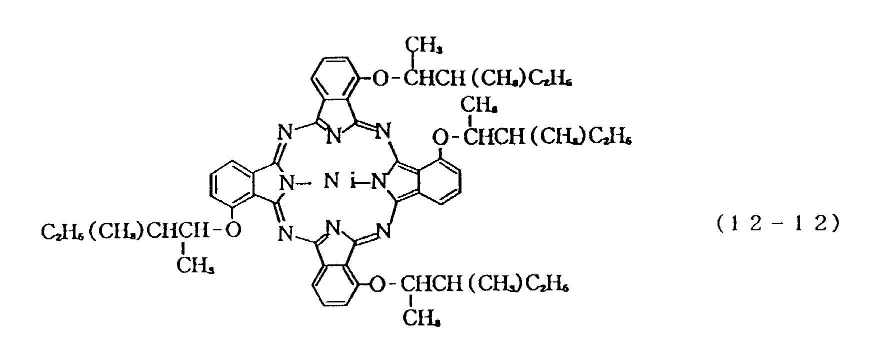 were added to a mixed solvent of 100 g (69 ml) of 1,1,2-trichloroethane and 50 g (50 ml) of water. Next, 3.2 g (20.02 mmol) of bromine were added, and reaction was then carried out at 60°C for 2 hours. After cooling to 30°C, 20 g of a 10% aqueous sodium hydrogensulfite solution were added to wash the solution. After separation, the organic layer was added dropwise to 360 g of methanol, and the precipitated crystals were then filtered to obtain 16.2 g of a mixture of brominated phthalocyanines represented by the following formulae (2-14), (3-27), (1-27) and (4-21). The maximum absorption wave length λₘₐₓ of the mixture was 708 nm, and εₘₐₓ was 2.1 × 10⁵g⁻¹cm².

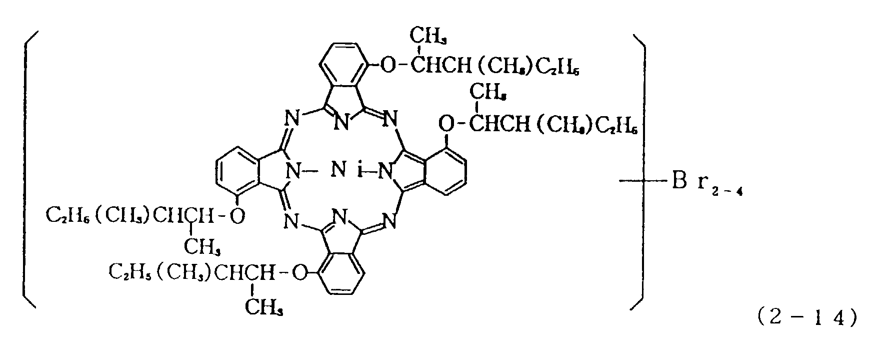

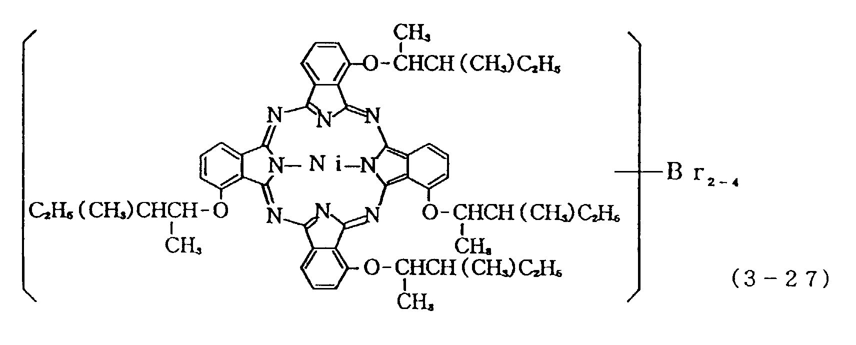

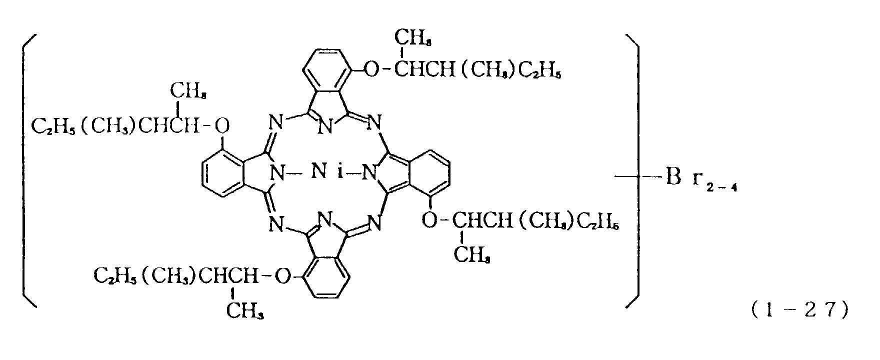

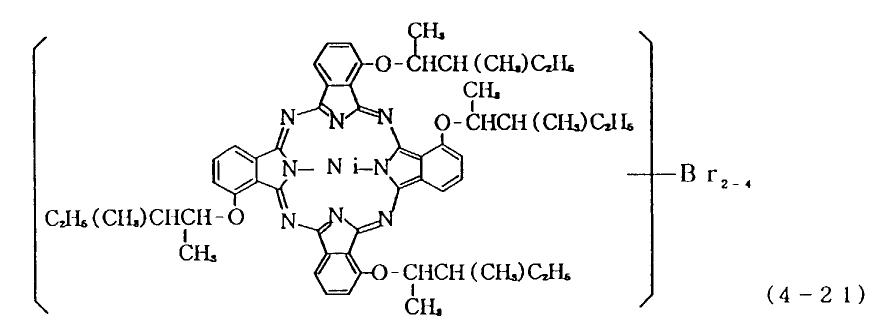

### Example 20

Six grams of the mixture synthesized in Example 18 were dissolved (30 g/l concentration) in 200 ml of cyclooctane, and the mixture solution was applied on a polycarbonate substrate by a spin coating method. Here, industrially continuous coating properties and film formation properties were tested, and the results were good. A film thickness was 120 nm. The film of gold having a thickness of 50 nm was formed thereon by sputtering. An ultraviolet-setting acrylic resin was further applied thereon by the spin coating method and then irradiated with ultraviolet rays to cure the resin. Thus, a CD-WO optical recording medium was prepared. A reflectance was 70%. A sensitivity was 65 dB of CN ratio when recording was carried out by using a semiconductor laser at 1.4 m/sec, 7 mW and 785 nm. Furthermore, the obtained optical recording medium was excellent in humidity resistance and light resistance.

### Examples 21 to 23

Optical recording media for CD-WO were prepared following the same procedure as in Example 20 by the use of mixtures obtained in the above-mentioned examples, and industrially continuous coating properties and film formation properties were tested. For the obtained optical recording media for CD-WO, optimum power and recording sensitivity were inspected. The results are set forth in Table 19. Incidentally, reflectances were in the range of 65 to 72%.

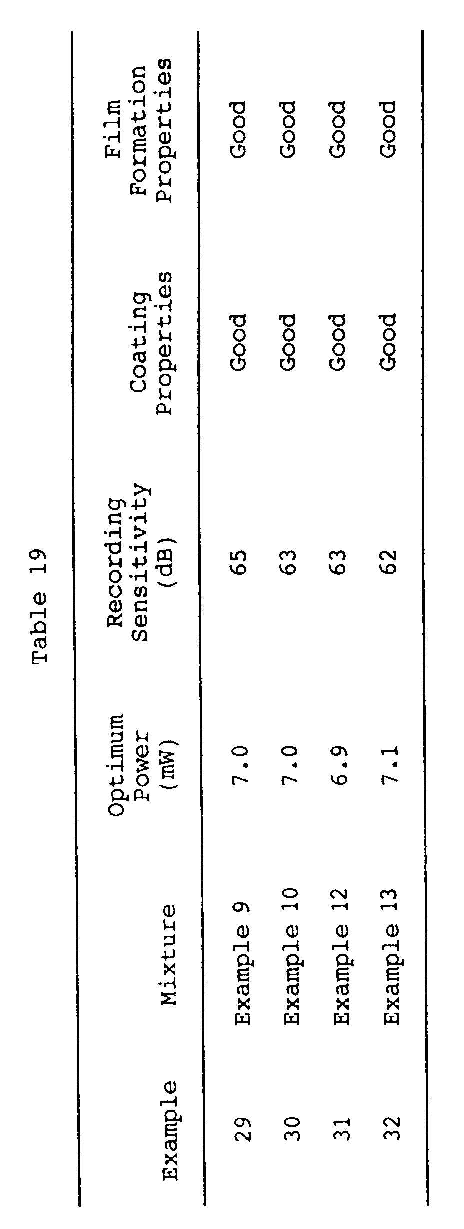

### Comparative Examples 9 to 16

In the preparation of the media of Comparative Examples 1 to 8, industrially continuous coating properties and film formation properties were tested as in Example 20. The results are set forth in Table 20.

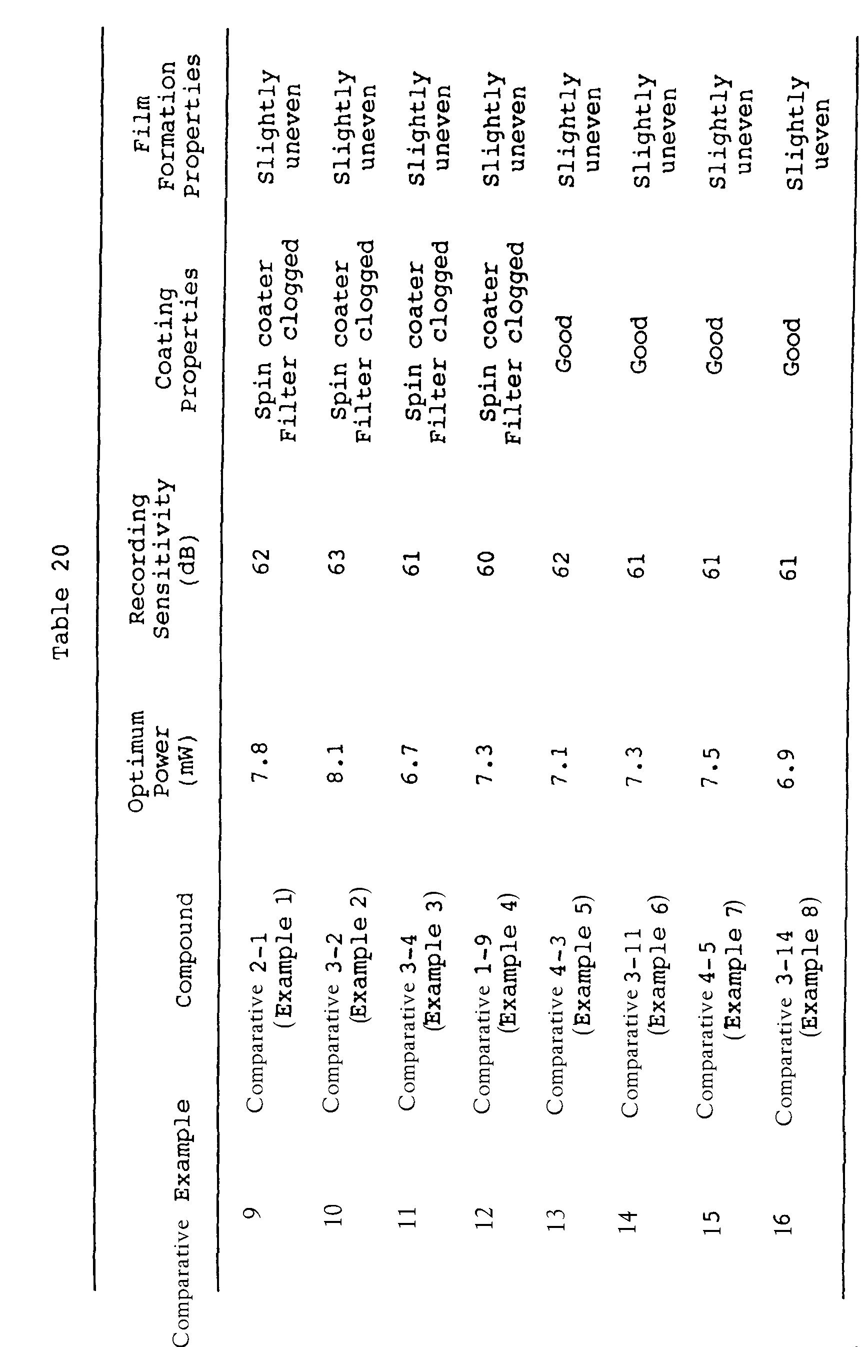

### Example 24

Two grams (1.96 mmol) of palladium tetraα-(2-methylpentoxy)phthalocyanine having the formula (9-13) 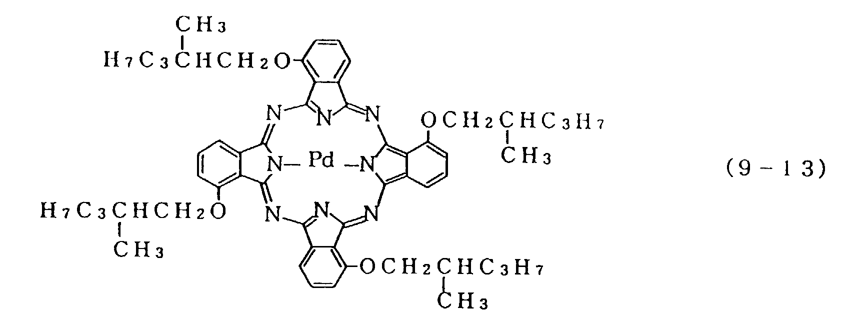 were dissolved in 40 g (61 ml) of n-hexane, and 10 g (10 ml) of water were then added. Next, 2.1 g (15.56 mmol) of sulfuryl chloride were added dropwise at 25 to 30°C, and the solution was heated up to 60 to 65°C. Reaction was then carried out at 60 to 65°C for 3 hours, and 10 g of a 10% aqueous sodium hydrogensulfite solution were added to wash the solution. The reaction solution was washed with 30 g of water three times and then dried over anhydrous sodium sulfate, and n-hexane was distilled off to obtain 2.4 g of a deep green solid.

The thus obtained solid was purified by silica gel chromatography (a development solvent was toluene). Yield was 2.0 g.
- Visible light absorption:: λₘₐₓ = 690 nm,
logε_{g} = 5.0 (toluene)

According to elemental analysis, it was apparent that the number of the substituted chlorine atoms was 4.3. Yield was 87.3%.

| Elemental analysis: C₅₆H_{59.7}N₈O₄Cl_{4.3}Pd | | | | |
|---|---|---|---|---|
| | C | H | N | Cl |
| Calcd. (%) | 57.60 | 5.15 | 9.60 | 13.06 |
| Found (%) | 57.64 | 5.22 | 9.48 | 12.85 |

According to liquid chromatography analysis, the product was a mixture of two isomers, and each single component was separated through a column and identified by NMR and MS analysis. As a result, it was apparent that the two isomers were compounds represented the formula (1-28) (present ratio 70%) and the formula (1-29) (present ratio 30%)

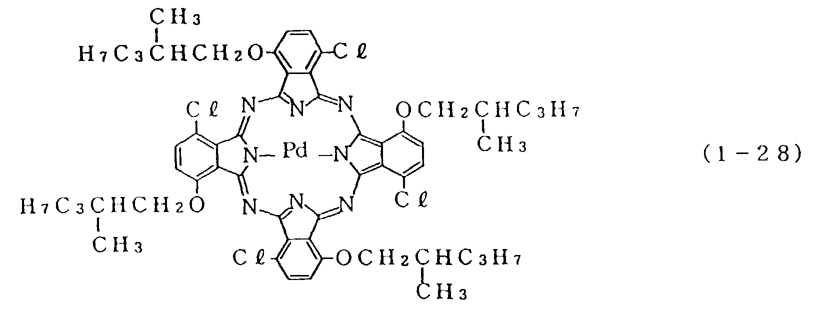

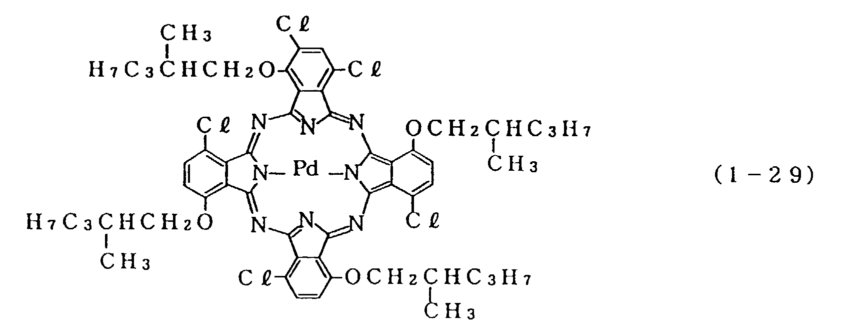

### Example 25

Ten grams (10.29 mmol) of nickel tetraα-(1,3-dimethylbutyloxy)phthalocyanine having the formula (11-13) 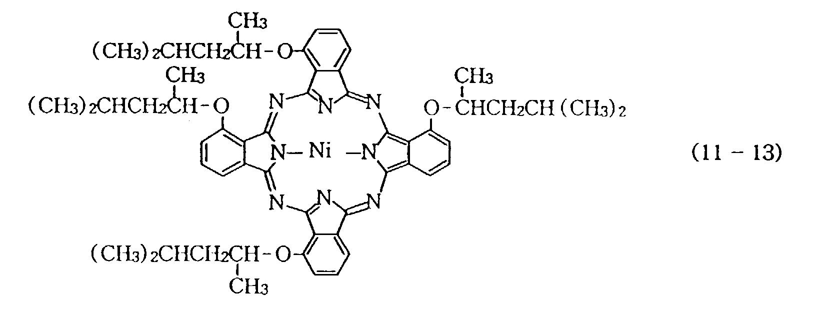 were dissolved in 140 g (212 ml) of n-hexane, and 50 g (50 ml) of water were added. Next, a mixture solution of 6.0 g (37.54 mmol) of bromine and 10 ml of acetic acid was added dropwise at 25 to 30°C, and the solution was then heated up to 60 to 65°C. Reaction was carried out at 60 to 65°C for 2 hours, and 50 g of a 10% aqueous sodium hydrogensulfite solution were added to wash the solution. The reaction solution was washed with 100 g of water three times and then dried over anhydrous sodium sulfate, and n-hexane was distilled off to obtain 11.0 g of a deep green solid.

The thus obtained solid was purified by the use of silica gel chromatography (a development solvent was toluene).

Yield was 9.7 g.
- Visible light absorption:: λₘₐₓ = 704.5 nm,
logε_{g} = 5.1 (toluene)

According to elemental analysis, it was apparent that the number of the substituted bromine atoms was 1.9. Yield was 84.3%.

| Elemental analysis: C₅₆H_{62.1}N₈O₄Br_{1.9}Ni | | | | |
|---|---|---|---|---|
| | C | H | N | Br |
| Calcd. (%) | 59.96 | 5.58 | 9.99 | 13.53 |
| Found (%) | 60.25 | 5.81 | 9.45 | 13.72 |

According to liquid chromatography analysis, the product was a mixture of two isomers, and each single component was separated through a column and identified by NMR and MS analysis. As a result, it was apparent that the two isomers were compounds represented by the formula (3-28) (present ratio 90%) and the formula (3-29) (present ratio 10%).

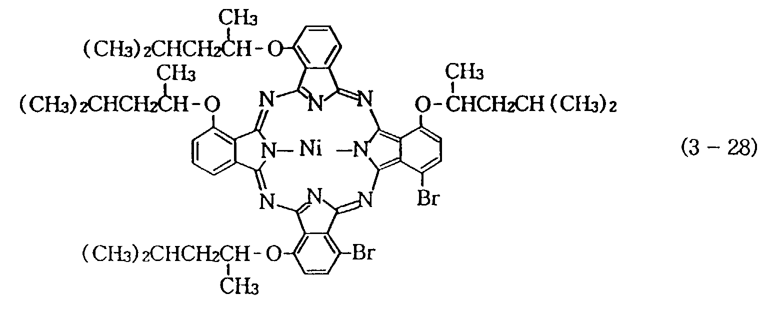

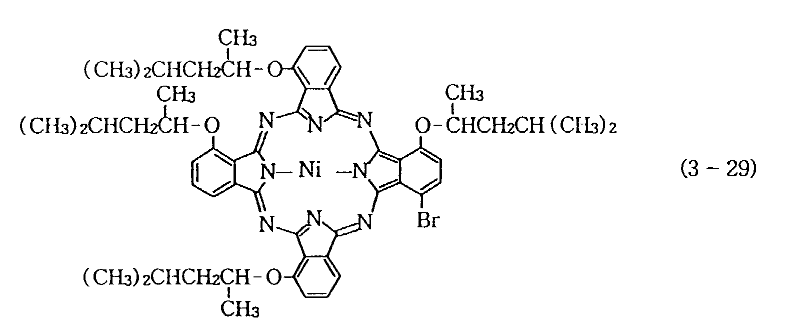

### Example 26

Five grams (4.65 mmol) of a mixture of palladium tetraα-(1-isopropyl-2-methylpropyloxy)phthalocyanines in which the ratio of the above-mentioned formulae (12-3) and (10-3) was 90:5 were dissolved in 35 g (53 ml) of n-hexane and 45 g (51 ml) of tetrahydrofuran, and 25 g (25 ml) of water were further added. Next, a mixture solution of 2.4 g (15.02 mmol) of bromine and 5 g of acetic acid was added dropwise at 25 to 30°C, and the solution was then heated up to 50 to 55°C. Reaction was carried out at 50 to 55°C for 2 hours, and 30 g of a 10% aqueous sodium hydrogensulfite solution were added to wash the solution. The reaction solution was washed with 50 g of water three times and then dried over anhydrous sodium sulfate. The n-hexane and the tetrahydrofuran were distilled off to obtain 6.5 g of a deep green solid.

The thus obtained solid was purified by silica gel chromatography (a development solvent was toluene/n-hexane = 1/1). Yield was 5.3 g.
- Visible light absorption:: λₘₐₓ = 706 nm,
logε_{g} = 5.1 (toluene)

According to elemental analysis, it was apparent that the number of the substituted bromine atoms was 2.8.

Yield was 89.3%.

| Elemental analysis: C₆₀H_{69.2}N₈O₄Br_{2.8}Pd | | | | |
|---|---|---|---|---|
| | C | H | N | Br |
| Calcd. (%) | 55.58 | 5.38 | 8.64 | 17.26 |
| Found (%) | 54.42 | 5.73 | 8.54 | 17.08 |

According to liquid chromatography and MS analysis, the product was a mixture of two isomers of the above-mentioned formulae (4-12) and (2-5).

### Example 27

Fifty grams (46.8 mmol) of a mixture of palladium tetraα-(1-isopropyl-2-methylpropyloxy)phthalocyanines in which the ratio between the above-mentioned formulae (11-3) and (9-3) was 90:10 were dissolved in 318 g (200 ml) of 1,1,2,2-tetrachloroethane and 630 g (800 ml) of ethylcyclohexane, and 500 g (500 ml) of water were further added. Next, a mixture solution of 21.2 g (132.65 mmol) of bromine and 64 g (40 ml) of 1,1,2,2-tetrachloroethane was added dropwise at 55 to 60°C, and reaction was then carried out at 55 to 60°C for 1 hour, and 250 g of a 10% aqueous sodium hydrogensulfite solution were added to wash the solution. The reaction solution was washed with 500 g of water three times and then dried over anhydrous sodium sulfate. The reaction solution was concentrated and then purified by the use of silica gel chromatography (a development solvent was toluene/n-hexane = 1/1). Yield was 56.1 g.
- Visible light absorption:: λₘₐₓ = 706 nm,
logε_{g} = 5.1 (toluene)

According to elemental analysis, it was apparent that the number of the substituted bromine atoms was 2.8. Yield was 93.0%.

| Elemental analysis: C₆₀H_{69.2}N₈O₄Br_{2.8}Pd | | | | |
|---|---|---|---|---|
| | C | H | N | Br |
| Calcd. (%) | 55.58 | 5.38 | 8.64 | 17.26 |
| Found (%) | 56.28 | 5.42 | 8.36 | 17.23 |

According to liquid chromatography and MS analysis, the product was a mixture of isomers of the above-mentioned formulae (3-18) and (1-18).

### Example 28

Fifty grams (46.48 mmol) of a mixture of palladium tetraα-(1-isopropyl-2-methylpropyloxy)phthalocyanines in which the ratio of the above-mentioned formulae (11-3) to (9-3) was 90:10 were dissolved in 288 g (200 ml) of 1,1,2-trichloroethane, and 100 g (100 ml) of water were then added. Next, a mixture solution of 22.3 g (139.53 mmol) of bromine and 58 g (36 ml) of 1,1,2-trichloroethane was added dropwise at 55 to 60°C, and reaction was then carried out at 55 to 60°C for 1 hour, and 50 g of a 10% aqueous sodium hydrogen-sulfite solution were added to wash the solution. The resultant organic layer was added dropwise to 790 g of methanol, and the precipitated crystals were filtered. Yield was 59.8 g.
- Visible light absorption:: λₘₐₓ = 706.5 nm,
logε_{g} = 5.1 (toluene)

According to elemental analysis, it was apparent that the number of the substituted bromine atoms was 3.0.

Yield was 98.0%.

| Elemental analysis: C₆₀H₆₉N₈O₄Br₃Pd | | | | |
|---|---|---|---|---|
| | C | H | N | Br |
| Calcd. (%) | 54.91 | 5.30 | 8.59 | 18.27 |
| Found (%) | 55.60 | 5.04 | 8.64 | 18.36 |

According to liquid chromatography and MS analysis, the product was a mixture of isomers of the above-mentioned formulae (3-18) and (1-18).

### Example 29

Ten grams (8.73 mmol) of a mixture of copper tetraα-(1-isopropyl-2-methylpropyloxy)phthalocyanines in which the ratio between the following formulae (11-14) and (10-13) was 90:10 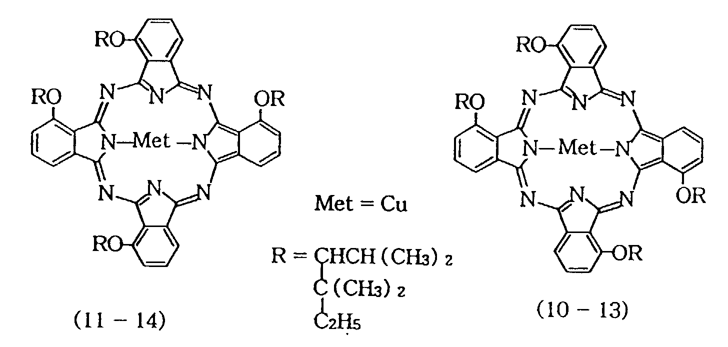 were dissolved in 54 g (40 ml) of 1,1,1-trichloroethane, and 20 g (20 ml) of water were then added. Next, a mixture solution of 4.19 g (26.22 mmol) of bromine and 11 g (8 ml) of 1,1,1-trichloroethane was added dropwise at 45 to 50°C, and reaction was then carried out at 50 to 55°C for 2 hours, and 20 g of a 10% aqueous sodium hydrogensulfite solution were added to wash the solution. The resultant organic layer was added dropwise to 152 g of methanol, and the precipitated crystals were filtered.

Yield (amount) was 11.8 g, and yield (ratio) was 97.5%.
- Visible light absorption:: λₘₐₓ = 718.0 nm,
logε_{g} = 5.1 (toluene)

According to elemental analysis, it was apparent that the number of the substituted bromine atoms was 3.0.

| Elemental analysis: C₆₈H₈₅N₈O₄Br₃Cu | | | | |
|---|---|---|---|---|
| | C | H | N | Br |
| Calcd. (%) | 59.11 | 6.20 | 8.11 | 17.35 |
| Found (%) | 59.41 | 5.92 | 8.00 | 17.53 |

According to liquid chromatography analysis, the product was a mixture of three isomers, and each single component was separated through a column and identified by NMR and MS. As a result, it was apparent that the three isomers were compounds represented by the formula (3-30) (present ratio 50%), the formula (3-31) (present ratio 40%) and the formula (2-15).

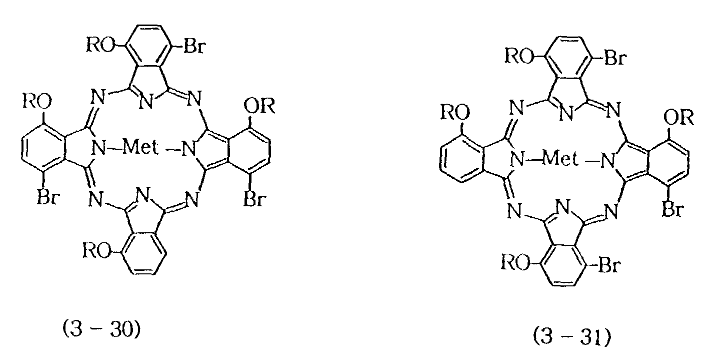

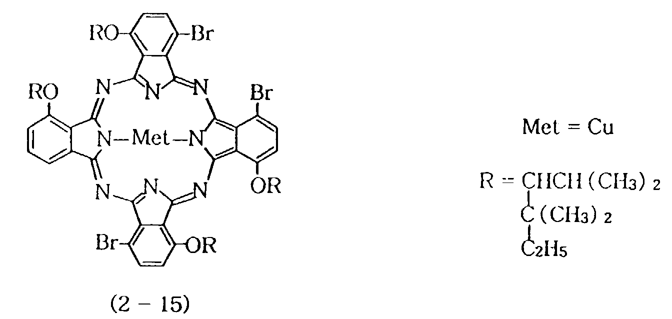

### Comparative Example 17

Ten grams (9.81 mmol) of a mixture of palladium tetraα-(1,3-dimethylbutyloxy)phthalocyanines having the formulae (9-14) 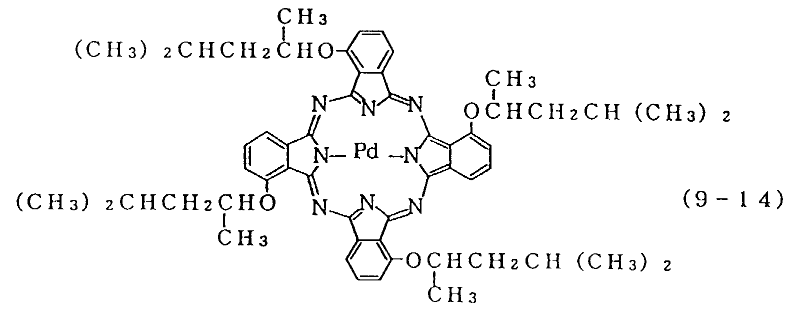 were dissolved in 400 g (251 ml) of carbon tetrachloride, and a mixture solution of 12.5 g (78.21 mmol) of bromine and 10 g of acetic acid was added dropwise at 25 to 30°C, and the solution was then heated up to 60 to 65°C. Reaction was carried out at 60 to 65°C for 10 minutes and at this point of time, a blackish green solid was precipitated. Under this state, the reaction was performed at 60 to 65°C for 4 hours, and 50 g of a 10% aqueous sodium hydrogensulfite solution were then added to wash the solution. 200 g of chloroform were added to the reaction solution to dissolve the solid, washed with 200 g of water 3 times, and then dried over anhydrous sodium sulfate. Carbon tetrachloride was distilled off to obtain a deep green solid.

The thus obtained solid was purified by silica gel chromatography (a development solvent was toluene). Yield was 6.7 g, and 3 g of the raw material were recovered.
- Visible light absorption:: λₘₐₓ = 699.5 nm,
logε_{g} = 5.1 (toluene)

According to elemental analysis, it was apparent that the number of the substituted bromine atoms was 3.6.

Yield was 52.4%, and there was no water in the solvent, and therefore the reaction did not proceed sufficiently and effective bromination could not be achieved.

| Elemental analysis: C₅₆H_{60.4}N₈O₄Br_{3.6}Pd | | | | |
|---|---|---|---|---|
| | C | H | N | Br |
| Calcd. (%) | 51.91 | 4.71 | 8.56 | 21.58 |
| Found (%) | 52.42 | 4.83 | 8.77 | 21.77 |

According to liquid chromatography and MS analysis, it was apparent that the product was a compound represented by the formula (1-30).

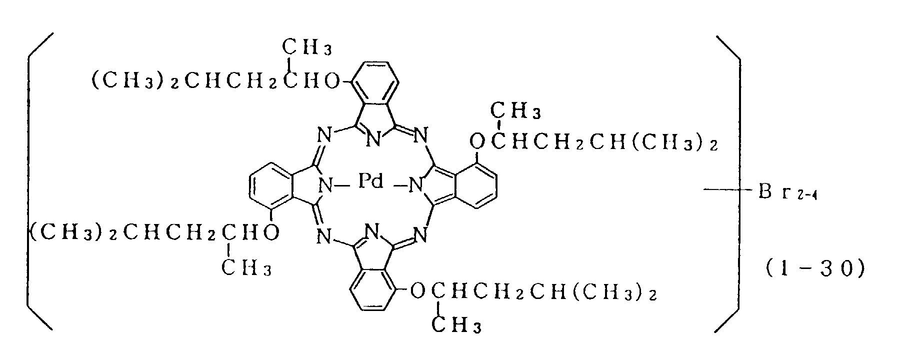

### Comparative Example 18

Five grams (4.65 mmol) of a mixture of palladium tetraα-(1-isopropyl-2-methylbutyloxy)phthalocyanines in which the ratio of the formula (11-3) to the formula (9-3) was 90:10 were dissolved in 58 g (40 ml) of 1,1,2-trichloroethane, and a mixture solution of 2.23 g (13.95 mmol) of bromine and 6 g (4 ml) of 1,1,2-trichloroethane was added dropwise at 50 to 55°C in order to introduce three bromine atoms on the average, and reaction was carried out at 55 to 60°C for 1 hour. 10 g of a 15% aqueous sodium hydrogensulfite solution were then added to wash the solution. The resultant organic layer was added dropwise to 80 g of methanol, and the precipitated crystals were filtered. Yield was 5.3 g.
- Visible light absorption:: λₘₐₓ = 696.0 nm,
logε_{g} = 5.3 (toluene)

According to elemental analysis, it was apparent that the number of the substituted bromine atoms was 1.0.

| Elemental analysis: C₆₀H₇₁N₈O₄Br₁Pd | | | | |
|---|---|---|---|---|
| | C | H | N | Br |
| Calcd. (%) | 62.42 | 6.20 | 9.71 | 6.92 |
| Found (%) | 62.60 | 6.04 | 9.54 | 7.08 |

According to liquid chromatography and MS analysis, the product was a mixture of isomers having the following formulae (3-32) and (1-31), and the desired three-substituted product could not be obtained.

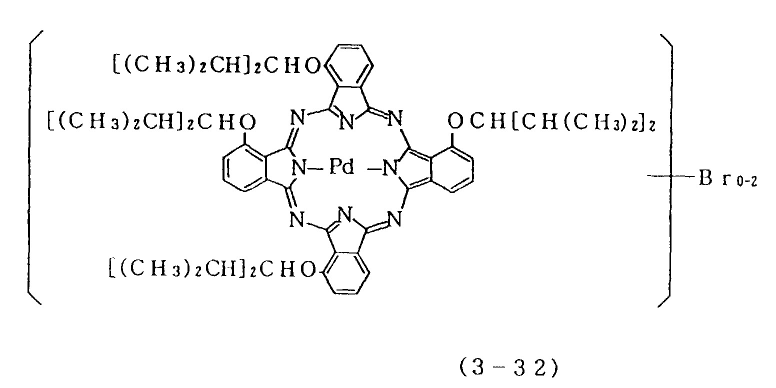

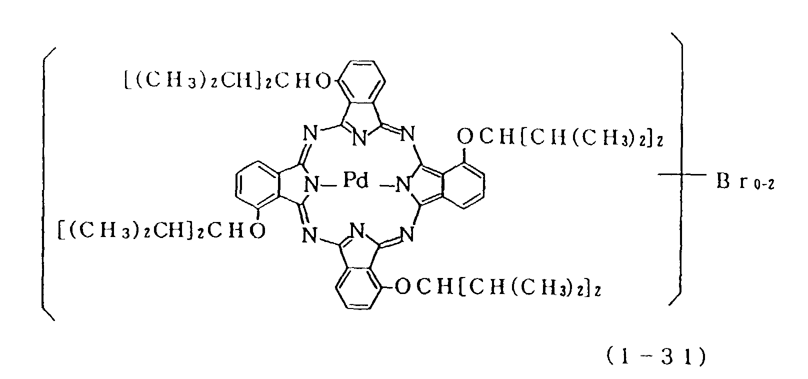

As described above, in the present invention, a halogenated alkoxyphthalocyanine which can be obtained by halogenating a phthalocyanine having, at the α-position, an alkoxy group having 2 to 4 of the secondary, tertiary or quaternary carbon atoms in all contains 5 or more isomers. Therefore, the alkoxyphthalocyanine is excellent in solubility in a solvent and film formation properties in a coating process. Furthermore, an absorption wave length can be changed by adjusting the amount of the halogen atoms to be introduced. Moreover, the phthalocyanine compound of the present invention has an alkoxy group having a large steric hindrance and the halogen atoms, and therefore it can provide recording layers of optical recording media having excellent sensitivity.

## Claims

1. A mixture of halogenated alkoxyphthalocyanine compounds represented by the formulae (1) to (4): 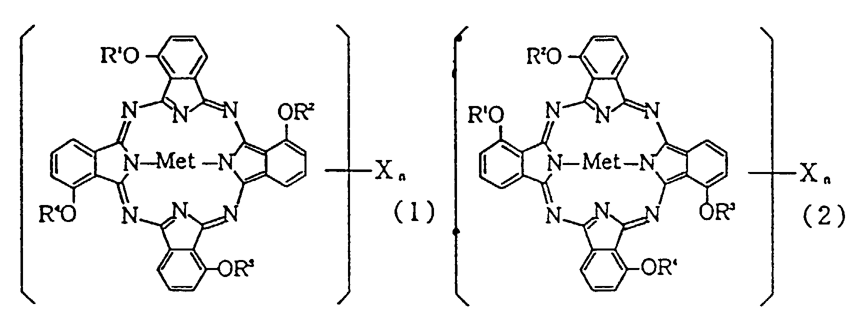 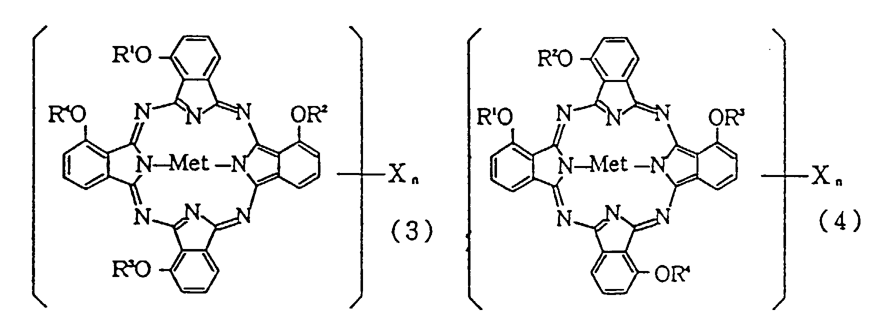 wherein each of R¹ to R⁴ is independently an alkyl group which has 2 to 4 of the secondary, tertiary or quaternary carbon atoms and in which the number of the total carbon atoms is from 6 to 9; Met is a divalent metallic atom, a trivalent one-substituted metallic atom or a tetravalent two-substituted metallic atom; X is a halogen atom; and n is the substitution number of X and it is from 1 to 4.

2. A mixture of the compounds according to claim 1, wherein the substituent X of said halogenated alkoxyphthalocyanine is a bromine atom.

3. A mixture of the compounds according to claim 2, wherein the substituents R¹ to R⁴ of said halogenated alkoxyphthalocyanine are one selected from the group consisting of a 1-iso-propyl-2-methylbutyl group, 1-t-butyl-2-methylpropyl group, 1-iso-propyl-2-methylpropyl group, 1,2-dimethylbutyl group, 1-iso-propylpropyl group and 1-iso-propylbutyl group.

4. A mixture of the compounds according to claim 3, wherein the central metal Met of said halogenated alkoxyphthalocyanine is one selected from the group consisting of iron, cobalt, nickel, copper, zinc, ruthenium, rhodium, palladium and platinum.

5. A method for preparing a mixture of halogenated alkoxyphthalocyanine compounds described in Claim 1 which comprises the steps of selecting one to four kinds of alkoxyphthalonitriles or alkoxydiiminoisoindolines represented by the formula (5) or (6) 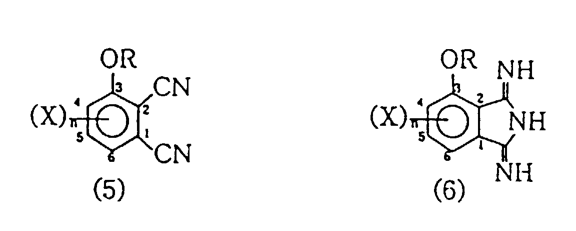 wherein R is an alkyl group which has 2 to 4 of the secondary, tertiary or quaternary carbon atoms and in which the number of the total carbon atoms is from 6 to 9; X is a halogen atom and is bonded at the 4-position or 6-position; and n is 0 or 1, at least one of these compounds having the substituent X; and then reacting the selected compound with a metal or metallic compound.

6. The method according to Claim 5 wherein said substituent R is one selected from the group consisting of a 1-iso-propyl-2-methylbutyl group, 1-t-butyl-2-methylpropyl group, 1-iso-propyl-2-methylpropyl group, 1,2-dimethylbutyl group, 1-iso-propylpropyl group and 1-iso-propylbutyl group.

7. The preparation method according to Claim 6 wherein said metal or metallic compound is one selected from the group consisting of iron chloride, iron bromide, iron acetate, cobalt chloride, cobalt bromide, cobalt acetate, nickel chloride, nickel bromide, nickel acetate, copper chloride, copper bromide, copper iodide, copper acetate, zinc chloride, zinc bromide, zinc acetate, ruthenium chloride, rhodium chloride, rhodium bromide, palladium chloride, palladium bromide, palladium acetate, platinum chloride and platinum bromide.

8. The preparation method according to Claim 7 wherein said reaction is carried out by the use of a solvent.

9. The preparation method according to Claim 8 wherein said solvent is an alcohol.

10. The preparation method according to Claim 9 wherein a reaction temperature is from 10 to 300°C.

11. The preparation method according to Claim 10 wherein said raw material is phthalonitrile represented by the formula (5) and said reaction temperature is from 80 to 160°C.

12. The preparation method according to Claim 9 wherein said raw material is diiminoisoindoline represented by the formula (6) and said reaction temperature is from 140 to 200°C.

13. An optical recording medium containing said mixture of halogenated alkoxyphthalocyanine compounds described in Claim 4 in a recording layer.

14. An optical recording medium comprising a substrate, a recording layer, a reflective layer and a protective layer, and containing said mixture of halogenated alkoxyphthalocyanine compounds described in Claim 4 in said recording layer.

15. The optical recording medium according to claim 13, wherein said halogenated alkoxyphthalocyanine contains a mixture of the halogenated alkoxyphthalocyanine compounds represented by the formulae (1) to (4) and at least one kind of isomer of the halogenated alkoxyphthalocyanine compounds represented by the formulae (1) to (4) in which the isomer has a different bromination degree.

16. A method for preparing a halogenated phthalocyanine compound represented by the formula (20) 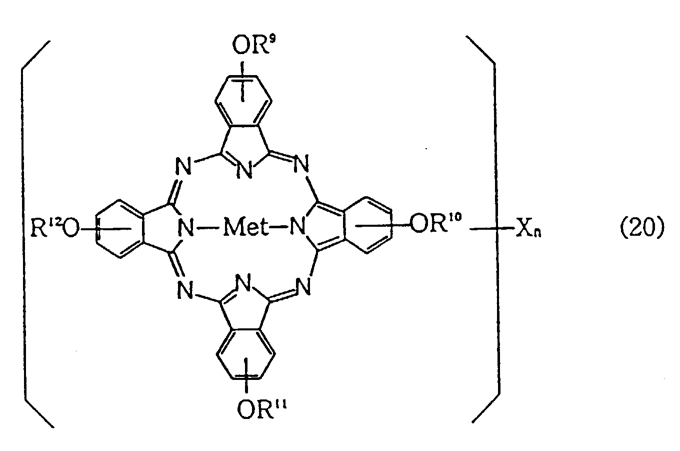 wherein each of R⁹ to R¹² independently is a substituted or unsubstituted alkyl group; and Met is two hydrogen atoms, a divalent metallic atom, a trivalent or a tetravalent metallic derivative; X is chlorine, bromine or iodine; and n is 1 ≤ n ≤ 12,
which comprises reacting a phthalocyanine compound represented by the formula (19) 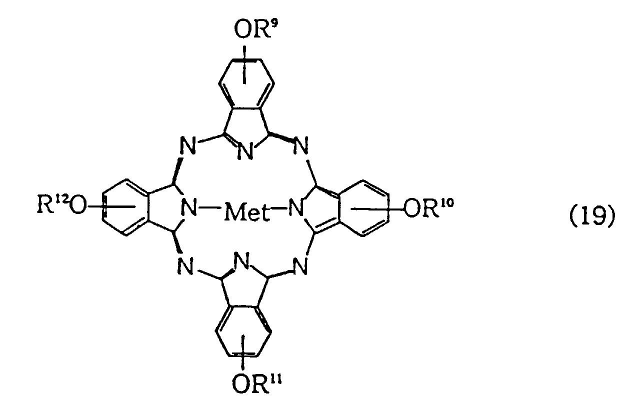 wherein R⁹ to R¹² and Met have the same meanings as in the formula (20),
with a halogenating agent at 20 to 90°C in a mixed solvent of an organic solvent which is substantially immiscible with water and water.

17. The method for preparing a halogenated phthalocyanine compound according to Claim 16 wherein said phthalocyanine compound is a compound represented by the formula (21), (22), (23) or (24) 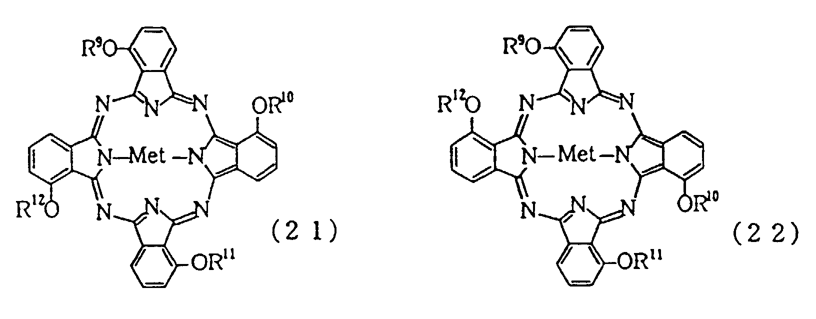 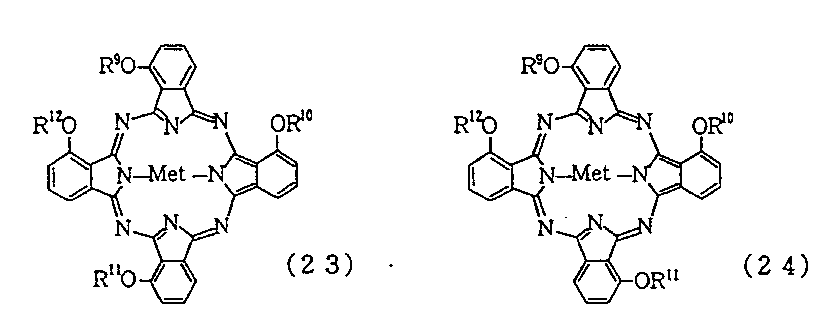 wherein R⁹ to R¹² and Met have the same meanings as in the formula (19), or a mixture of these compounds; and said halogenated phthalocyanine compound is a compound represented by the formula (25), (26), (27) or (28) 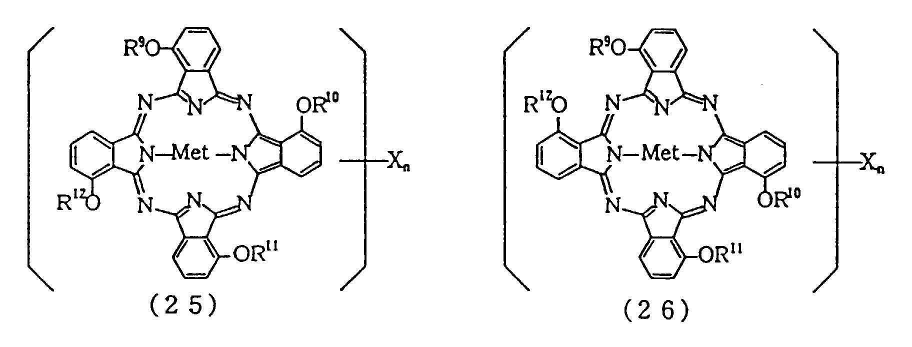 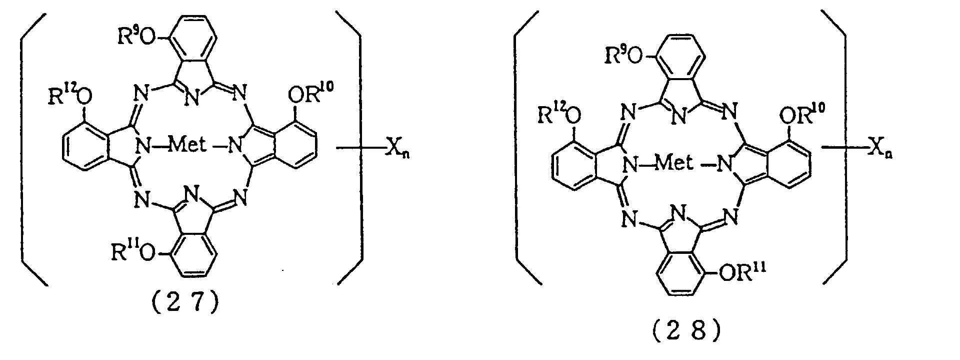 wherein R⁹ to R¹², Met, X and n have the same meanings as in the formula (20), or a mixture of these compounds.

18. The method for preparing a halogenated phthalocyanine compound according to Claim 17 wherein at least one of R⁹ to R¹² is a branched alkyl group.

19. The method for preparing a halogenated phthalocyanine compound according to Claim 18 wherein R⁹ to R¹² are secondary alkyl groups.

20. The method for preparing a halogenated phthalocyanine compound according to Clain 16 wherein X is bromine; n is 1 ≤ n ≤ 4; and Met is Pd, Cu, Ni, Co or VO.

21. The method for preparing a halogenated phthalocyanine compound according to Claim 16wherein said halogenating agent is bromine.

22. The method for preparing a halogenated phthalocyanine compound according to Claim 16 wherein said organic solvent is one or more selected from saturated hydrocarbons, ethers and halogenated hydrocarbons.

23. The method for preparing a halogenated phthalocyanine compound according to Claim 22 wherein said organic solvent is one or more selected from the group consisting of n-hexane, n-pentane, n-octane, cyclohexane, methylcyclohexane, ethylcyclohexane, tetrahydrofuran, n-butyl ether, n-propyl ether, isopropyl ether, carbon tetrachloride, chloroform, dichloromethane, 1,1,1-trichloroethane, 1,1,2-trichloroethane and 1,1,2,2-tetrachloroethane.

24. The method for preparing a halogenated phthalocyanine compound according to Claim 16 wherein the amount of said organic solvent is 2 to 200 times by weight as much as that of said phthalocyanine compound and said mixture.

25. The method for preparing a halogenated phthalocyanine compound according to Claim 16. wherein the amount of water is 0.1 to 5 times by weight as much as that of said organic solvent.

26. The method for preparing a halogenated phthalocyanine compound according to Claim 16 wherein the amount of said halogenating agent is in a molar ratio of 1-16 to said phthalocyanine compound and said mixture.

27. The method for preparing a halogenated phthalocyanine compound according to Claim 24 wherein the amount of said organic solvent is 4 to 10 times by weight as much as that of said phthalocyanine compound and said mixture.

28. The method for preparing a halogenated phthalocyanine compound according to Claim 26 wherein the amount of bromine is in a molar ratio of 1-4 to said phthalocyanine compound and said mixture.

## Patentansprüche

1. Mischung aus halogenierten Alkoxyphthalocyaninverbindungen, die durch die Formel (1) bis (4): 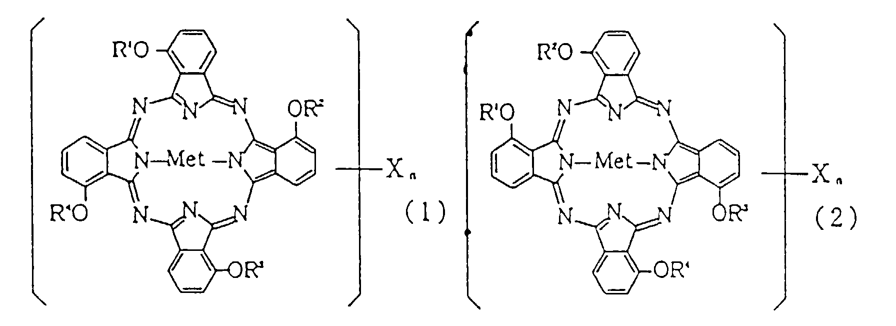 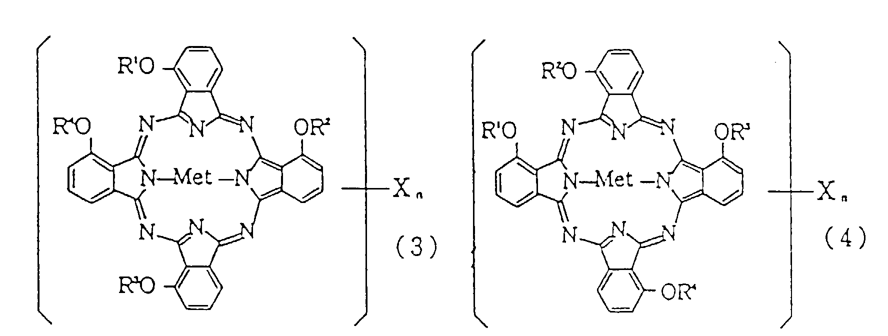 beschrieben werden, wobei jeder R¹ bis R⁴ unabhängig eine Alkylgruppe ist, die 2 bis 4 sekundäre, tertiäre oder quaternäre Kohlenstoffatome aufweist und in der die gesamte Anzahl der Kohlenstoffatome zwischen 6 und 9 liegt, Met ein zweiwertiges Metallatom, ein dreiwertiges, einfach substituiertes Metallatom oder ein vierwertiges, zweifach substituiertes Metallatom ist, X ein Halogenatom ist und n die Anzahl der Substitutionen an X ist und einen Wert von 1 bis 4 hat.

2. Mischung aus den Verbindungen nach Anspruch 1, wobei der Substituent X des halogenierten Alkoxyphthalocyanins ein Bromatom ist.

3. Mischung aus den Verbindungen nach Anspruch 2, wobei die Substituenten R¹ bis R⁴ des halogenierten Alkoxyphthalocyanins aus der Gruppe ausgewählt sind, die aus einer 1-Isopropyl-2-methylbutylgruppe, 1-t-Butyl-2-methylpropylgruppe, 1-Isopropyl-2-methylpropylgruppe, 1,2-Dimethylbutylgruppe, 1-Isopropypropylgruppe und 1-Isopropylbutylgruppe besteht.

4. Mischung aus den Verbindungen nach Anspruch 3, wobei das zentrale Metall Met des halogenierten Alkoxyphthalocyanins aus der Gruppe ausgewählt sind, die aus Eisen, Kobalt, Nickel, Kupfer, Zink, Ruthenium, Rhodium, Palladium und Platin besteht.

5. Verfahren zur Herstellung einer in Anspruch 5 beschriebenen Mischung aus halogenierten Alkoxyphthalocyaninverbindungen, die die Schritte umfaßt, ein bis vier Arten Alkoxyphthalonitrile oder Alkoxydiiminoisoindoline auszuwählen, die durch die Formeln (5) oder (6) 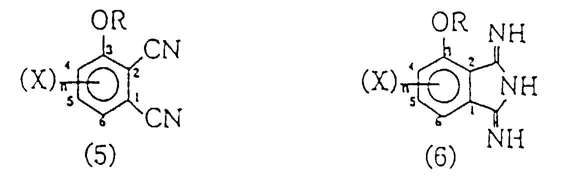 wiedergegeben werden, wobei R eine Alkylgruppe ist, die 2 bis 4 sekundäre, tertiäre oder quaternäre Kohlenstoffatome aufweist und in der die gesamte Anzahl an Kohlenstoffatome einen Wert von 6 bis 9 hat, X ein Halogenatom ist und an Position 4 oder Position 6 gebunden ist, und n 0 oder 1 ist, wobei zumindest eine dieser Verbindungen den Substituenten X aufweist, und dann die ausgewählte Verbindung mit einem Metall oder einer Metallverbindung zur Reaktion zu bringen.

6. Verfahren nach Anspruch 5, wobei der Substituent R aus der Gruppe ausgewählt ist, die aus einer 1 -Isopropyl-2-methylbutylgruppe, 1-t-Butyl-2-methylpropylgruppe, 1-Isopropyl-2-methylpropylgruppe, 1,2-Dimethylbutylgruppe, 1-Isopropypropylgruppe und 1-Isopropylbutylgruppe besteht.

7. Herstellungsverfahren nach Anspruch 6, wobei das Metall oder die Metallverbindung aus der Gruppe ausgewählt sind, die aus Eisenchlorid, Eisenbromid, Eisenacetat, Kobaltchlorid, Kobaltbromid, Kobaltacetat, Nickelchlorid, Nickelbromid, Nickelacetat, Kupferchlorid, Kupferbromid, Kupferiodid, Kupferacetat, Zinkchlorid, Zinkbromid, Zinkacetat, Rutheniumchlorid, Rhodiumchlorid, Rhodiumbromid, Palladiumchlorid, Palladiumbromid, Palladiumacetat, Platinchlorid und Platinbromid besteht.

8. Herstellungsverfahren nach Anspruch 7, wobei die Reaktion unter Verwendung eines Lösungsmittels durchgeführt wird.

9. Herstellungsverfahren nach Anspruch 8, wobei das Lösungsmittel ein Alkohol ist.

10. Herstellungsverfahren nach Anspruch 9, wobei die Reaktionstemperatur zwischen 10 und 300°C liegt.

11. Herstellungsverfahren nach Anspruch 10, wobei das Ausgangsmaterial Phthalonitril ist, das durch die Formel (5) wiedergegeben wird, und die Reaktionstemperatur zwischen 80 und 160° liegt.

12. Herstellungsverfahren nach Anspruch 9, wobei das Ausgangsmaterial Diiminoisoindolin ist, das durch die Formel (6) wiedergegeben wird, und die Reaktionstemperatur zwischen 140 und 200°C liegt.

13. Optisches Aufzeichnungsmedium, das in einer Aufzeichnungsschicht besagte, in Anspruch 4 beschriebene Mischung aus halogenierten Alkoxyphthalocyaninverbindungen enthält.

14. Optisches Aufzeichnungsmedium, das ein Substrat, eine Aufzeichnungsschicht, eine Reflexionsschicht und eine Schutzschicht aufweist, und besagte, in Anspruch 4 beschriebene Mischung aus halogenierten Alkoxyphthalocyaninverbindungen enthält.

15. Optisches Aufzeichnungsmedium nach Anspruch 13, wobei besagtes halogeniertes Alkoxyphthalocyanin eine Mischung aus den halogenierten Alkoxyphthalocyaninverbindungen enthält, die durch die Formeln (1) bis (4) wiedergegeben werden, und zumindest eine Isomerenart der halogenierten Alkoxyphthalocyaninverbindungen, die durch die Formeln (1) bis (4) wiedergegeben werden, in denen das Isomer einen unterschiedlichen Bromierungsgrad hat.

16. Verfahren zur Herstellung einer halogenierten Phthalocyaninverbindung, wiedergegeben durch die Formel (20) 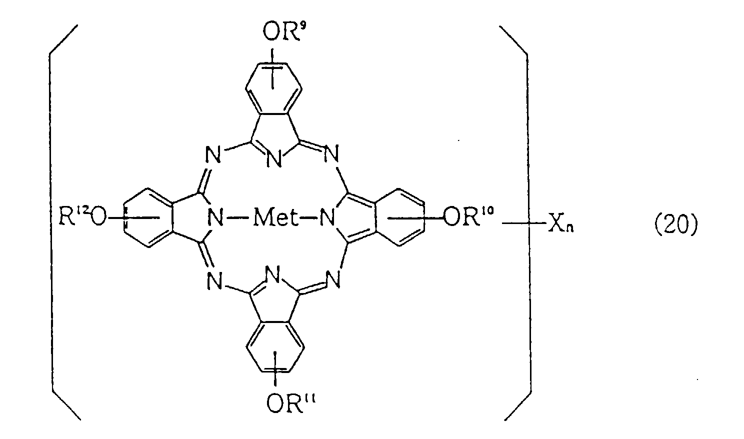 wobei jeder R⁹ bis R¹² unabhängig eine substituierte oder unsubstituierte Alkylgruppe ist und Met aus zwei Wasserstoffatomen, einem zweiwertigen Metallatom, einem dreiwertigen oder einem vierwertigen Metallderivat besteht, X Chlor, Brom oder Iod ist und für n 1 ≤ n ≤ 12 gilt,
das beinhaltet, daß eine Phthalocyaninverbindung, die durch die Formel (19) 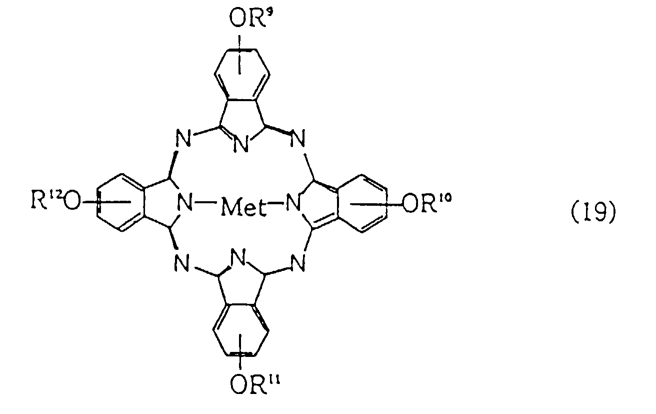 wiedergegeben wird, wobei R⁹ bis R¹² und Met dieselbe Bedeutung wie in Formel (20) haben,
mit einem Halogenierungsmittel in einem gemischten Lösungsmittel aus einem organischen Lösungsmittel, das im wesentlichen mit Wasser nicht mischbar ist, und aus Wasser bei 20 bis 90°C zur Reaktion gebracht wird.

17. Verfahren zur Herstellung einer halogenierten Phthalocyaninverbindung nach Anspruch 16, wobei besagte Phthalocyaninverbindung eine Verbindung ist, die durch die Formeln (21), (22), (23) oder (24) 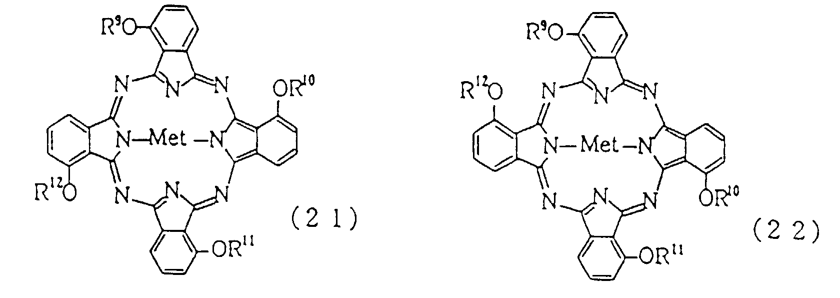 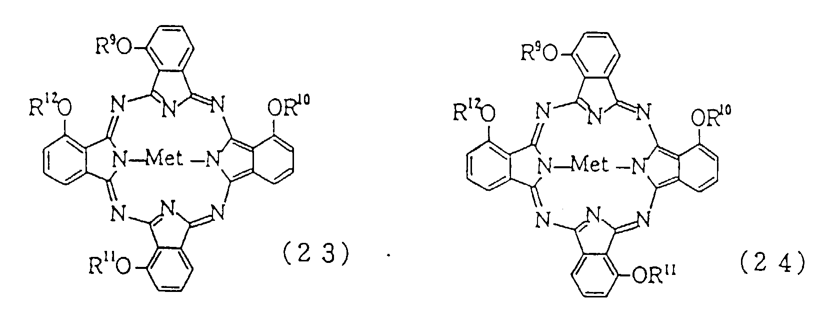 wiedergegeben werden, wobei R⁹ bis R¹² und Met dieselbe Bedeutung wie in Formel (19) haben, oder eine Mischung dieser Verbindungen und besagte halogenierte Phthalocyaninverbindung eine Verbindung ist, die durch die Formeln (25, (26), (27) oder (28) 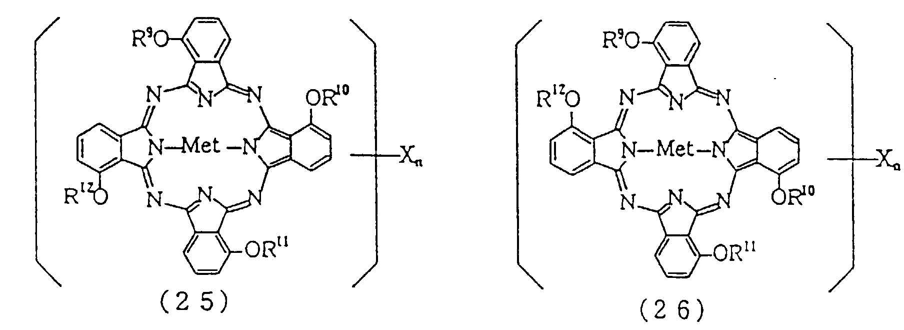 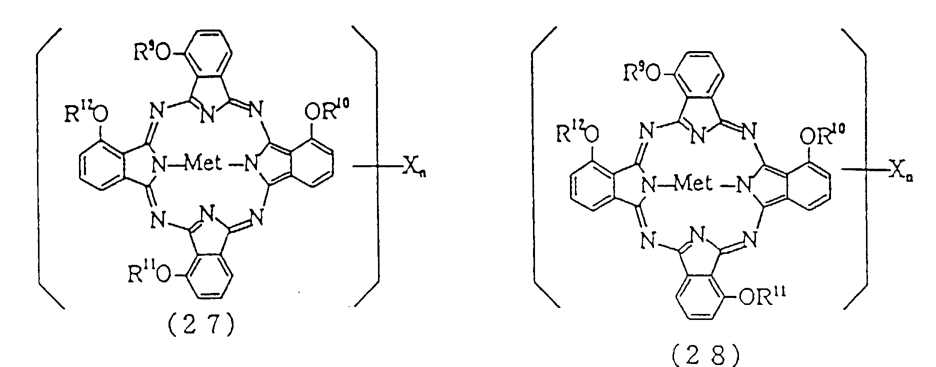 wiedergegeben werden, wobei R⁹ bis R¹², Met, X und n dieselbe Bedeutung wie in Formel (20) haben, oder eine Mischung aus diesen Verbindungen.

18. Verfahren zur Herstellung einer halogenierten Phthalocyaninverbindung nach Anspruch 17, wobei zumindest einer aus R⁹ bis R¹² eine verzweigte Alkylgruppe ist.

19. Verfahren zur Herstellung einer halogenierten Phthalocyaninverbindung nach Anspruch 18, wobei R⁹ bis R¹² sekundäre Alkylgruppen sind.

20. Verfahren zur Herstellung einer halogenierten Phthalocyaninverbindung nach Anspruch 16, wobei X Brom ist, für n 1 ≤ n ≤ 4 gilt und Met Pd, Cu, Ni, Co oder VO ist.

21. Verfahren zur Herstellung einer halogenierten Phthalocyaninverbindung nach Anspruch 16, wobei das Halogenierungsmittel Brom ist.

22. Verfahren zur Herstellung einer halogenierten Phthalocyaninverbindung nach Anspruch 16, wobei besagtes organisches Lösungsmittel aus einer oder mehreren Verbindungen besteht, die aus gesättigten Kohlenwasserstoffen, Ethern und halogenierten Kohlenwasserstoffen ausgewählt sind.

23. Verfahren zur Herstellung einer halogenierten Phthalocyaninverbindung nach Anspruch 22, wobei besagtes organisches Lösungsmittel aus einer oder mehreren Verbindungen besteht, die aus der Gruppe ausgewählt sind, die aus n-Hexan, n-Pentan, n-Octan, Cyclohexan, Methylcyclohexan, Ethylcyclohexan, Tetrahydrofuran, n-Butylether, n-Propylether, Isopropylether, Tetrachlorkohlenstoff, Chloroform, Dichlormethan, 1,1,1-Trichlorethan, 1,1,2-Trichlorethan und 1,1,2,2-Tetrachlorethan besteht.

24. Verfahren zur Herstellung einer halogenierten Phthalocyaninverbindung nach Anspruch 16, wobei die Menge des organischen Lösungsmittels das 2 bis 200-fache der Menge der Phthalocyaninverbindung und besagter Mischung in Gewicht beträgt.

25. Verfahren zur Herstellung einer halogenierten Phthalocyaninverbindung nach Anspruch 16, wobei die Menge des Wasser das 0,1 bis 5-fache der Menge des organischen Lösungsmittels in Gewicht beträgt.

26. Verfahren zur Herstellung einer halogenierten Phthalocyaninverbindung nach Anspruch 16, wobei die Menge des Halogenierungsmittels in bezug auf die Phthalocyaninverbindung und besagte Mischung in einem molaren Verhältnis von 1-16 vorliegt.

27. Verfahren zur Herstellung einer halogenierten Phthalocyaninverbindung nach Anspruch 24, wobei die Menge des organischen Lösungsmittels das 4 bis 10-fache der Menge der Phthalocyaninverbindung und besagter Mischung in Gewicht beträgt.

28. Verfahren zur Herstellung einer halogenierten Phthalocyaninverbindung nach Anspruch 26, wobei die Menge an Brom in bezug auf die Phthalocyaninverbindung und besagte Mischung in einem molaren Verhältnis von 1-4 vorliegt.

## Revendications

1. Mélange de composés d'alcoxyphtalocyanine halogénée représentés par les formules (1) à (4) 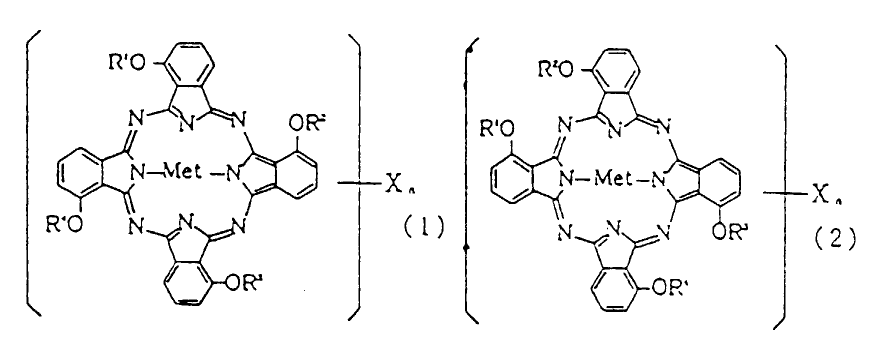 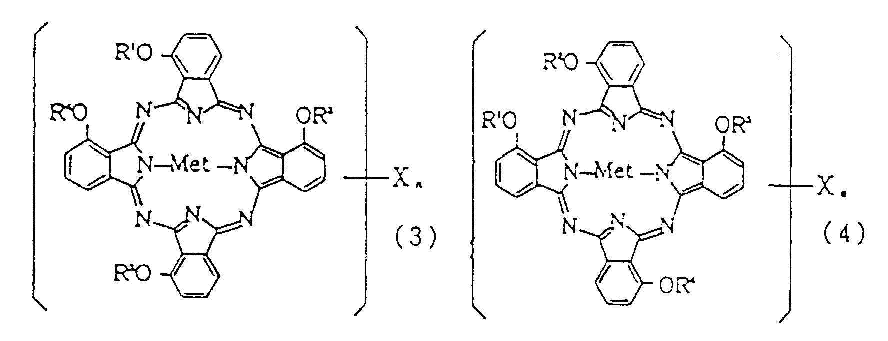 dans lesquelles chacun des R¹ à R⁴ est indépendamment un groupe alkyle ayant 2 à 4 atomes de carbone secondaires, tertiaires ou quaternaires, et dont le nombre total des atomes de carbone est de 6 à 9; Met est un atome métallique divalent, un atome métallique trivalent monosubstitué ou un atome métallique tétravalent disubstitué; X est un atome d'halogène; et n est l'indice de substitution de X et est compris entre 1 et 4.

2. Mélange de composés selon la revendication 1, dans lequel le substituant X de ladite alcoxyphtalocyanine halogénée est un atome de brome.

3. Mélange de composés selon la revendication 2, dans lequel les substituants R¹ à R⁴ de ladite alcoxyphtalocyanine halogénée sont choisis dans le groupe constitué par le groupe 1-isopropyl-2-méthylbutyle, le groupe 1-t-butyl-2-méthylpropyle, le groupe 1-isopropyl-2-méthylpropyle, le groupe 1,2-diméthylbutyle, le groupe 1-isopropylpropyle et le groupe 1-isopropylbutyle.

4. Mélange de composés selon la revendication 3, dans lequel le métal central Met de ladite alcoxyphtalocyanine halogénée est choisi dans le groupe constitué par le fer, le cobalt, le nickel, le cuivre, le zinc, le ruthénium, le rhodium, le palladium et le platine.

5. Procédé de préparation d'un mélange de composés d'alcoxyphtalocyanine halogénée décrit dans la revendication 1, qui comprend les étapes selon lesquelles on choisit un à quatre types d'alcoxyphtalonitriles ou d'alcoxydiiminoisoindolines représentés par la formule (5) ou (6) 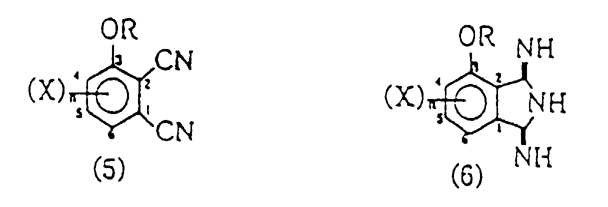 dans lesquelles R est un groupe alkyle ayant 2 à 4 atomes de carbone secondaires, tertiaires ou quaternaires, et dont le nombre total des atomes de carbone est de 6 à 9; X est un atome d'halogène et est lié en position 4 ou en position 6; et n est 0 ou 1, au moins l'un de ces composés ayant le substituant X; puis on fait réagir le composé choisi avec un métal ou un composé métallique.

6. Procédé selon la revendication 5, dans lequel ledit substituant R est choisi dans le groupe constitué par le groupe 1-isopropyl-2-méthylbutyle, le groupe 1-t-butyl-2-méthylpropyle, le groupe 1-isopropyl-2-méthylpropyle, le groupe 1,2-diméthylbutyle, le groupe 1-isopropylpropyle et le groupe 1-isopropylbutyle.

7. Procédé de préparation selon la revendication 6, dans lequel ledit métal ou composé métallique est choisi dans le groupe constitué par le chlorure de fer, le bromure de fer, l'acétate de fer, le chlorure de cobalt, le bromure de cobalt, l'acétate de cobalt, le chlorure de nickel, le bromure de nickel, l'acétate de nickel, le chlorure de cuivre, le bromure de cuivre, l'iodure de cuivre, l'acétate de cuivre, le chlorure de zinc, le bromure de zinc, l'acétate de zinc, le chlorure de ruthénium, le chlorure de rhodium, le bromure de rhodium, le chlorure de palladium, le bromure de palladium, l'acétate de palladium, le chlorure de platine et le bromure de platine.

8. Procédé de préparation selon la revendication 7, dans lequel on effectue ladite réaction en utilisant un solvant.

9. Procédé de préparation selon la revendication 8, dans lequel ledit solvant est un alcool.

10. Procédé de préparation selon la revendication 9, dans lequel la température de la réaction est comprise entre 10 et 300°C.

11. Procédé de préparation selon la revendication 10, dans lequel ledit produit de départ est un phtalonitrile représenté par la formule (5) et ladite température de la réaction est comprise entre 80 et 160°C.

12. Procédé de préparation selon la revendication 9, dans lequel ledit produit de départ est une diiminoisoindoline représentée par la formule (6) et ladite température de la réaction est comprise entre 140 et 200°C.

13. Support d'enregistrement optique contenant ledit mélange de composés d'alcoxyphtalocyanine halogénée décrit dans la revendication 4 dans une couche d'enregistrement.

14. Support d'enregistrement optique comprenant un substrat, une couche d'enregistrement, une couche réfléchissante et une couche protectrice, et contenant ledit mélange de composés d'alcoxyphtalocyanine halogénée décrit dans la revendication 4 dans ladite couche d'enregistrement.

15. Support d'enregistrement optique selon la revendication 13, dans lequel ladite alcoxyphtalocyanine halogénée contient un mélange de composés d'alcoxyphtalocyanine halogénée représentés par les formules (1) à (4) et au moins un type d'isomère des composés d'alcoxyphtalocyanine halogénée représentés par les formules (1) à (4) où l'isomère a un degré de bromation différent.

16. Procédé de préparation d'un composé de phtalocyanine halogénée représenté par la formule (20) 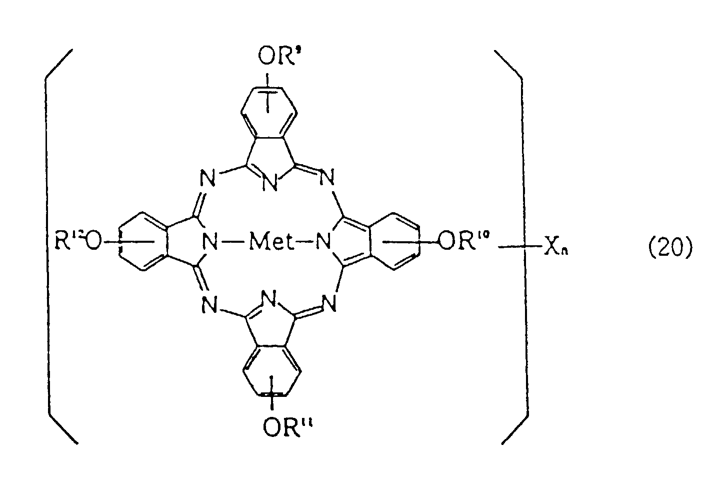 dans laquelle chacun des R⁹ à R¹² est indépendamment un groupe alkyle substitué ou non substitué; et Met représente 2 atomes d'hydrogène, un atome métallique divalent ou un dérivé de métal trivalent ou tétravalent; X est le chlore, le brome ou l'iode; et n est tel que 1 ≤ n ≤ 12,
qui comprend la réaction d'un composé de phtalocyanine représenté par la formule (19) 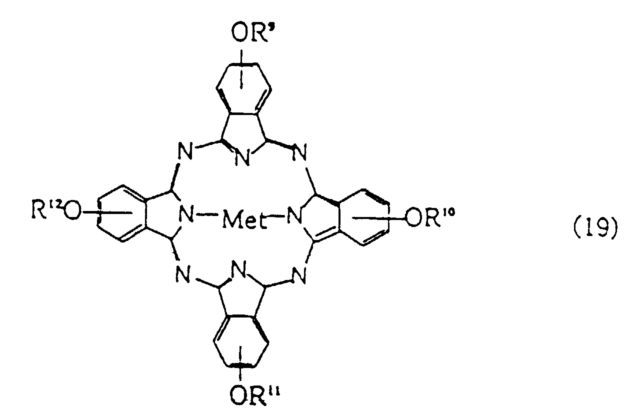 dans laquelle les R⁹ à R¹² et Met ont la même signification que dans la formule (20),
avec un agent d'halogénation à une température de 20 à 90°C, dans un solvant mixte constitué d'un solvant organique essentiellement non miscible à l'eau et d'eau.

17. Procédé de préparation d'un composé de phtalocyanine halogénée selon la revendication 16, dans lequel ledit composé de phtalocyanine est un composé représenté par la formule (21), (22), (23) ou (24) 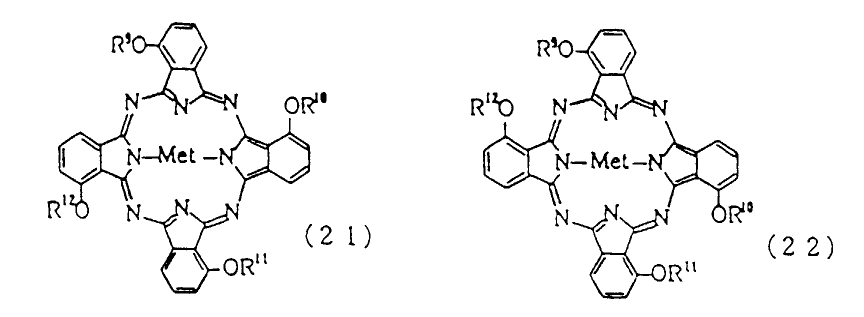 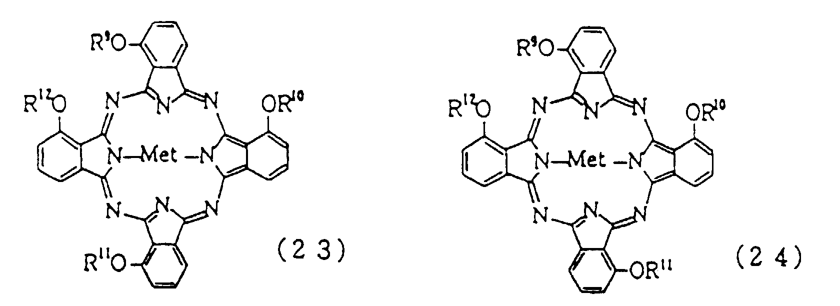 dans lesquelles les R⁹ à R¹² et Met ont la même signification que dans la formule (19), ou un mélange de ces composés; et ledit composé de phtalocyanine halogénée est un composé représenté par la formule (25), (26), (27) ou (28) 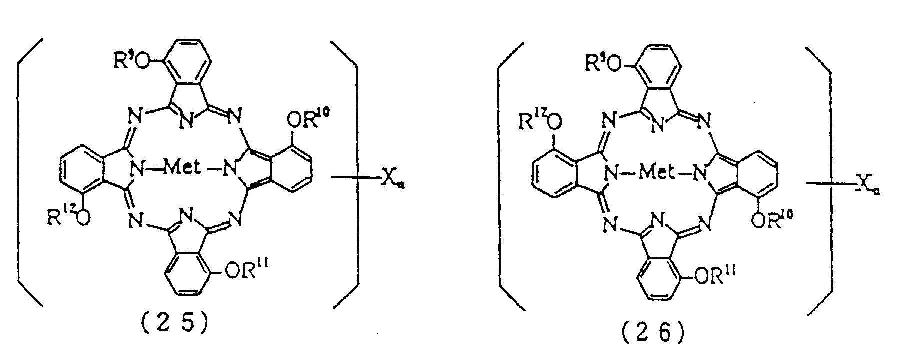 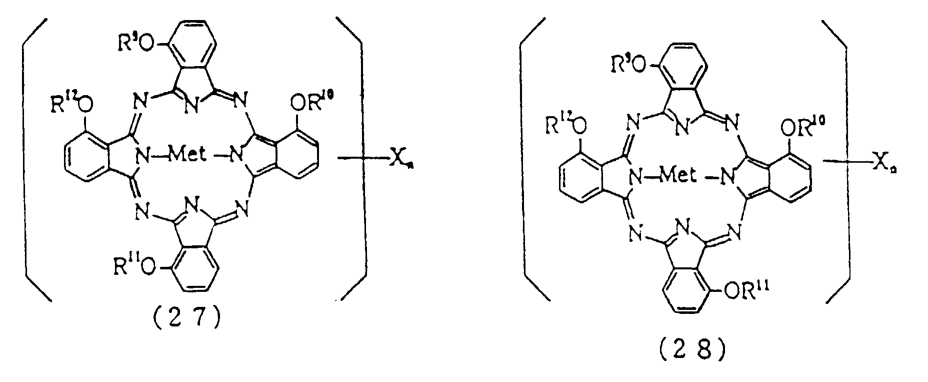 dans lesquelles les R⁹ à R¹², Met, X et n ont la même signification que dans la formule (20), ou un mélange de ces composés.

18. Procédé de préparation d'un composé de phtalocyanine halogénée selon la revendication 17, dans lequel au moins l'un des R⁹ à R¹² est un groupe alkyle ramifié.

19. Procédé de préparation d'un composé de phtalocyanine halogénée selon la revendication 18, dans lequel les R⁹ à R¹² sont des groupes alkyle secondaires.

20. Procédé de préparation d'un composé de phtalocyanine halogénée selon la revendication 16, dans lequel X est le brome; n est tel que 1 ≤ n ≤ 4; et Met est Pd, Cu, Ni, Co ou VO.

21. Procédé de préparation d'un composé de phtalocyanine halogénée selon la revendication 16, dans lequel ledit agent d'halogénation est le brome.

22. Procédé de préparation d'un composé de phtalocyanine halogénée selon la revendication 16, dans lequel ledit solvant organique est un ou plusieurs solvants choisis parmi des hydrocarbures saturés, des éthers et des hydrocarbures halogénés.

23. Procédé de préparation d'un composé de phtalocyanine halogénée selon la revendication 22, dans lequel ledit solvant organique est un ou plusieurs solvants choisis dans le groupe constitué par le n-hexane, le n-pentane, le n-octane, le cyclohexane, le méthylcyclohexane, l'éthylcyclohexane, le tétrahydrofurane, l'éther n-butylique, l'éther n-propylique, l'éther isopropylique, le tétrachlorure de carbone, le chloroforme, le dichlorométhane, le 1,1,1-trichloroéthane, le 1,1,2-trichloroéthane et le 1,1,2,2-tétrachloroéthane.

24. Procédé de préparation d'un composé de phtalocyanine halogénée selon la revendication 16, dans lequel la quantité dudit solvant organique est de 2 à 200 fois en masse celle dudit composé de phtalocyanine et dudit mélange.

25. Procédé de préparation d'un composé de phtalocyanine halogénée selon la revendication 16, dans lequel la quantité d'eau est de 0,1 à 5 fois en masse celle dudit solvant organique.

26. Procédé de préparation d'un composé de phtalocyanine halogénée selon la revendication 16, dans lequel la quantité dudit agent d'halogénation est comprise dans un rapport molaire de 1-16 par rapport audit composé de phtalocyanine et audit mélange.

27. Procédé de préparation d'un composé de phtalocyanine halogénée selon la revendication 24, dans lequel la quantité dudit solvant organique est de 4 à 10 fois en masse celle dudit composé de phtalocyanine et dudit mélange.

28. Procédé de préparation d'un composé de phtalocyanine halogénée selon la revendication 26, dans lequel la quantité de brome est comprise dans un rapport molaire de 1-4 par rapport audit composé de phtalocyanine et audit mélange.
